# EUROPEAN PATENT APPLICATION

(11) **EP 4 265 618 A1**
(43) Date of publication of application: **25.10.2023**
(21) Application number: 21911234.9
(22) Date of filing: 05.11.2021
(51) Int. Cl.: C07D 491/052, C07D 495/04, H01L 51/00, H01L 51/50

(54) **HETEROCYCLIC COMPOUND, ORGANIC LIGHT-EMITTING DEVICE COMPRISING SAME, MANUFACTURING METHOD THEREFOR, AND COMPOSITION FOR ORGANIC LAYER**

(30) Priority: 21.12.2020 KR 20200180226
(71) Applicant: LT Materials Co., Ltd., Yongin-si, Gyeonggi-do 17118 (KR)
(72) Inventor: CHAE, Woo Jeong, Yongin-si, Gyeonggi-do 17118 (KR); MO, Jun Tae, Yongin-si, Gyeonggi-do 17118 (KR); KIM, Dong Jun, Yongin-si, Gyeonggi-do 17118 (KR); LEE, Sol, Yongin-si, Gyeonggi-do 17118 (KR)
(74) Representative: Goddar, Heinz J.
(86) International application number: PCT/KR2021/016012
(87) International publication number: WO 2022/139167

(57) **Abstract**

The present specification relates to a heterocyclic compound represented by Formula 1, an organic light emitting device comprising the same, a method for preparing the same, and a composition for organic layer.

## Description

### [Technical Field]

The present application claims the benefit of priority based on Korean Patent Application No. 10-2020-0180226 filed on December 21, 2020, the entire contents of which are incorporated herein by reference.

The present invention relates to a heterocyclic compound, an organic light emitting device comprising the same, a composition for organic layer of the organic light emitting device, and a method for manufacturing the organic light emitting device.

### [Background Art]

An organic light emitting device is a kind of self-emitting display device, which has the advantage of having a wide viewing angle, excellent contrast, and also fast response speed.

The organic light emitting device has a structure in which an organic thin film is disposed between two electrodes. When a voltage is applied to the organic light emitting device having such structure, electrons and holes injected from the two electrodes are combined in the organic thin film to form a pair, and then emit light while disappearing. The organic thin film may be composed of a single layer or multiple layers, as required.

The material of the organic thin film may have a light emitting function, if necessary. For example, as a material for the organic thin film, a compound capable of forming a light emitting layer by itself alone may be used, or a compound capable of serving as a host or dopant of a host-dopant-based light emitting layer may also be used. In addition, as a material for the organic thin film, a compound capable of performing the roles of hole injection, hole transport, electron blocking, hole blocking, electron transport, electron injection, and the like may be used.

In order to improve the performance, lifetime, or efficiency of an organic light emitting device, there is a continuous demand for the development of materials for the organic thin film.

### [Prior Art Document]

[Patent Document]
US Patent No. 4,356,429

### [Disclosure]

### [Technical Problem]

It is an object of the present invention to provide a heterocyclic compound, an organic light emitting device comprising the same, a manufacturing method thereof, and a composition for organic layer.

### [Technical Solution]

The present invention provides a heterocyclic compound represented by Formula 1 below: 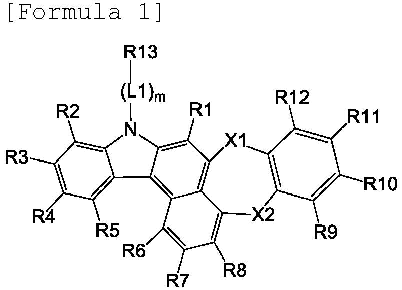 wherein,
one of X1 and X2 is O or S, and the other is a direct bond,
L1 is a single bond; a substituted or unsubstituted C6 to C60 arylene group; or a substituted or unsubstituted C2 to C60 heteroarylene group,
m is an integer from 0 to 5, and when m is 2 or more, each L1 is the same as or different from each other,
R1 to R13 are the same as or different from each other and are each independently selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; - P(=O)R101R102; -SiR101R102R103; and -NR101R102, or two or more groups adjacent to each other are combined with each other to form a substituted or unsubstituted C6 to C60 aromatic hydrocarbon ring or a substituted or unsubstituted C2 to C60 heterocyclic ring, wherein R101, R102, and R103 are the same as or different from each other and are each independently a substituted or unsubstituted C1 to C60 alkyl group; a substituted or unsubstituted C6 to C60 aryl group; or a substituted or unsubstituted C2 to C60 heteroaryl group.

In addition, the present invention provides an organic light emitting device comprising a first electrode; a second electrode provided to face the first electrode; and one or more organic layers provided between the first electrode and the second electrode, wherein at least one of the one or more organic layers comprises a heterocyclic compound represented by Formula 1 above.

In addition, the present invention provides an organic light emitting device wherein the organic layer further comprises a heterocyclic compound represented by Formula 2 below: 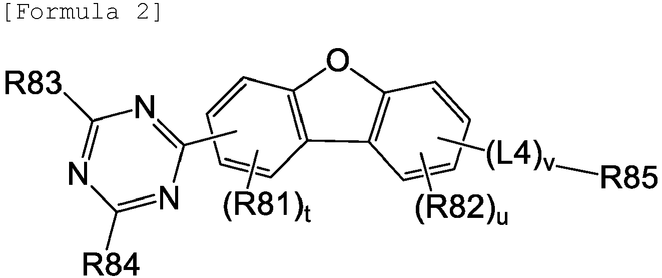 wherein,
L4 is a single bond; a substituted or unsubstituted C6 to C60 arylene group; or a substituted or unsubstituted C2 to C60 heteroarylene group,
R81 to R85 are the same as or different from each other, and each independently are selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; - P(=O)R101R102; -SiR101R102R103; and -NR101R102, or two or more groups adjacent to each other are combined with each other to form a substituted or unsubstituted C6 to C60 aromatic hydrocarbon ring or a substituted or unsubstituted C2 to C60 heterocycle, wherein R101, R102, and R103 are the same as or different from each other, and are each independently a substituted or unsubstituted C1 to C60 alkyl group; a substituted or unsubstituted C6 to C60 aryl group; or a substituted or unsubstituted C2 to C60 heteroaryl group,
t is an integer from 0 to 3, and when t is 2 or more, each R81 is the same as or different from each other, u is an integer from 0 to 3, and when u is 2 or more, each R82 is the same as or different from each other, v is an integer from 0 to 5, and when v is 2 or more, each L4 is the same as or different from each other.

In addition, the present invention provides a composition for organic layer of an organic light emitting device comprising the heterocyclic compound represented by Formula 1 above and the heterocyclic compound represented by Formula 2 above.

In addition, the present invention provides a method for manufacturing an organic light emitting device comprising the steps of preparing a substrate; forming a first electrode on the substrate; forming one or more organic layers on the first electrode; and forming a second electrode on the one or more organic layers, wherein the step of forming the one or more organic layers comprises a step of forming one or more organic layers using the composition for organic layer of the organic light emitting device.

### [Advantageous Effect]

The compounds described herein can be used as a material for the organic layer of an organic light emitting device. The heterocyclic compound may serve as a material for a hole injection layer, a material for a hole transport layer, a material for a light emitting layer, a material for an electron transport layer, a material for an electron injection layer, and the like in an organic light emitting device. In particular, the heterocyclic compound can be used as a material for a light emitting layer of an organic light emitting device.

Specifically, the heterocyclic compound may be used alone as a light emitting material, and may be used as a host material or a dopant material of the light emitting layer. In addition, when the heterocyclic compound is used as a host material of a light emitting layer of an organic light emitting device, it is possible to lower the operating voltage of the device, improve the light efficiency, and improve the lifetime characteristics of the device.

### [Description of Drawing]

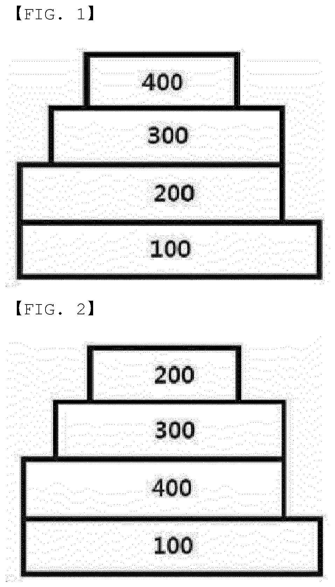

FIGs. 1 to 4 are views schematically showing a stacked structure of an organic light emitting device according to an embodiment of the present invention, respectively.

### [Best Mode]

Hereinafter, the present application will be described in detail.

In the present specification, when any part "comprises" any component, this may mean that other components may be further comprised, rather than excluding other components, unless otherwise stated.

In the present specification, the term "substituted" means that a hydrogen atom bonded to a carbon atom of a compound is replaced with another substituent, the position to be substituted is not limited as long as it is a position where a hydrogen atom may be substituted, that is, it is a position that can be substituted by a substituent, and when substituted by two or more substituents, two or more substituents may be the same as or different from each other.

In the present specification, the term "substituted or unsubstituted" means that it is substituted or unsubstituted by one or more substituents selected from the group consisting of deuterium; C1 to C60 straight or branched chain alkyl; C2 to C60 straight or branched alkenyl; C2 to C60 straight or branched alkynyl; C3 to C60 monocyclic or polycyclic cycloalkyl; C2 to C60 monocyclic or polycyclic heterocycloalkyl; C6 to C60 monocyclic or polycyclic aryl; C2 to C60 monocyclic or polycyclic heteroaryl; -SiRR'R"; -P(=O)RR'; C1 to C20 alkylamine; C6 to C60 monocyclic or polycyclic arylamine; and C2 to C60 monocyclic or polycyclic heteroarylamine, or means that it is substituted or unsubstituted with a substituent formed by connecting two or more substituents selected from the above-exemplified substituents.

More specifically, in the present specification, "substituted or unsubstituted" may means that it is substituted or unsubstituted with one or more substituents selected from the group consisting of a monocyclic or polycyclic aryl group having 6 to 60 carbon atoms or a monocyclic or polycyclic heteroaryl group having 2 to 60 carbon atoms.

In the present specification, the halogen may be fluorine, chlorine, bromine, or iodine.

In the present specification, the alkyl group comprises a straight or branched chain having 1 to 60 carbon atoms, and may be further substituted by other substituents. The number of carbon atoms in the alkyl group may be 1 to 60, specifically 1 to 40, more specifically, 1 to 20. Specific examples of the alkyl group may be, but is not limited to, a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethylpropyl group, a 1,1-dimethyl-propyl group, an isohexyl group, a 2-methylpentyl group, a 4-methylhexyl group, a 5-methylhexyl group and the like.

In the present specification, the alkenyl group comprises a straight or branched chain having 2 to 60 carbon atoms, and may be further substituted by other substituents. The carbon number of the alkenyl group may be 2 to 60, specifically 2 to 40, more specifically 2 to 20. Specific examples of the alkenyl group may be, but is not limited to, a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like.

In the present specification, the alkynyl group comprises a straight or branched chain having 2 to 60 carbon atoms, and may be further substituted by other substituents. The carbon number of the alkynyl group may be 2 to 60, specifically 2 to 40, more specifically 2 to 20.

In the present specification, the alkoxy group may be a straight-chain, a branched-chain, or a cyclic chain. Although the number of carbon atoms in the alkoxy group is not particularly limited, it is preferable that the number of carbon atoms is 1 to 20. Specific examples of the alkoxy group may be, but is not limited to, methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like.

In the present specification, the cycloalkyl group comprises a monocyclic or polycyclic ring having 3 to 60 carbon atoms, and may be further substituted by other substituents. In this case, the polycyclic ring refers to a group formed by directly connecting or condensing a cycloalkyl group with another cyclic group. In this case, the other cyclic group may be a cycloalkyl group, but may be a different type of cyclic group, for example, a heterocycloalkyl group, an aryl group, a heteroaryl group, or the like. The number of carbon atoms in the cycloalkyl group may be 3 to 60, specifically 3 to 40, more specifically 5 to 20. Specific examples of the cycloalkyl group may be, but is not limited to, a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like.

In the present specification, the heterocycloalkyl group comprises a monocyclic or polycyclic ring containing O, S, Se, N, or Si as a hetero atom and having 2 to 60 carbon atoms, and may be further substituted by other substituents. In this case, the polycyclic ring refers to a group formed by directly connecting or condensing a heterocycloalkyl group with another cyclic group. In this case, the other cyclic group may be a heterocycloalkyl group, but may be a different type of cyclic group, for example, a cycloalkyl group, an aryl group, a heteroaryl group, or the like. The number of carbon atoms in the heterocycloalkyl group may be 2 to 60, specifically 2 to 40, more specifically 3 to 20.

In the present specification, the aryl group comprises a monocyclic or polycyclic ring having 6 to 60 carbon atoms, and may be further substituted by other substituents. In this case, the polycyclic ring means a group formed by directly connecting or condensing an aryl group with another cyclic group. In this case, the other cyclic group may be an aryl group, but may be a different type of cyclic group, for example, a cycloalkyl group, a heterocycloalkyl group, a heteroaryl group, or the like. The aryl group comprises a spiro group. The number of carbon atoms in the aryl group may be 6 to 60, specifically 6 to 40, more specifically 6 to 25. Specific examples of the aryl group may be, but is not limited to, a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthyl group, a benzofluorenyl group, a spirobifluorenyl group, a 2,3-dihydro-1H-indenyl group, condensed ring groups thereof and the like.

In the present specification, the phosphine oxide group is represented by -P(=O)R101R102, wherein R101 and R102 are the same as or different from each other, and may each independently be a substituent consisting of at least one of hydrogen; deuterium; a halogen group; an alkyl group; an alkenyl group; an alkoxy group; a cycloalkyl group; an aryl group; and a heterocyclic group. Specifically, the phosphine oxide group is, but is not limited to, a diphenyl phosphine oxide group, dinaphthyl phosphine oxide group, or the like.

In the present specification, the silyl group contains Si and is a substituent formed by directly connecting the Si atoms as a radical, and is represented by -SiR104R105R106, wherein R104 to R106 are the same as or different from each other, and may each independently be a substituent consisting of at least one of hydrogen; deuterium; a halogen group; an alkyl group; an alkenyl group; an alkoxy group; a cycloalkyl group; an aryl group; and a heterocyclic group. Specific examples of the silyl group may be, but is not limited to, a trimethylsilyl group, a triethylsilyl group, a t-butyldimethylsilyl group, vinyldimethylsilyl group, a propyldimethylsilyl group, a triphenylsilyl group, a diphenylsilyl group, a phenylsilyl group and the like.

In the present specification, the fluorenyl group may be substituted, and adjacent substituents may be combined with each other to form a ring.

In the present specification, the spiro group is a group comprising a spiro structure, and may have 15 to 60 carbon atoms. For example, the spiro group may comprises a structure in which a 2,3-dihydro-1H-indene group or a cyclohexane group is spiro bonded to a fluorenyl group. Specifically, the following spiro group may comprise any one of the groups of Structural Formula below.

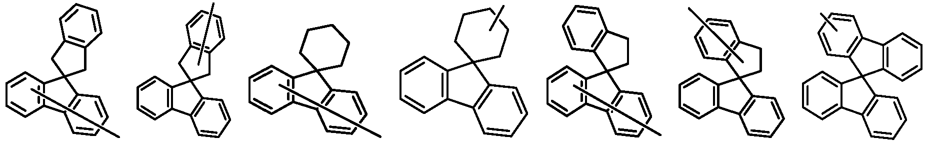

In the present specification, the heteroaryl group comprises a monocyclic or polycyclic ring containing S, O, Se, N, or Si as a hetero atom and having 2 to 60 carbon atoms, and may be further substituted by other substituents. In this case, the polycyclic ring refers to a group formed by directly connecting or condensing a heteroaryl group with another cyclic group. In this case, the other cyclic group may be a heteroaryl group, but may be a different type of cyclic group, for example, a cycloalkyl group, a heterocycloalkyl group, an aryl group, or the like. The number of carbon atoms in the heteroaryl group may be 2 to 60, specifically 2 to 40, more specifically 3 to 25. Specific examples of the heteroaryl group may be, but is not limited to, a pyridyl group, a pyrrolyl group, a pyrimidyl group, a pyridazinyl group, a furanyl group, a thiophenyl group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, tetrazinyl group, a quinolyl group, an isoquinolyl group, a quinazolinyl group, an isoquinazolinyl group, a quinazolylyl group, a naphthyridyl group, an acridinyl group, a phenanthridinyl group, an imidazopyridinyl group, a diazanaphthalenyl group, a triazaindenyl group, an indolyl group, an indolizinyl group, a benzothiazolyl group, a benzoxazolyl group, a benzimidazolyl group, a benzothiophenyl group, a benzofuranyl group, a dibenzothiophenyl group, a dibenzofuranyl group, a carbazolyl group, a benzocarbazolyl group, a dibenzocarbazolyl group, a phenazinyl group, a dibenzosilolyl group, spirobi(dibenzosilolyl), a dihydrophenazinyl group, a phenoxazinyl group, a phenanthridyl group, an imidazopyridinyl group, a thienyl group, an indolo[2,3-a] carbazolyl group, an indolo[2,3-b] carbazolyl group, an indolinyl group, a 10,11-dihydro-dibenzo[b,f]azepinyl group, a 9,10-dihydroacridinyl group, a phenanthrazinyl group, a phenothiazinyl group, phthalazinyl group, a naphthylidinyl group, a phenanthrolinyl group, a benzo[c] [1,2,5]thiadiazolyl group, 5,10-dihydrodibenzo[b,e] [1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and the number of carbon atoms of the amine group is not particularly limited, but is preferably 1 to 30. Specific examples of the amine group may be, but is not limited to, a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like.

In the present specification, the arylene group means that the aryl group has two bonding positions, that is, a divalent group. These are the same as those described for the aryl group described above, except that each of them is a divalent group. In addition, the heteroarylene group means that the heteroaryl group has two bonding positions, that is, a divalent group. These are the same as those described for the heteroaryl group described above, except that each of them is a divalent group.

In the present specification, an "adjacent" group may mean a substituent substituted at the atom directly linked to the atom substituted by that substituent, a substituent that is sterically closest to that substituent, or another substituent substituted at the atom substituted by that substituent. For example, two substituents substituted at an ortho position in a benzene ring and two substituents substituted at the same carbon in an aliphatic ring may be interpreted as "adjacent" groups to each other.

In the present invention, "when a substituent is not indicated in the structure of the formula or the compound" means that a hydrogen atom is bonded to a carbon atom. However, since deuterium (²H) is an isotope of hydrogen, some hydrogen atoms may be deuterium.

In one embodiment of the present invention, "when a substituent is not indicated in the structure of the formula or the compound" may mean that all positions that may be substituted by the substituent are hydrogen or deuterium. That is, in the case of deuterium, some hydrogen atoms as an isotope of hydrogen may be deuterium that is an isotope. In this case, the content of deuterium may be 0% to 100%.

In one embodiment of the present invention, in the case of "when a substituent is not indicated in the structure of the formula or the compound", if deuterium is not explicitly excluded, such as "the content of deuterium is 0%", "the content of hydrogen is 100%", "all substituents are hydrogen" etc., hydrogen and deuterium may be used in admixture in the compound.

In one embodiment of the present invention, deuterium is one of the isotopes of hydrogen, and is an element having as an atomic nucleus a deuteron consisting of one proton and one neutron, and can be expressed as hydrogen-2, and the element symbol can also be written as D or ²H.

In one embodiment of the present invention, an isotope means an atom with the same atomic number (Z) but a different mass number (A), and can also be interpreted as an element that has the same number of protons but the different number of neutrons.

In one embodiment of the present invention, the meaning of content T% of a specific substituent may be defined as T2/T1X100 = T% wherein, T1 is defined as the total number of substituents that the basic compound can have, and T2 is defined as the number of specific substituents substituted among them.

That is, in one example, the 20% content of deuterium in the phenyl group represented by 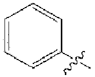 may mean that the total number of substituents that the phenyl group can have is 5 (T1 in the formula), and the number of deuterium among them is 1 (T2 in the formula). That is, in the phenyl group, that the content of deuterium is 20% may be represented by Structural Formulas below:

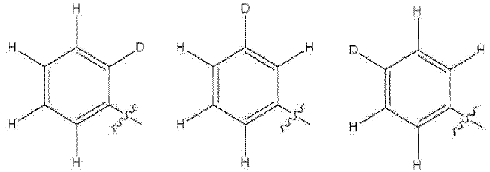

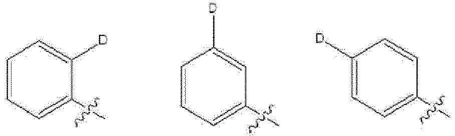

Also, in one embodiment of the present invention, in the case of "a phenyl group having a deuterium content of 0%", it means a phenyl group that does not contain a deuterium atom, i.e., has 5 hydrogen atoms.

In the present invention, the content of deuterium in the heterocyclic compound represented by Formula 1 may be 0 to 100%, more preferably 30 to 100%.

In the present invention, C6 to C60 aromatic hydrocarbon ring means a compound containing an aromatic ring consisting of C6 to C60 carbons and hydrogens, and for example, may be, but is not limited to, benzene, biphenyl, terphenyl, triphenylene, naphthalene, anthracene, phenalene, phenanthrene, fluorene, pyrene, chrysene, perylene, azulene and the like, and comprises all of the aromatic hydrocarbon ring compounds known in the art as satisfying the carbon number described above.

The present invention provides a heterocyclic compound represented by the following Formula 1: 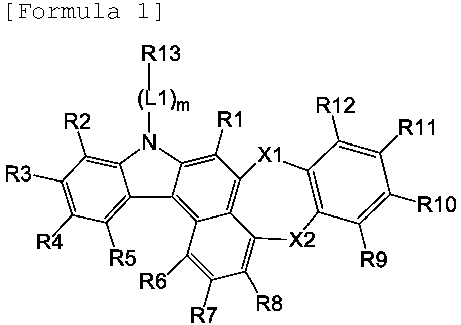 wherein,
one of X1 and X2 is O or S, and the other is a direct bond,
L1 is a single bond; a substituted or unsubstituted C6 to C60 arylene group; or a substituted or unsubstituted C2 to C60 heteroarylene group,
m is an integer from 0 to 5, and when m is 2 or more, each L1 is the same as or different from each other, and
R1 to R13 are the same as or different from each other and are each independently selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; - P(=O)R101R102; -SiR101R102R103; and -NR101R102, or two or more groups adjacent to each other are combined with each other to form a substituted or unsubstituted C6 to C60 aromatic hydrocarbon ring or a substituted or unsubstituted C2 to C60 heterocyclic ring, wherein R101, R102, and R103 are the same as or different from each other and are each independently a substituted or unsubstituted C1 to C60 alkyl group; a substituted or unsubstituted C6 to C60 aryl group; or a substituted or unsubstituted C2 to C60 heteroaryl group.

In one embodiment of the present invention, one of X1 and X2 may be O or S, and the other may be a direct bond.

In other embodiment of the present invention, X1 may be O, and X2 may be a direct bond.

In other embodiment of the present invention, X1 may be S, and X2 may be a direct bond.

In other embodiment of the present invention, X2 may be O, and X1 may be a direct bond.

In other embodiment of the present invention, X2 may be S, and X1 may be a direct bond.

In one embodiment of the present invention, L1 may be a single bond, a substituted or unsubstituted C6 to C60 arylene group or a substituted or unsubstituted C2 to C60 heteroarylene group.

In other embodiment of the present invention, L1 may be a single bond, a substituted or unsubstituted C6 to C40 arylene group or a substituted or unsubstituted C2 to C40 heteroarylene group.

In other embodiment of the present invention, L1 may be a single bond, a substituted or unsubstituted C6 to C20 arylene group or a substituted or unsubstituted C2 to C20 heteroarylene group.

In other embodiment of the present invention, L1 may be selected from the group consisting of a single bond, substituted or unsubstituted phenylene, biphenylene, naphthalene, anthracenylene, pyrene, phenanthrene, pyridine, pyrimidine, triazine, quinoline, and quinazoline.

In other embodiment of the present invention, L1 may be selected from the group consisting of a single bond, substituted or unsubstituted phenylene, pyrimidine, triazine, quinoline, and quinazoline.

In other embodiment of the present invention, L1 may be a single bond.

In other embodiment of the present invention, L1 may be substituted or unsubstituted phenylene.

In one embodiment of the present invention, m may be an integer of 0 to 5, and when m is 2 or more, each L1 may be the same as or different from each other.

In one embodiment of the present invention, R1 to R13 may be the same as or different from each other, and may each independently be selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; -P(=O)R101R102; - SiR101R102R103; and -NR101R102, or two or more groups adjacent to each other may be combined with each other to form a substituted or unsubstituted C6 to C60 aromatic hydrocarbon ring or a substituted or unsubstituted C2 to C60 heterocycle, wherein R101, R102, and R103 may be the same as or different from each other, and may each independently be a substituted or unsubstituted C1 to C60 alkyl group; a substituted or unsubstituted C6 to C60 aryl group; or a substituted or unsubstituted C2 to C60 heteroaryl group.

In other embodiment of the present invention, R1 to R13 may be the same as or different from each other, and may each independently be selected from the group consisting of hydrogen; deuterium; halogen; a cyano group; a nitro group; a substituted or unsubstituted C1 to C40 alkyl group; a substituted or unsubstituted C2 to C40 alkenyl group; a substituted or unsubstituted C2 to C40 alkynyl group; a substituted or unsubstituted C3 to C40 cycloalkyl group; a substituted or unsubstituted C2 to C40 heterocycloalkyl group; a substituted or unsubstituted C6 to C40 aryl group; a substituted or unsubstituted C2 to C40 heteroaryl group; a substituted or unsubstituted C1 to C40 alkoxy group; a substituted or unsubstituted C6 to C40 aryloxy group; -P(=O)R101R102; - SiR101R102R103; and -NR101R102, or two or more groups adjacent to each other may be combined with each other to form a substituted or unsubstituted C6 to C40 aromatic hydrocarbon ring or a substituted or unsubstituted C2 to C40 heterocycle, wherein R101, R102, and R103 may be the same as or different from each other, and may each independently be a substituted or unsubstituted C1 to C40 alkyl group; a substituted or unsubstituted C6 to C40 aryl group; or a substituted or unsubstituted C2 to C40 heteroaryl group.

In other embodiment of the present invention, R1 to R13 may be the same as or different from each other, and may each independently be selected from the group consisting of hydrogen; deuterium; halogen; a cyano group; a nitro group; a substituted or unsubstituted C1 to C20 alkyl group; a substituted or unsubstituted C2 to C20 alkenyl group; a substituted or unsubstituted C2 to C20 alkynyl group; a substituted or unsubstituted C3 to C20 cycloalkyl group; a substituted or unsubstituted C2 to C20 heterocycloalkyl group; a substituted or unsubstituted C6 to C20 aryl group; a substituted or unsubstituted C2 to C20 heteroaryl group; a substituted or unsubstituted C1 to C20 alkoxy group; a substituted or unsubstituted C6 to C20 aryloxy group; -P(=O)R101R102; - SiR101R102R103; and -NR101R102, or two or more groups adjacent to each other may be combined with each other to form a substituted or unsubstituted C6 to C20 aromatic hydrocarbon ring or a substituted or unsubstituted C2 to C20 heterocycle, and R101, R102, and R103 may be the same as or different from each other, and may each independently be a substituted or unsubstituted C1 to C20 alkyl group; a substituted or unsubstituted C6 to C20 aryl group; or a substituted or unsubstituted C2 to C20 heteroaryl group.

In other embodiment of the present invention, R1 to R13 may be the same as or different from each other, and may each independently be hydrogen; deuterium; halogen; a cyano group; a nitro group; a substituted or unsubstituted C1 to C20 alkyl group; a substituted or unsubstituted C2 to C20 alkenyl group; a substituted or unsubstituted C2 to C20 alkynyl group; a substituted or unsubstituted C6 to C20 aryl group; or a substituted or unsubstituted C2 to C20 heteroaryl group.

In other embodiment of the present invention, R1 to R13 may be the same as or different from each other, and may each independently be hydrogen; deuterium; halogen; a cyano group; a nitro group; a substituted or unsubstituted C1 to C10 alkyl group; a substituted or unsubstituted C2 to C10 alkenyl group; a substituted or unsubstituted C2 to C10 alkynyl group; a substituted or unsubstituted C6 to C10 aryl group; or a substituted or unsubstituted C2 to C10 heteroaryl group.

In other embodiment of the present invention, R1 to R13 may be the same as or different from each other, and may each independently be hydrogen; deuterium; a substituted or unsubstituted C1 to C10 alkyl group; a substituted or unsubstituted C2 to C10 alkenyl group; a substituted or unsubstituted C2 to C10 alkynyl group; a substituted or unsubstituted C6 to C10 aryl group; or a substituted or unsubstituted C2 to C10 heteroaryl group.

In other embodiment of the present invention, R1 to R13 may be the same as or different from each other, and may each independently be hydrogen; deuterium; a substituted or unsubstituted C6 to C20 aryl group; or a substituted or unsubstituted C2 to C20 heteroaryl group.

In other embodiment of the present invention, R1 to R13 may be the same as or different from each other, and may each independently be hydrogen; deuterium; a substituted or unsubstituted C6 to C10 aryl group; or a substituted or unsubstituted C2 to C10 heteroaryl group.

In other embodiment of the present invention, R1 to R13 may be the same as or different from each other, and may each independently be hydrogen; deuterium; substituted or unsubstituted phenyl, naphthyl, pyridinyl, anthracenyl, carbazolyl, dibenzothiophenyl, dibenzofuranyl, or phenanthrenyl group.

In other embodiment of the present invention, R1 to R13 may be the same as or different from each other, and may each independently be hydrogen; deuterium; substituted or unsubstituted carbazolyl, dibenzothiophenyl, or dibenzofuranyl group.

In other embodiment of the present invention, R1 to R13 may be the same as or different from each other, and may each independently be hydrogen; deuterium; a substituted or unsubstituted carbazolyl group.

In one embodiment of the present invention, Formula 1 above may be represented by Formula 1-1 or Formula 1-2 below: 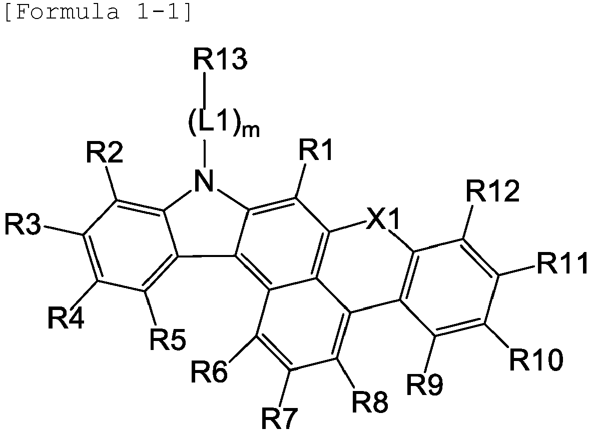 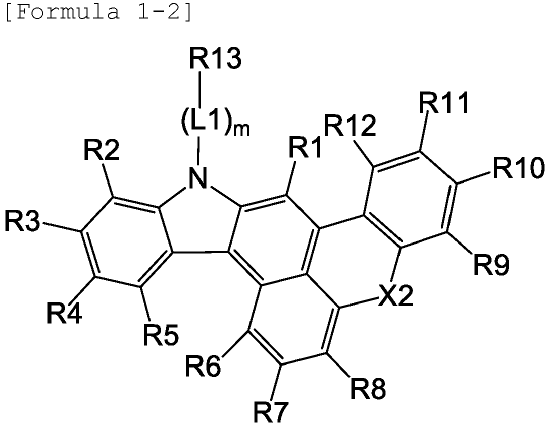 wherein X1, X2, L1, R1 to R13, and m are the same as defined in Formula 1 above.

In other embodiment of the present invention, Formula 1 above may be represented by Formula 1-1 above.

In other embodiment of the present invention, Formula 1 may be represented by Formula 1-2 above.

In one embodiment of the present invention, any one of R1 to R13 may be represented by Structural Formula A or Structural Formula B below: 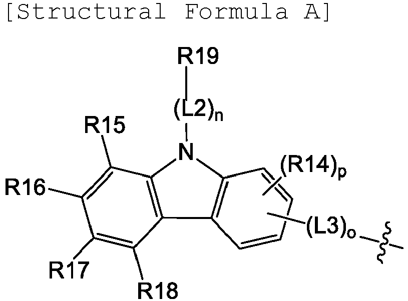 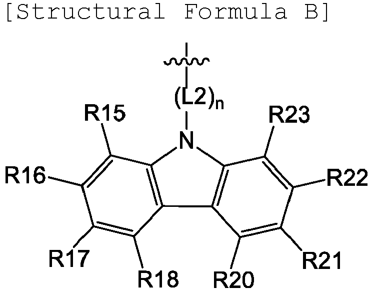 wherein,
L2 and L3 are the same as or different from each other, and are each independently a single bond; a substituted or unsubstituted C6 to C60 arylene group; or a substituted or unsubstituted C2 to C60 heteroarylene group,
n is an integer from 0 to 5, and when n is 2 or more, each L2 is the same as or different from each other, o is an integer from 0 to 5, and when o is 2 or more, each L3 is the same as or different from each other, p is an integer from 0 to 3, and when p is 2 or more, each R14 is the same as or different from each other,
R14 to R23 are the same as or different from each other, and are each independently selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; - P(=O)R101R102; -SiR101R102R103; and -NR101R102, or two or more groups adjacent to each other are combined with each other to form a substituted or unsubstituted C6 to C60 aromatic hydrocarbon ring or a substituted or unsubstituted C2 to C60 heterocycle, and R101, R102, and R103 are the same as or different from each other, and are each independently a substituted or unsubstituted C1 to C60 alkyl group; a substituted or unsubstituted C6 to C60 aryl group; or a substituted or unsubstituted C2 to C60 heteroaryl group.

In one embodiment of the present invention, L2 and L3 may be the same as or different from each other, and may each independently be a single bond; a substituted or unsubstituted C6 to C60 arylene group; or a substituted or unsubstituted C2 to C60 heteroarylene group.

In other embodiment of the present invention, L2 and L3 may be the same as or different from each other, and may each independently be a single bond, a substituted or unsubstituted C6 to C40 arylene group, or a substituted or unsubstituted C2 to C40 heteroarylene group.

In other embodiment of the present invention, L2 and L3 may be the same as or different from each other, and may each independently be a single bond, a substituted or unsubstituted C6 to C20 arylene group, or a substituted or unsubstituted C2 to C20 heteroarylene group.

In other embodiment of the present invention, L2 and L3 may be the same as or different from each other, and may each independently be selected from the group consisting of a single bond, substituted or unsubstituted phenylene, biphenylene, naphthalene, anthracenylene, pyrene, phenanthrene, pyridine, pyrimidine, triazine, quinoline, and quinazoline.

In other embodiment of the present invention, L2 and L3 may be the same as or different from each other, and may each independently be selected from the group consisting of a single bond, substituted or unsubstituted phenylene, pyrimidine, triazine, quinoline, and quinazoline.

In other embodiment of the present invention, L2 and L3 may be the same as or different from each other, and may each independently be a single bond.

In other embodiment of the present invention, L2 and L3 may be the same as or different from each other, and may each independently be a substituted or unsubstituted phenylene.

In one embodiment of the present invention, n above may be an integer from 0 to 5, and when n is 2 or more, each L2 may be the same as or different from each other, o may be an integer from 0 to 5, and when o is 2 or more, each L3 may be the same as or different from each other, p may be an integer of 0 to 3, and when p is 2 or more, each R14 may be the same as or different from each other.

In one embodiment of the present invention, R14 to R23 may be the same as or different from each other, and may each independently be selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; -P(=O)R101R102; - SiR101R102R103; and -NR101R102, or two or more groups adjacent to each other may be combined with each other to form a substituted or unsubstituted C6 to C60 aromatic hydrocarbon ring or a substituted or unsubstituted C2 to C60 heterocycle, wherein R101, R102, and R103 may be the same as or different from each other, and may each independently be a substituted or unsubstituted C1 to C60 alkyl group; a substituted or unsubstituted C6 to C60 aryl group; or a substituted or unsubstituted C2 to C60 heteroaryl group.

In other embodiment of the present invention, R14 to R23 may be the same as or different from each other, and may each independently be selected from the group consisting of hydrogen; deuterium; halogen; a cyano group; a nitro group; a substituted or unsubstituted C1 to C40 alkyl group; a substituted or unsubstituted C2 to C40 alkenyl group; a substituted or unsubstituted C2 to C40 alkynyl group; a substituted or unsubstituted C3 to C40 cycloalkyl group; a substituted or unsubstituted C2 to C40 heterocycloalkyl group; a substituted or unsubstituted C6 to C40 aryl group; a substituted or unsubstituted C2 to C40 heteroaryl group; a substituted or unsubstituted C1 to C40 alkoxy group; a substituted or unsubstituted C6 to C40 aryloxy group; -P(=O)R101R102; - SiR101R102R103; and -NR101R102, or two or more groups adjacent to each other may be combined with each other to form a substituted or unsubstituted C6 to C40 aromatic hydrocarbon ring or a substituted or unsubstituted C2 to C40 heterocycle, wherein R101, R102, and R103 may be the same as or different from each other, and may each independently be a substituted or unsubstituted C1 to C40 alkyl group; a substituted or unsubstituted C6 to C40 aryl group; or a substituted or unsubstituted C2 to C40 heteroaryl group.

In other embodiment of the present invention, R14 to R23 may be the same as or different from each other, and may each independently be selected from the group consisting of hydrogen; deuterium; halogen; a cyano group; a nitro group; a substituted or unsubstituted C1 to C20 alkyl group; a substituted or unsubstituted C2 to C20 alkenyl group; a substituted or unsubstituted C2 to C20 alkynyl group; a substituted or unsubstituted C3 to C20 cycloalkyl group; a substituted or unsubstituted C2 to C20 heterocycloalkyl group; a substituted or unsubstituted C6 to C20 aryl group; a substituted or unsubstituted C2 to C20 heteroaryl group; a substituted or unsubstituted C1 to C20 alkoxy group; a substituted or unsubstituted C6 to C20 aryloxy group; -P(=O)R101R102; - SiR101R102R103; and -NR101R102, or two or more groups adjacent to each other may be combined with each other to form a substituted or unsubstituted C6 to C20 aromatic hydrocarbon ring or a substituted or unsubstituted C2 to C20 heterocycle, wherein R101, R102, and R103 may be the same as or different from each other, and may each independently be a substituted or unsubstituted C1 to C20 alkyl group; a substituted or unsubstituted C6 to C20 aryl group; or a substituted or unsubstituted C2 to C20 heteroaryl group.

In other embodiment of the present invention, R14 to R23 may be the same as or different from each other, and may each independently be hydrogen; deuterium; halogen; a cyano group; a nitro group; a substituted or unsubstituted C1 to C20 alkyl group; a substituted or unsubstituted C2 to C20 alkenyl group; a substituted or unsubstituted C2 to C20 alkynyl group; a substituted or unsubstituted C6 to C20 aryl group; or a substituted or unsubstituted C2 to C20 heteroaryl group.

In other embodiment of the present invention, R14 to R23 may be the same as or different from each other, and may each independently be hydrogen; deuterium; halogen; a cyano group; a nitro group; a substituted or unsubstituted C1 to C10 alkyl group; a substituted or unsubstituted C2 to C10 alkenyl group; a substituted or unsubstituted C2 to C10 alkynyl group; a substituted or unsubstituted C6 to C10 aryl group; or a substituted or unsubstituted C2 to C10 heteroaryl group.

In other embodiment of the present invention, R14 to R23 may be the same as or different from each other, and may each independently be hydrogen; deuterium; a substituted or unsubstituted C1 to C10 alkyl group; a substituted or unsubstituted C2 to C10 alkenyl group; a substituted or unsubstituted C2 to C10 alkynyl group; a substituted or unsubstituted C6 to C10 aryl group; or a substituted or unsubstituted C2 to C10 heteroaryl group.

In other embodiment of the present invention, R14 to R23 may be the same as or different from each other, and may each independently be hydrogen; deuterium; a substituted or unsubstituted C6 to C20 aryl group; or a substituted or unsubstituted C2 to C20 heteroaryl group.

In other embodiment of the present invention, R14 to R23 may be the same as or different from each other, and may each independently be hydrogen; deuterium; a substituted or unsubstituted C6 to C10 aryl group; or a substituted or unsubstituted C2 to C10 heteroaryl group.

In other embodiment of the present invention, R14 to R23 may be the same as or different from each other, and may each independently be hydrogen; deuterium; a substituted or unsubstituted phenyl, naphthyl, pyridinyl, anthracenyl, or phenanthrenyl group.

In other embodiment of the present invention, R14 to R23 may be the same as or different from each other, and may each independently be hydrogen; deuterium; a substituted or unsubstituted phenyl group.

In one embodiment of the present invention, Structural Formula A above may be represented by any one of Structural Formula A-1 to Structural Formula A-4 below: 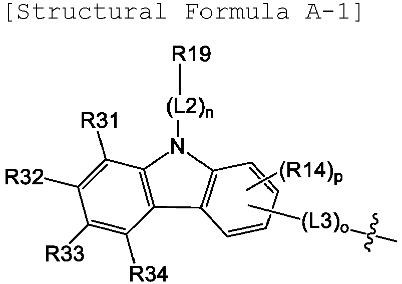 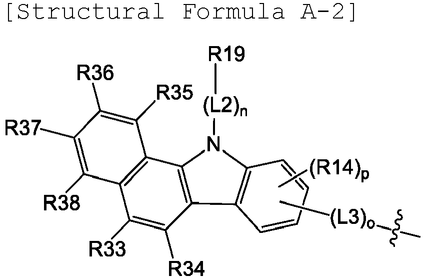 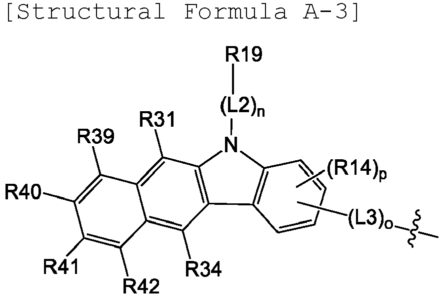 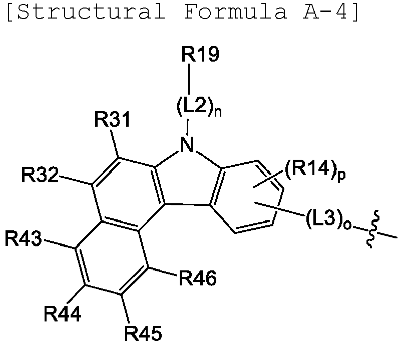 wherein,
R31 to R46 are the same as or different from each other, and are each independently selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; - P(=O)R101R102; -SiR101R102R103; and -NR101R102, or two or more groups adjacent to each other are combined with each other to form a substituted or unsubstituted C6 to C60 aromatic hydrocarbon ring or a substituted or unsubstituted C2 to C60 heterocycle, wherein R101, R102, and R103 are the same as or different from each other, and are each independently a substituted or unsubstituted C1 to C60 alkyl group; a substituted or unsubstituted C6 to C60 aryl group; or a substituted or unsubstituted C2 to C60 heteroaryl group, and
L2, L3, R14, R19, n, o, and p are the same as defined in Structural Formula A above.

In one embodiment of the present invention, R31 to R46 may be the same as or different from each other, and may each independently be selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; -P(=O)R101R102; - SiR101R102R103; and -NR101R102, or two or more groups adjacent to each other may be combined with each other to form a substituted or unsubstituted C6 to C60 aromatic hydrocarbon ring or a substituted or unsubstituted C2 to C60 heterocycle, wherein R101, R102, and R103 may be the same as or different from each other, and may each independently be a substituted or unsubstituted C1 to C60 alkyl group; a substituted or unsubstituted C6 to C60 aryl group; or a substituted or unsubstituted C2 to C60 heteroaryl group.

In other embodiment of the present invention, R31 to R46 may be the same as or different from each other, and may each independently be selected from the group consisting of hydrogen; deuterium; halogen; a cyano group; a nitro group; a substituted or unsubstituted C1 to C40 alkyl group; a substituted or unsubstituted C2 to C40 alkenyl group; a substituted or unsubstituted C2 to C40 alkynyl group; a substituted or unsubstituted C3 to C40 cycloalkyl group; a substituted or unsubstituted C2 to C40 heterocycloalkyl group; a substituted or unsubstituted C6 to C40 aryl group; a substituted or unsubstituted C2 to C40 heteroaryl group; a substituted or unsubstituted C1 to C40 alkoxy group; a substituted or unsubstituted C6 to C40 aryloxy group; -P(=O)R101R102; - SiR101R102R103; and -NR101R102, or two or more groups adjacent to each other may be combined with each other to form a substituted or unsubstituted C6 to C40 aromatic hydrocarbon ring or a substituted or unsubstituted C2 to C40 heterocycle, wherein R101, R102, and R103 may be the same as or different from each other, and may each independently be a substituted or unsubstituted C1 to C40 alkyl group; a substituted or unsubstituted C6 to C40 aryl group; or a substituted or unsubstituted C2 to C40 heteroaryl group.

In other embodiment of the present invention, R31 to R46 may be the same as or different from each other, and may each independently be selected from the group consisting of hydrogen; deuterium; halogen; a cyano group; a nitro group; a substituted or unsubstituted C1 to C20 alkyl group; a substituted or unsubstituted C2 to C20 alkenyl group; a substituted or unsubstituted C2 to C20 alkynyl group; a substituted or unsubstituted C3 to C20 cycloalkyl group; a substituted or unsubstituted C2 to C20 heterocycloalkyl group; a substituted or unsubstituted C6 to C20 aryl group; a substituted or unsubstituted C2 to C20 heteroaryl group; a substituted or unsubstituted C1 to C20 alkoxy group; a substituted or unsubstituted C6 to C20 aryloxy group; -P(=O)R101R102; - SiR101R102R103; and -NR101R102, or two or more groups adjacent to each other may be combined with each other to form a substituted or unsubstituted C6 to C20 aromatic hydrocarbon ring or a substituted or unsubstituted C2 to C20 heterocycle, wherein R101, R102, and R103 may be the same as or different from each other, and may each independently be a substituted or unsubstituted C1 to C20 alkyl group; a substituted or unsubstituted C6 to C20 aryl group; or a substituted or unsubstituted C2 to C20 heteroaryl group.

In other embodiment of the present invention, R31 to R46 may be the same as or different from each other, and may each independently be hydrogen; deuterium; halogen; a cyano group; a nitro group; a substituted or unsubstituted C1 to C20 alkyl group; a substituted or unsubstituted C2 to C20 alkenyl group; a substituted or unsubstituted C2 to C20 alkynyl group; a substituted or unsubstituted C6 to C20 aryl group; or a substituted or unsubstituted C2 to C20 heteroaryl group.

In other embodiment of the present invention, R31 to R46 may be the same as or different from each other, and may each independently be hydrogen; deuterium; halogen; a cyano group; a nitro group; a substituted or unsubstituted C1 to C10 alkyl group; a substituted or unsubstituted C2 to C10 alkenyl group; a substituted or unsubstituted C2 to C10 alkynyl group; a substituted or unsubstituted C6 to C10 aryl group; or a substituted or unsubstituted C2 to C10 heteroaryl group.

In other embodiment of the present invention, R31 to R46 may be the same as or different from each other, and may each independently be hydrogen; deuterium; a substituted or unsubstituted C1 to C10 alkyl group; a substituted or unsubstituted C2 to C10 alkenyl group; a substituted or unsubstituted C2 to C10 alkynyl group; a substituted or unsubstituted C6 to C10 aryl group; or a substituted or unsubstituted C2 to C10 heteroaryl group.

In other embodiment of the present invention, R31 to R46 may be the same as or different from each other, and may each independently be hydrogen; deuterium; a substituted or unsubstituted C6 to C20 aryl group; or a substituted or unsubstituted C2 to C20 heteroaryl group.

In other embodiment of the present invention, R31 to R46 may be the same as or different from each other, and may each independently be hydrogen; deuterium; a substituted or unsubstituted C6 to C10 aryl group; or a substituted or unsubstituted C2 to C10 heteroaryl group.

In other embodiment of the present invention, R31 to R46 may be the same as or different from each other, and may each independently be hydrogen; deuterium; a substituted or unsubstituted phenyl, naphthyl, pyridinyl, anthracenyl, or phenanthrenyl group.

In other embodiment of the present invention, R31 to R46 may be the same as or different from each other, and may each independently be hydrogen; deuterium; a substituted or unsubstituted phenyl group.

In one embodiment of the present invention, R19 above may be represented by any one of Structural Formula A-5-1 to Structural Formula A-5-5 below: 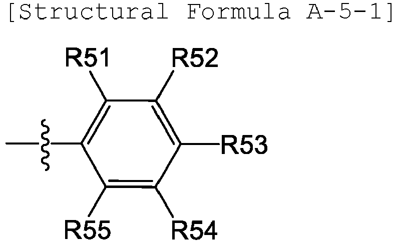 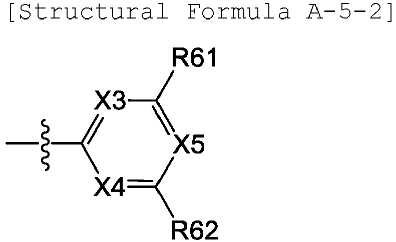 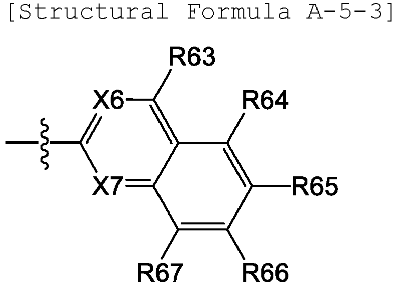 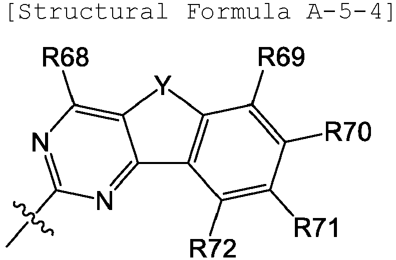 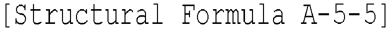 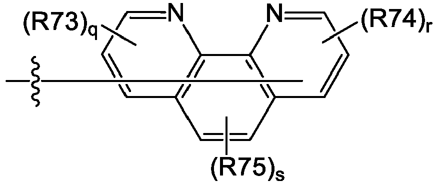 wherein,
X3 is N or CRa, X4 is N or CRb, X5 is N or CRc, X6 is N or CRd, and X7 is N or CRe,
Y is O or S,
R51 to R55, R61 to R75, and Ra to Re are the same as or different from each other, and are each independently selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; - P(=O)R101R102; -SiR101R102R103; and -NR101R102, or two or more groups adjacent to each other may be combined with each other to form a substituted or unsubstituted C6 to C60 aromatic hydrocarbon ring or a substituted or unsubstituted C2 to C60 heterocycle, wherein R101, R102, and R103 are the same as or different from each other, and are each independently a substituted or unsubstituted C1 to C60 alkyl group; a substituted or unsubstituted C6 to C60 aryl group; or a substituted or unsubstituted C2 to C60 heteroaryl group,
q is an integer from 0 to 3, and when q is 2 or more, each R73 is the same as or different from each other, r is an integer of 0 to 3, and when r is 2 or more, each R74 is the same as or different from each other, s is an integer from 0 to 2, and when s is 2, each R75 is the same as or different from each other.

In one embodiment of the present invention, X3 may be N or CRa, X4 may be N or CRb, X5 may be N or CRc, X6 may be N or CRd, and X7 may be N or CRe.

In other embodiment of the present invention, X3 may be N or CRa, X4 may be N or CRb, and X5 may be N or CRc.

In other embodiment of the present invention, X3, X4 and X5 may be N.

In other embodiment of the present invention, X3 and X4 may be N, and X5 may be CRc.

In other embodiment of the present invention, X4 and X5 may be N, and X3 may be CRa.

In other embodiment of the present invention, X5 and X3 may be N, and X4 may be CRb.

In other embodiment of the present invention, X3 may be N, and X4 may be CRb, and X5 may be CRc.

In other embodiment of the present invention, X4 may be N, X5 may be CRc, and X3 may be CRa.

In other embodiment of the present invention, X5 may be N, X3 may be CRa, and X4 may be CRb.

In other embodiment of the present invention, X3 may be CRa, X4 may be CRb, and X5 may be CRc.

In other embodiment of the present invention, X6 may be N or CRd, and X7 may be N or CRe.

In other embodiment of the present invention, X6 and X7 may be N.

In other embodiment of the present invention, X6 may be N, and X7 may be CRe.

In other embodiment of the present invention, X7 may be N, and X6 may be CRd.

In other embodiment of the present invention, X6 may be CRd, and X7 may be CRe.

In one embodiment of the present invention, Y may be O or S.

In other embodiment of the present invention, Y may be O.

In other embodiment of the present invention, Y may be S.

In one embodiment of the present invention, R51 to R55, R61 to R75, and Ra to Re may be the same as or different from each other, and may each independently be selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; - P(=O)R101R102; -SiR101R102R103; and -NR101R102, or two or more groups adjacent to each other may be combined with each other to form a substituted or unsubstituted C6 to C60 aromatic hydrocarbon ring or a substituted or unsubstituted C2 to C60 heterocycle, wherein R101, R102, and R103 may be the same as or different from each other, and may each independently be a substituted or unsubstituted C1 to C60 alkyl group; a substituted or unsubstituted C6 to C60 aryl group; or a substituted or unsubstituted C2 to C60 heteroaryl group.

In other embodiment of the present invention, R51 to R55, R61 to R75, and Ra to Re may be the same as or different from each other, and may be each independently selected from the group consisting of hydrogen; deuterium; halogen; a cyano group; a nitro group; a substituted or unsubstituted C1 to C40 alkyl group; a substituted or unsubstituted C2 to C40 alkenyl group; a substituted or unsubstituted C2 to C40 alkynyl group; a substituted or unsubstituted C3 to C40 cycloalkyl group; a substituted or unsubstituted C2 to C40 heterocycloalkyl group; a substituted or unsubstituted C6 to C40 aryl group; a substituted or unsubstituted C2 to C40 heteroaryl group; a substituted or unsubstituted C1 to C40 alkoxy group; a substituted or unsubstituted C6 to C40 aryloxy group; - P(=O)R101R102; -SiR101R102R103; and -NR101R102, or two or more groups adjacent to each other may be combined with each other to form a substituted or unsubstituted C6 to C40 aromatic hydrocarbon ring or a substituted or unsubstituted C2 to C40 heterocycle, wherein R101, R102, and R103 may be the same as or different from each other, and may each independently be a substituted or unsubstituted C1 to C40 alkyl group; a substituted or unsubstituted C6 to C40 aryl group; or a substituted or unsubstituted C2 to C40 heteroaryl group.

In other embodiment of the present invention, R51 to R55, R61 to R75, and Ra to Re may be the same as or different from each other, and may each independently be selected from the group consisting of hydrogen; deuterium; halogen; a cyano group; a nitro group; a substituted or unsubstituted C1 to C20 alkyl group; a substituted or unsubstituted C2 to C20 alkenyl group; a substituted or unsubstituted C2 to C20 alkynyl group; a substituted or unsubstituted C3 to C20 cycloalkyl group; a substituted or unsubstituted C2 to C20 heterocycloalkyl group; a substituted or unsubstituted C6 to C20 aryl group; a substituted or unsubstituted C2 to C20 heteroaryl group; a substituted or unsubstituted C1 to C20 alkoxy group; a substituted or unsubstituted C6 to C20 aryloxy group; - P(=O)R101R102; -SiR101R102R103; and -NR101R102, or two or more groups adjacent to each other may be combined with each other to form a substituted or unsubstituted C6 to C20 aromatic hydrocarbon ring or a substituted or unsubstituted C2 to C20 heterocycle, wherein R101, R102, and R103 may be the same as or different from each other, and may each independently be a substituted or unsubstituted C1 to C20 alkyl group; a substituted or unsubstituted C6 to C20 aryl group; or a substituted or unsubstituted C2 to C20 heteroaryl group.

In other embodiment of the present invention, R51 to R55, R61 to R75, and Ra to Re may be the same as or different from each other, and may each independently be selected from the group consisting of hydrogen; deuterium; halogen; a cyano group; a nitro group; a substituted or unsubstituted C1 to C20 alkyl group; a substituted or unsubstituted C2 to C20 alkenyl group; a substituted or unsubstituted C2 to C20 alkynyl group; a substituted or unsubstituted C6 to C20 aryl group; or a substituted or unsubstituted C2 to C20 heteroaryl group, or two or more groups adjacent to each other may be combined with each other to form a substituted or unsubstituted C6 to C20 aromatic hydrocarbon ring or a substituted or unsubstituted C2 to C20 heterocycle.

In other embodiment of the present invention, R51 to R55, R61 to R75, and Ra to Re may be the same as or different from each other, and may each independently be selected from the group consisting of hydrogen; deuterium; halogen; a cyano group; a nitro group; a substituted or unsubstituted C1 to C10 alkyl group; a substituted or unsubstituted C2 to C10 alkenyl group; a substituted or unsubstituted C2 to C10 alkynyl group; a substituted or unsubstituted C6 to C10 aryl group; or a substituted or unsubstituted C2 to C10 heteroaryl group, or two or more groups adjacent to each other may be combined with each other to form a substituted or unsubstituted C6 to C10 aromatic hydrocarbon ring or a substituted or unsubstituted C2 to C10 heterocycle.

In other embodiment of the present invention, R51 to R55, R61 to R75, and Ra to Re may be the same as or different from each other, and may each independently be hydrogen; deuterium; a substituted or unsubstituted C1 to C10 alkyl group; a substituted or unsubstituted C2 to C10 alkenyl group; a substituted or unsubstituted C2 to C10 alkynyl group; a substituted or unsubstituted C6 to C10 aryl group; or a substituted or unsubstituted C2 to C10 heteroaryl group.

In other embodiment of the present invention, R51 to R55, R61 to R75, and Ra to Re may be the same as or different from each other, and may each independently be hydrogen; deuterium; a substituted or unsubstituted C6 to C20 aryl group; or a substituted or unsubstituted C2 to C20 heteroaryl group.

In other embodiment of the present invention, R51 to R55, R61 to R75, and Ra to Re may be the same as or different from each other, and may each independently be hydrogen; deuterium; a substituted or unsubstituted C6 to C10 aryl group; or a substituted or unsubstituted C2 to C10 heteroaryl group.

In other embodiment of the present invention, R51 to R55, R61 to R75, and Ra to Re may be the same as or different from each other, and may each independently be hydrogen; deuterium; a substituted or unsubstituted phenyl, naphthyl, anthracenyl or phenanthrenyl group.

In one embodiment of the present invention, q may be an integer from 0 to 3, and when q is 2 or more, each R73 may be the same as or different from each other, r may be an integer of 0 to 3, and when r is 2 or more, each R74 may be the same as or different from each other, s may be an integer from 0 to 2, and when s is 2, each R75 may be the same as or different from each other.

In one embodiment of the present invention, any one of R3, R4, R10, R11, and R13 in Formula 1 above may be represented by Structural Formula A or Structural Formula B above.

In other embodiment of the present invention, any one of R3, R4, and R13 in Formula 1 may be represented by Structural Formula A or Structural Formula B above.

In other embodiment of the present invention, any one of R10, R11, and R13 in Formula 1 may be represented by Structural Formula A or Structural Formula B above.

In other embodiment of the present invention, any one of R3 and R4 in Formula 1 may be represented by Structural Formula A or Structural Formula B above.

In other embodiment of the present invention, any one of R10 and R11 in Formula 1 may be represented by Structural Formula A or Structural Formula B above.

In other embodiment of the present invention, R13 in Formula 1 may be represented by Structural Formula A or Structural Formula B above.

In one embodiment of the present invention, Formula 1 above may be represented by any one of the compounds below:

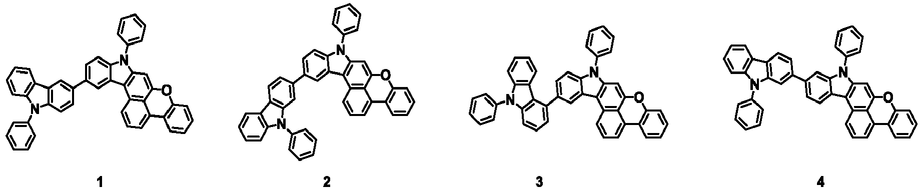

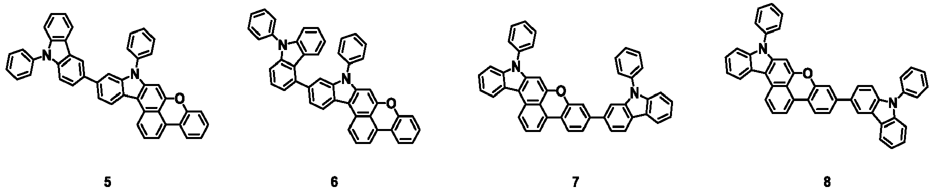

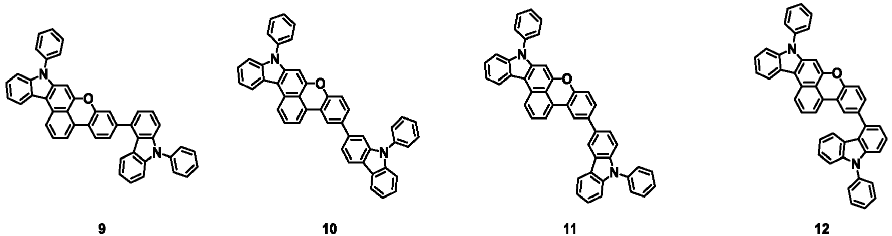

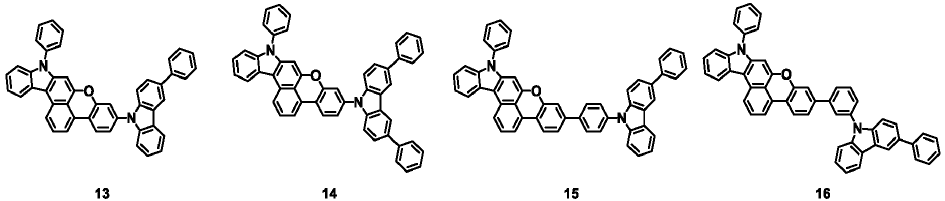

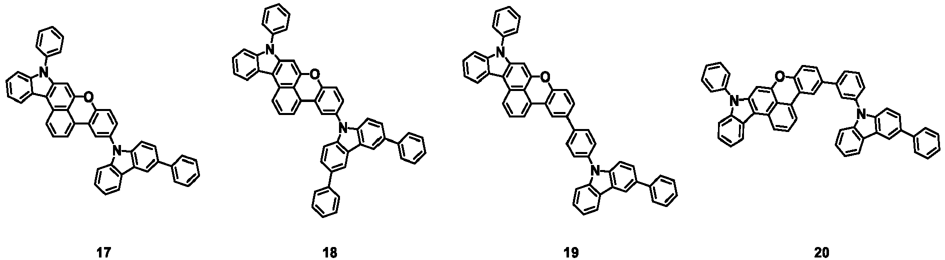

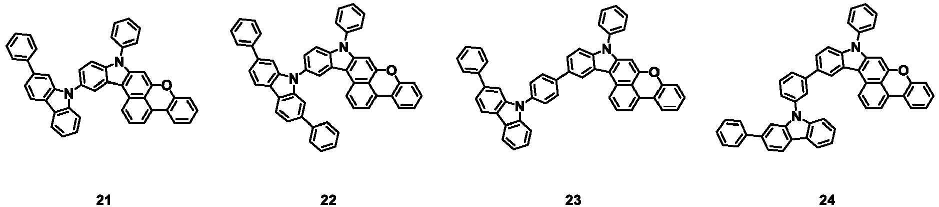

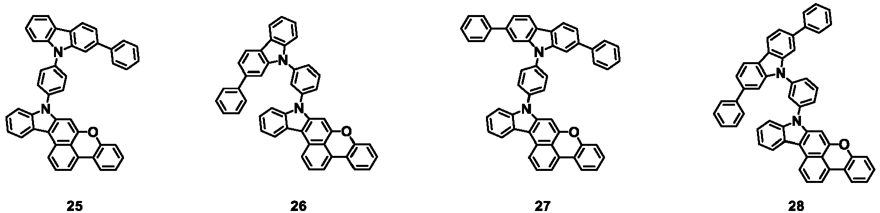

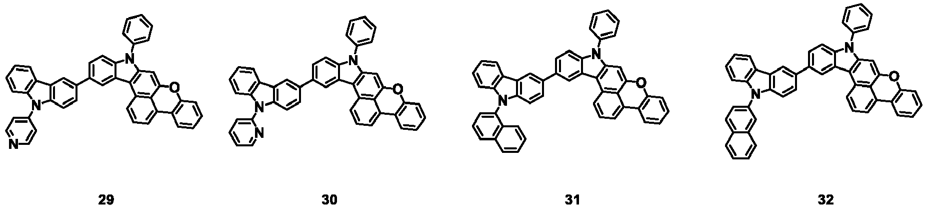

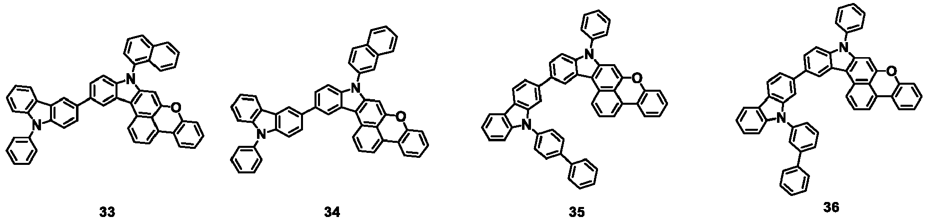

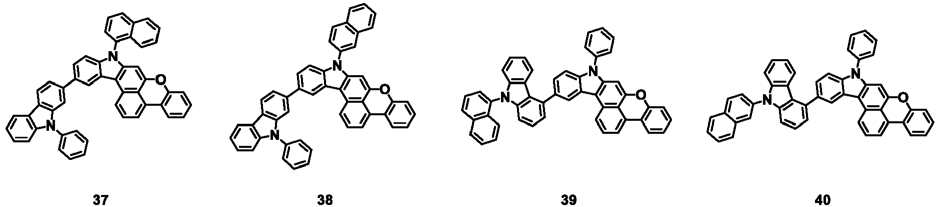

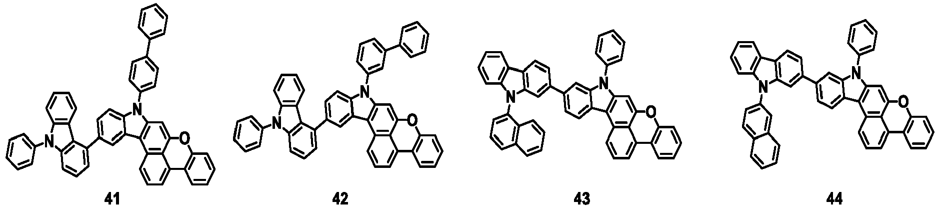

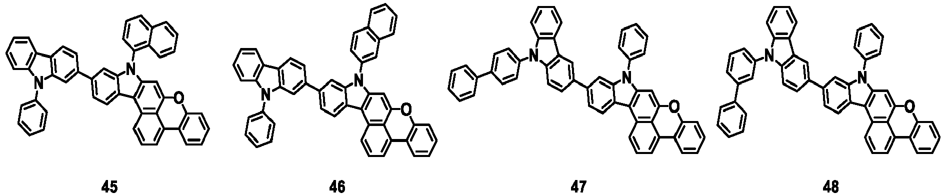

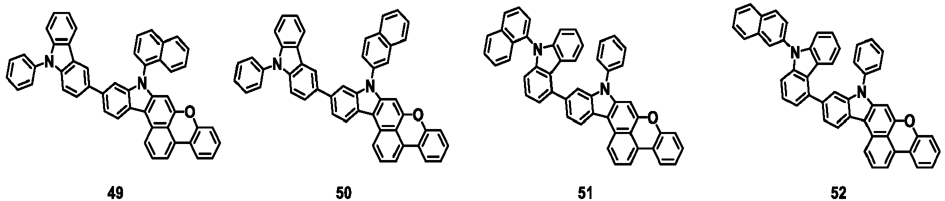

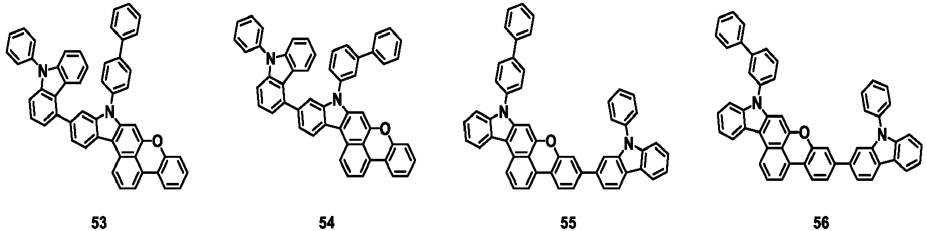

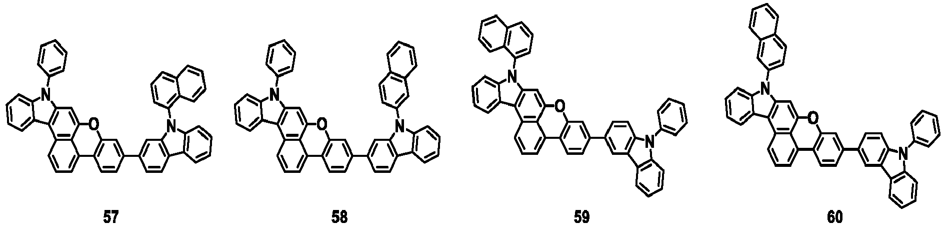

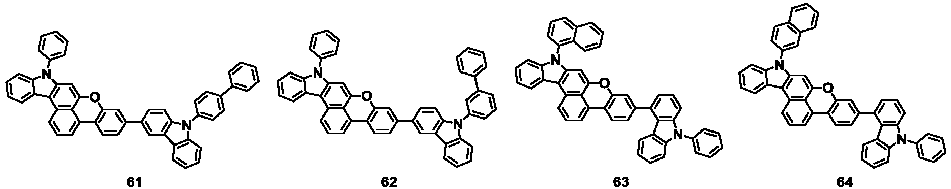

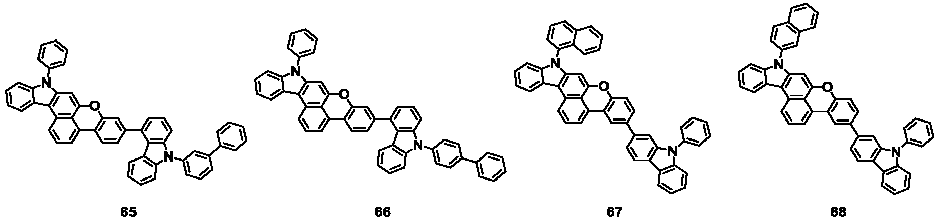

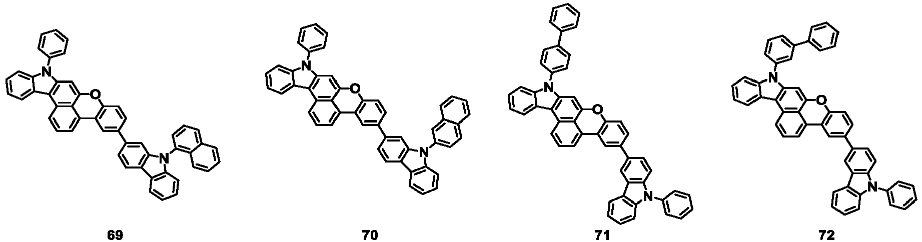

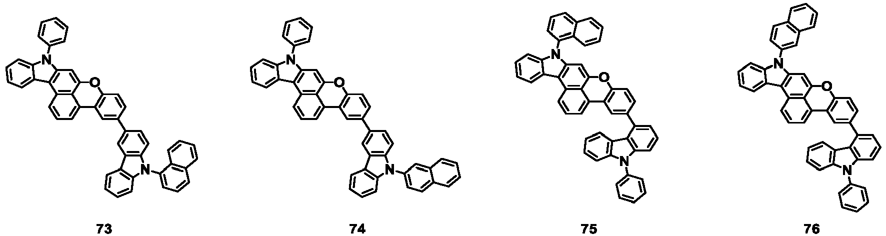

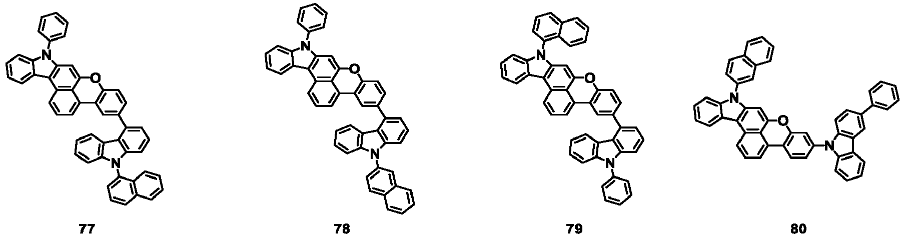

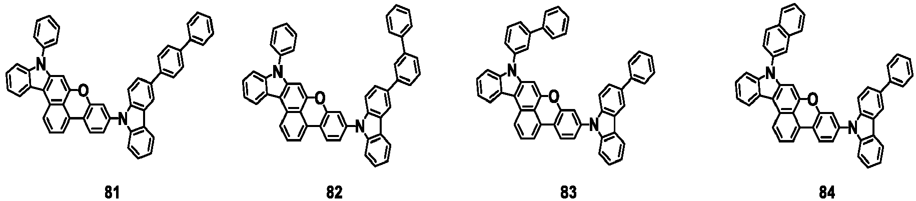

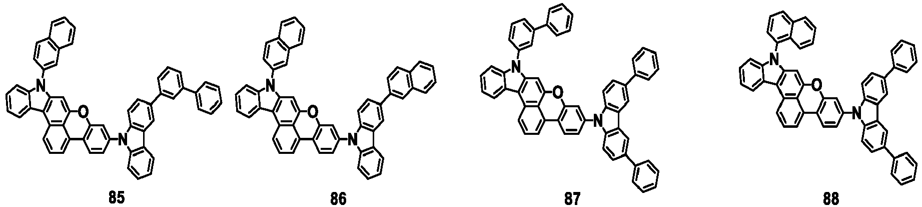

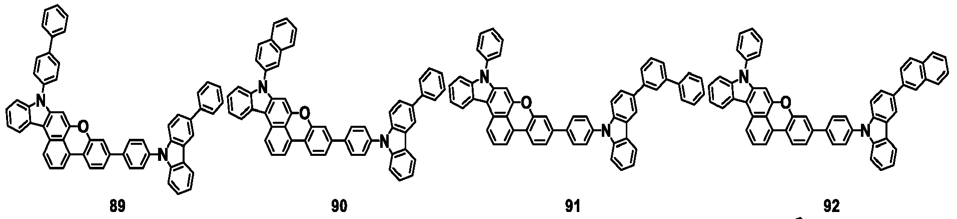

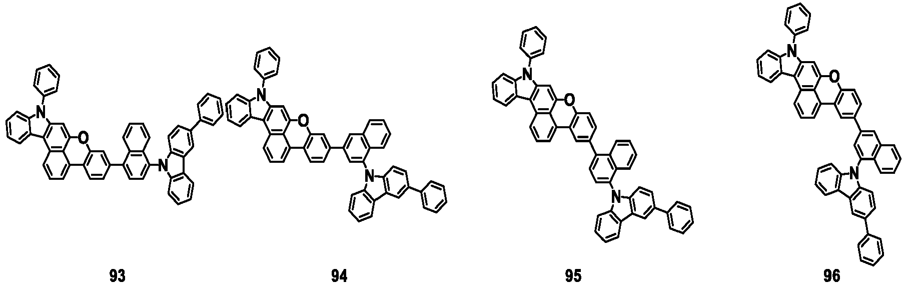

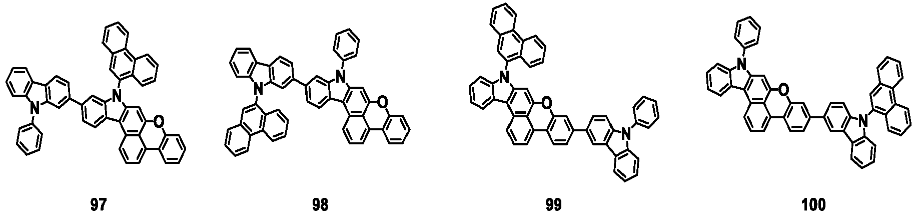

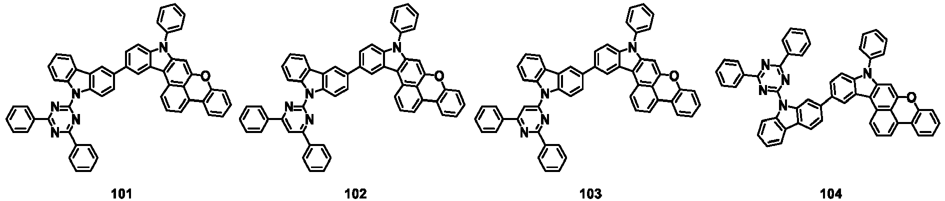

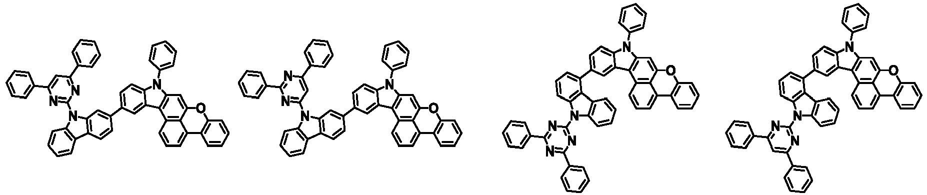

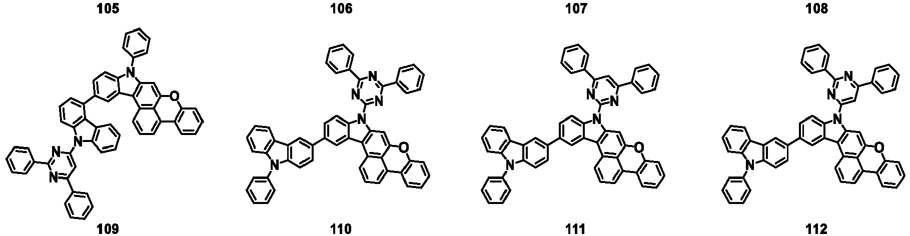

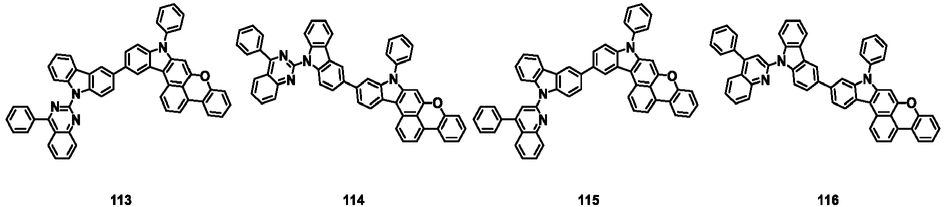

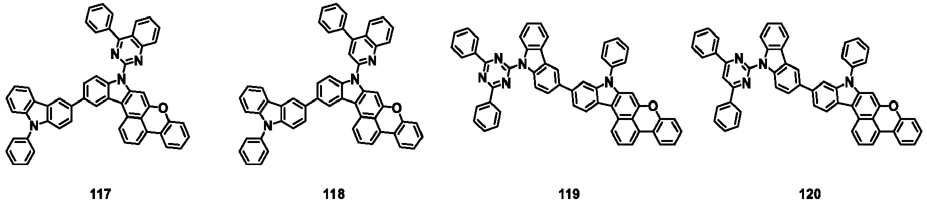

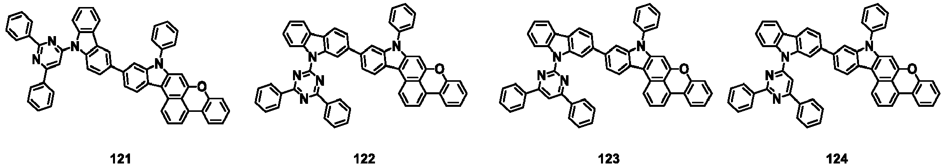

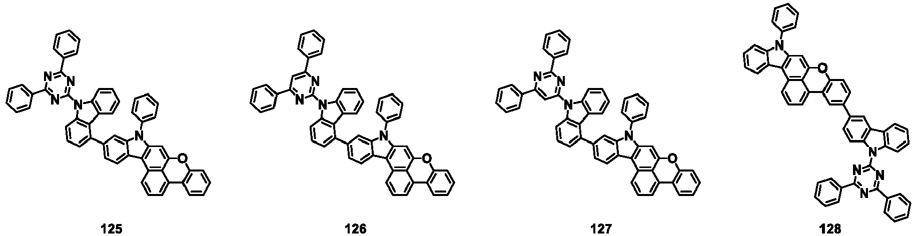

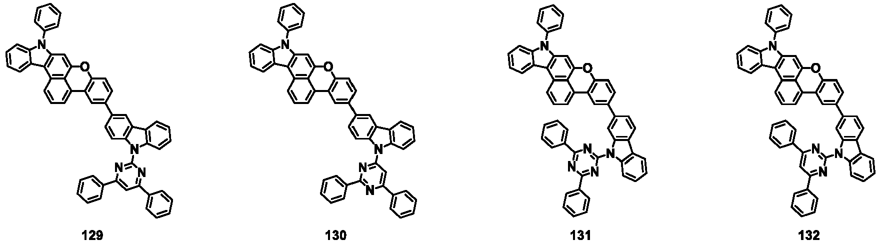

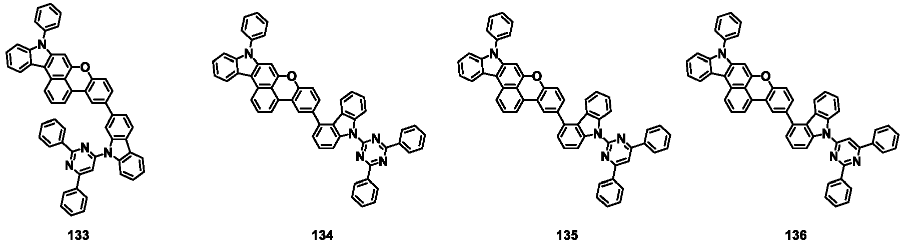

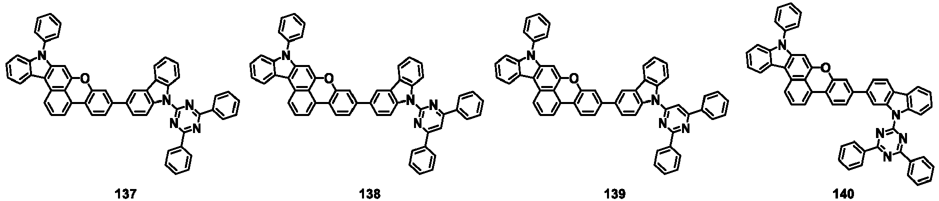

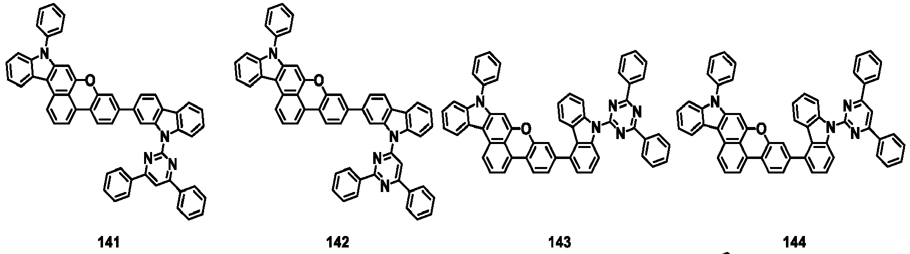

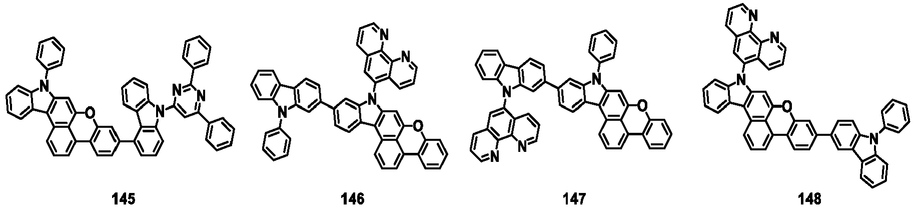

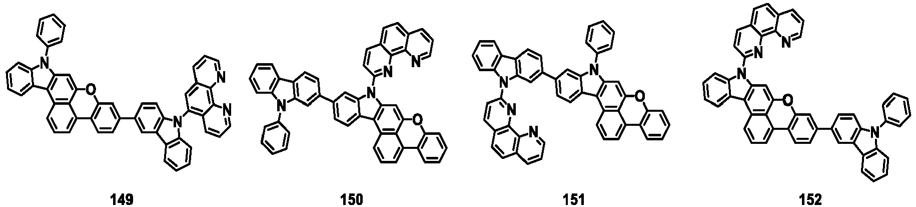

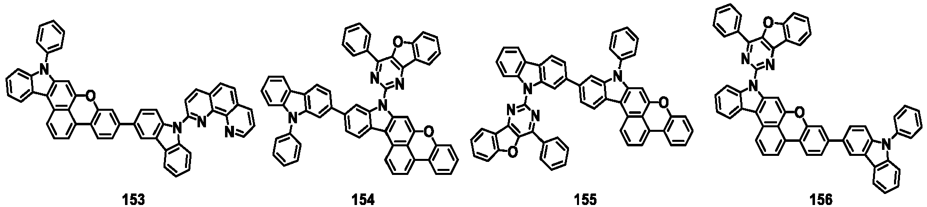

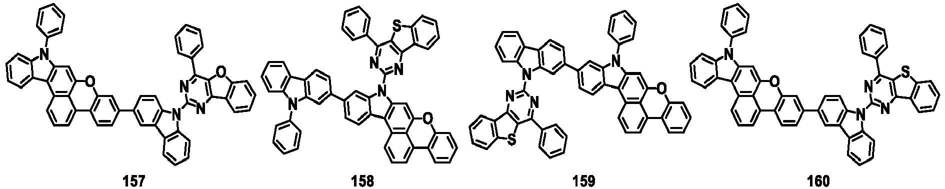

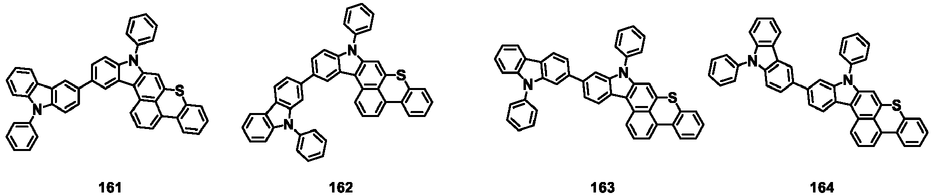

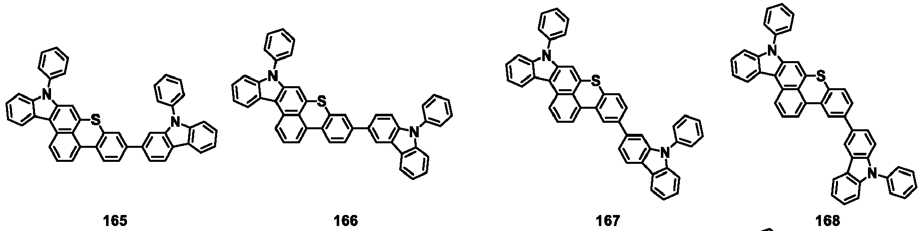

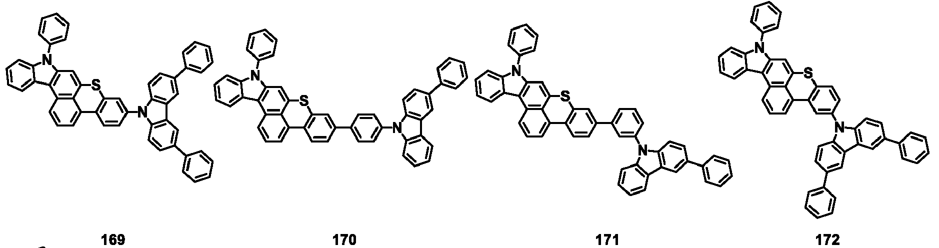

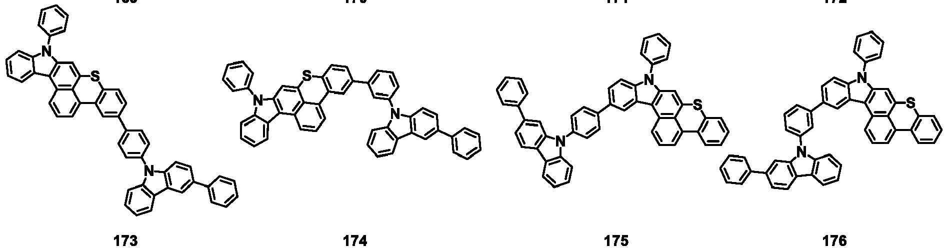

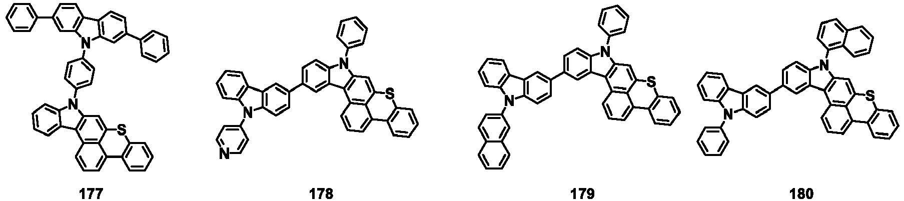

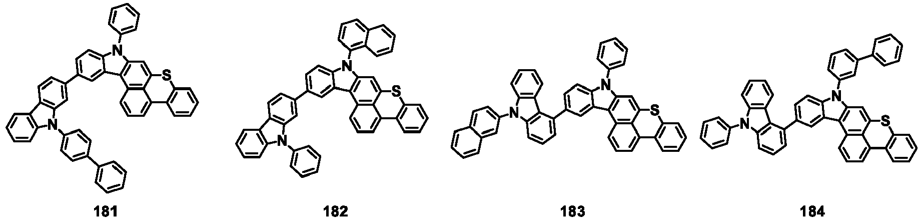

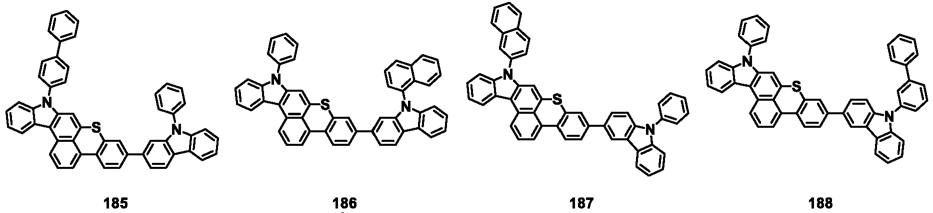

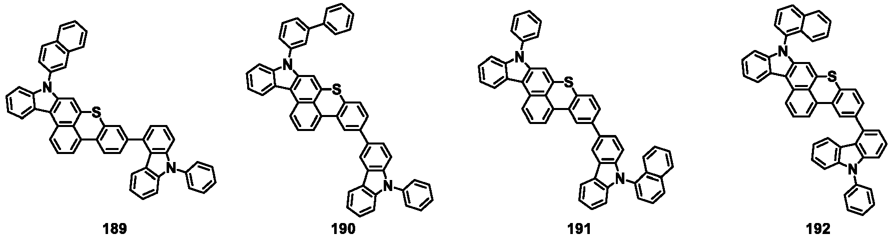

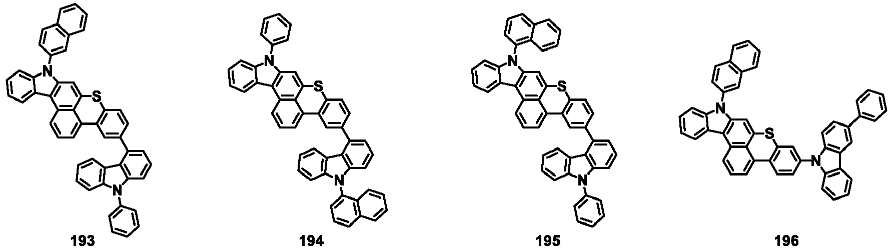

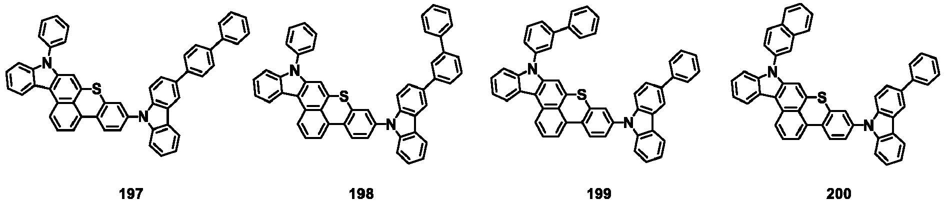

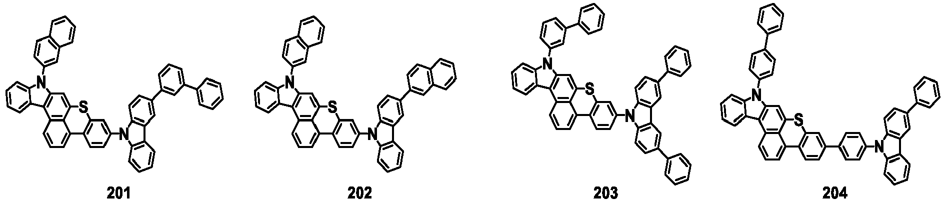

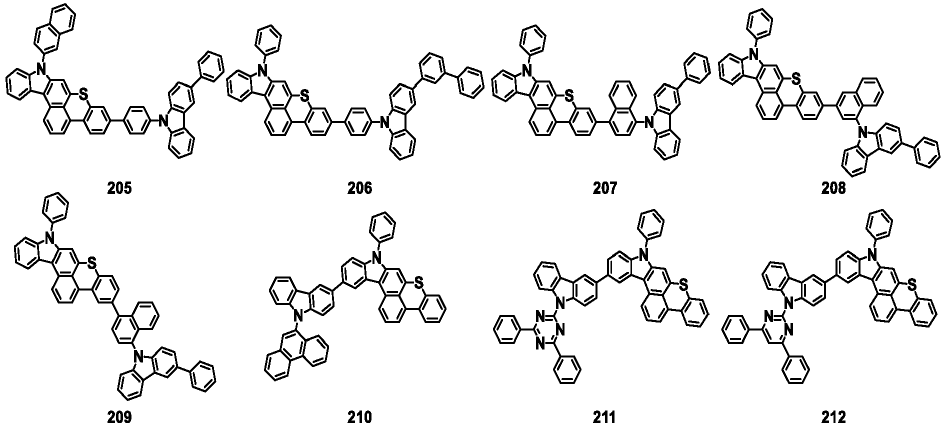

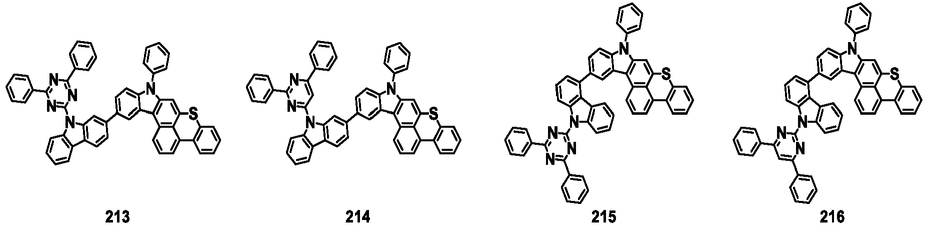

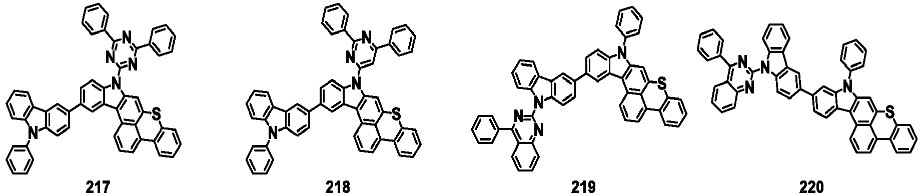

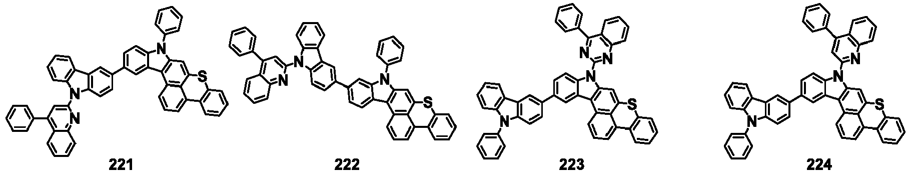

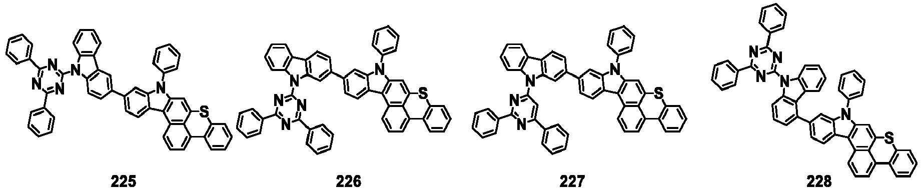

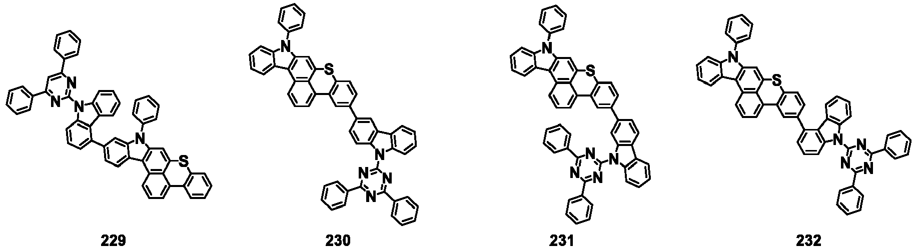

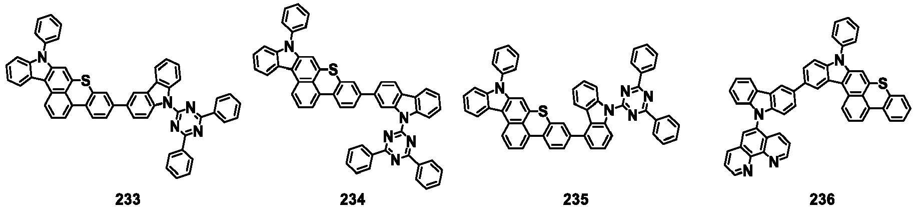

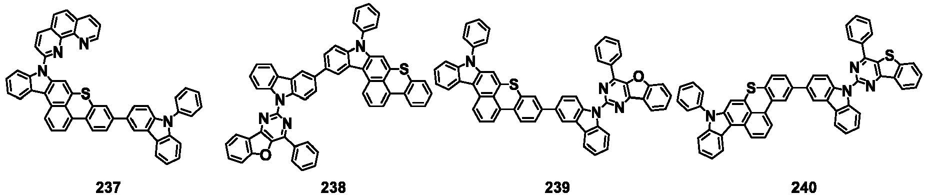

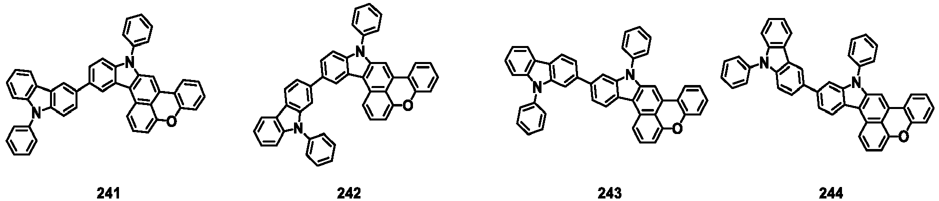

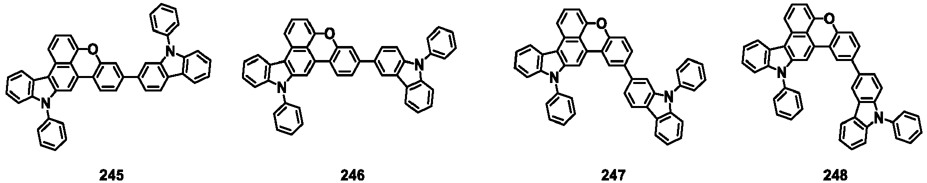

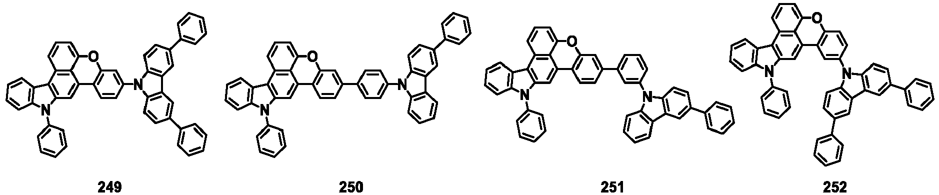

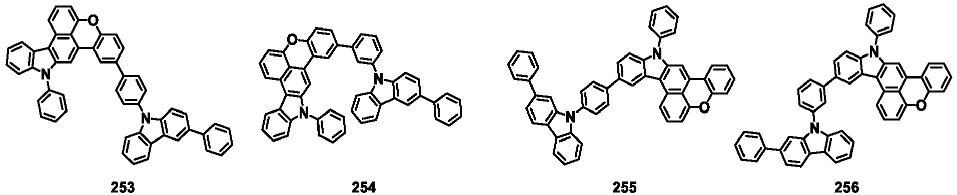

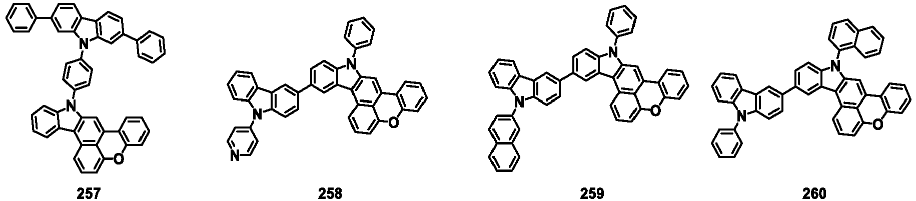

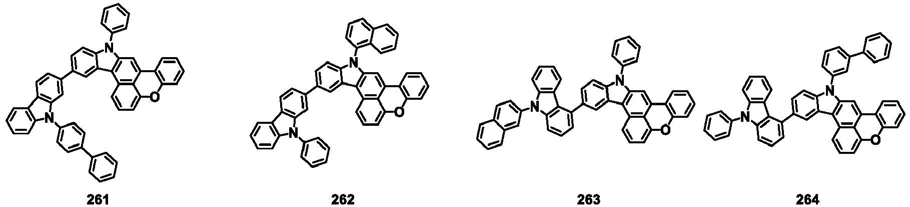

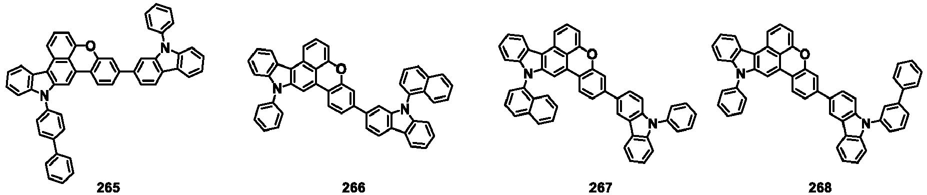

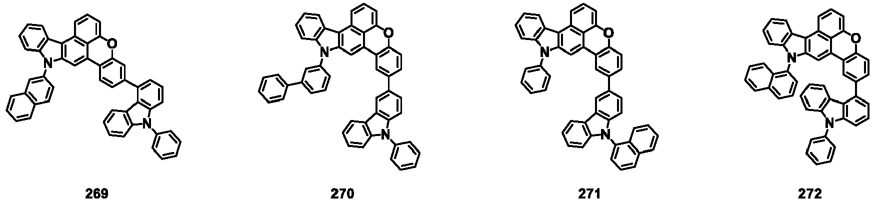

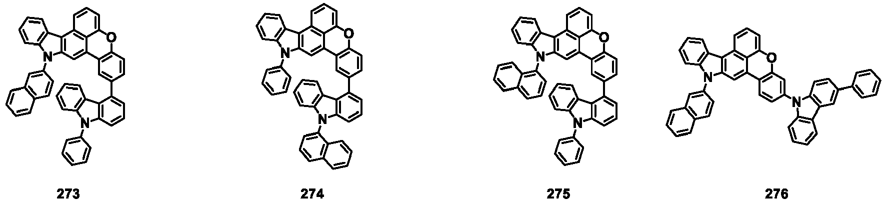

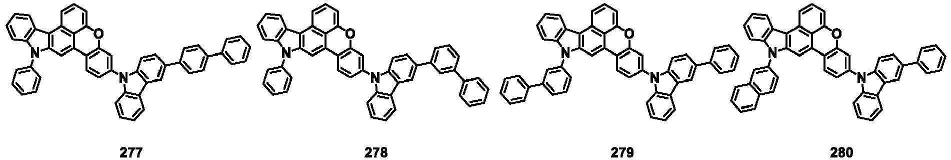

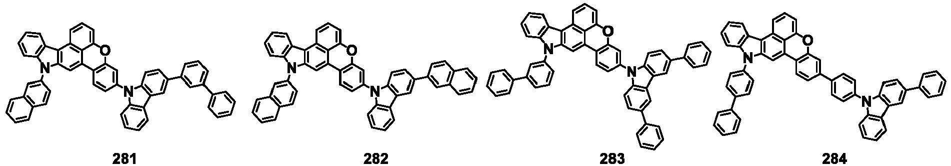

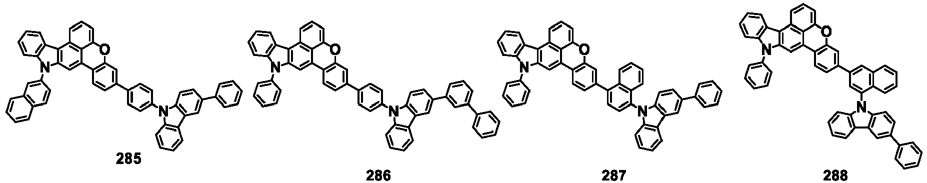

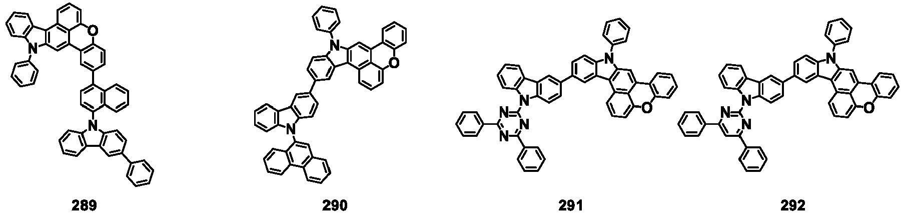

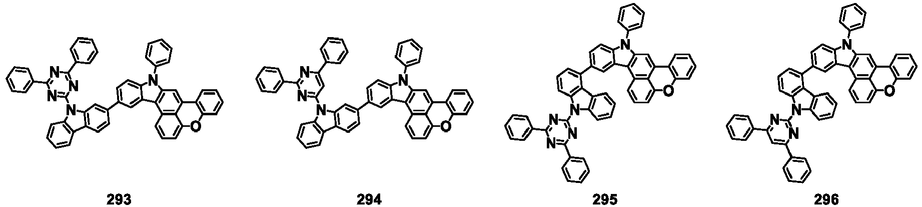

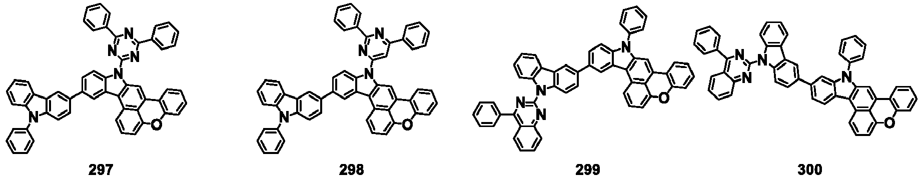

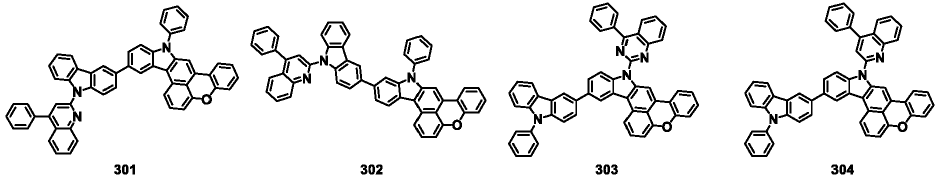

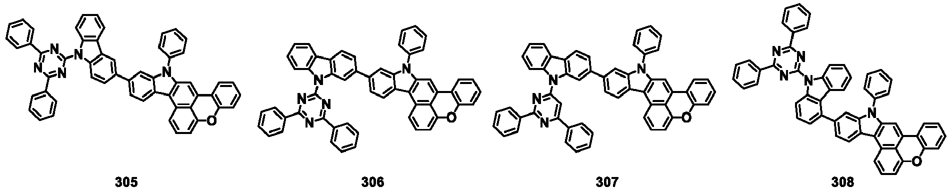

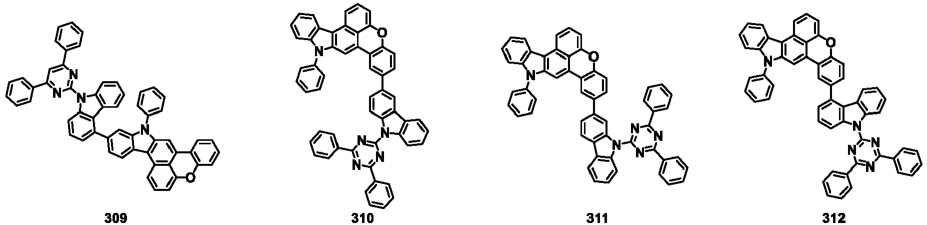

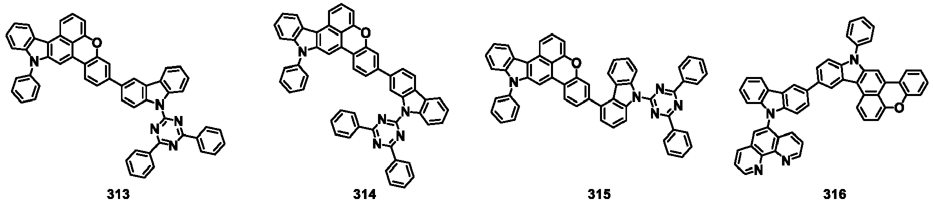

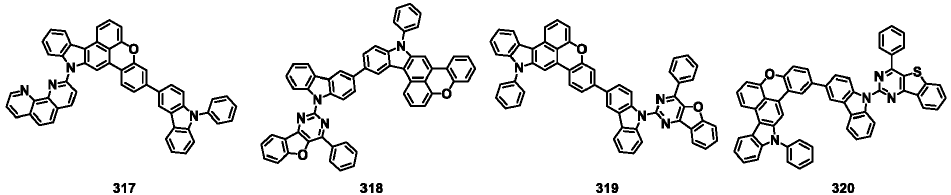

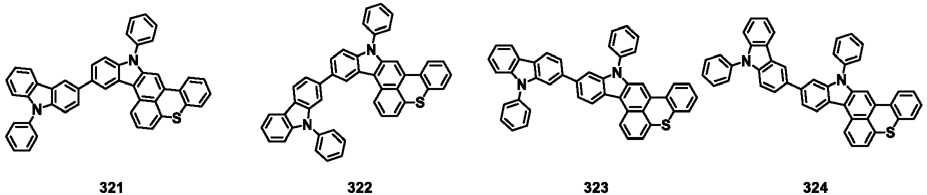

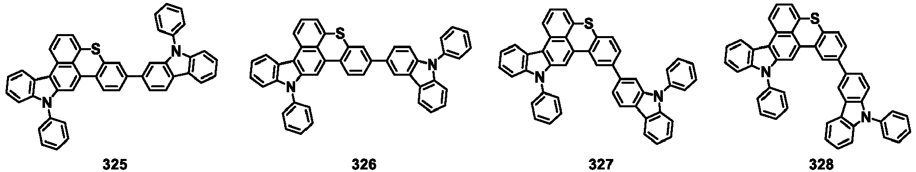

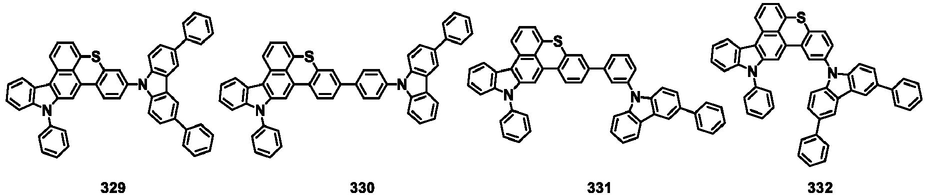

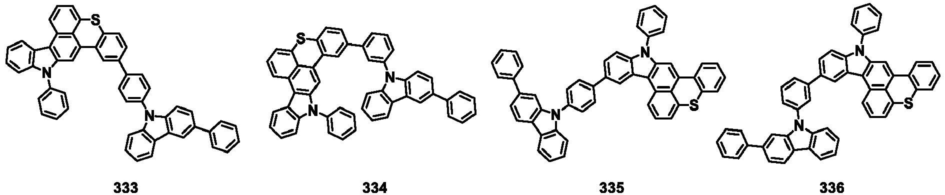

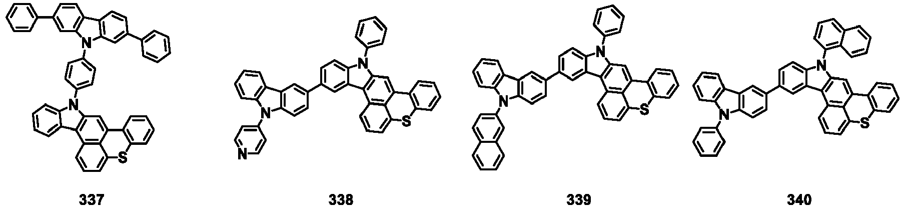

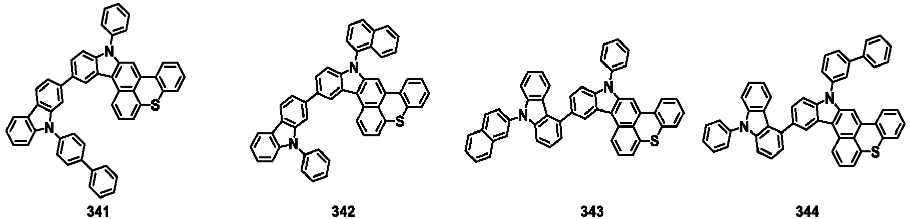

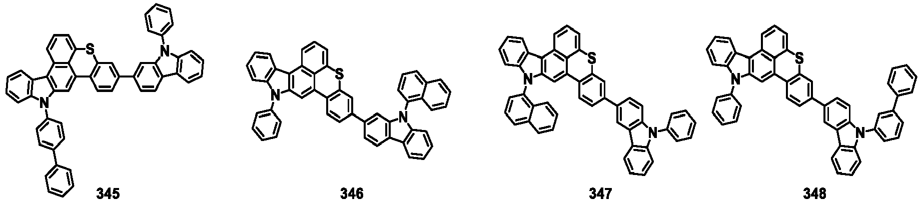

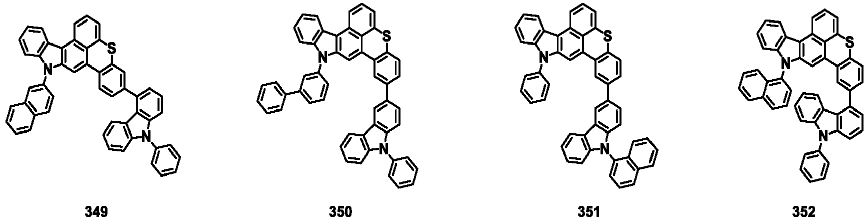

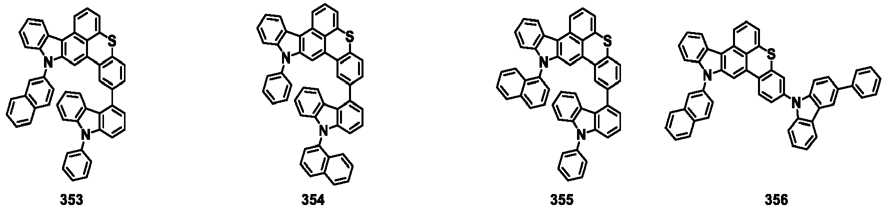

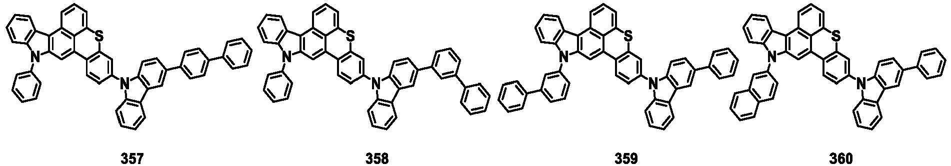

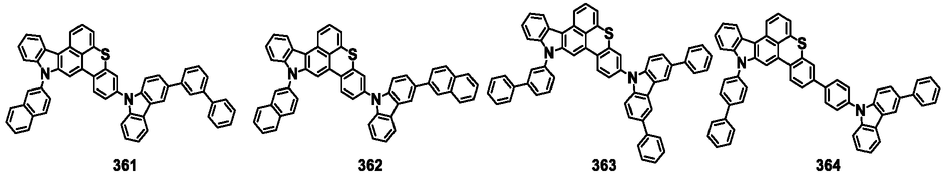

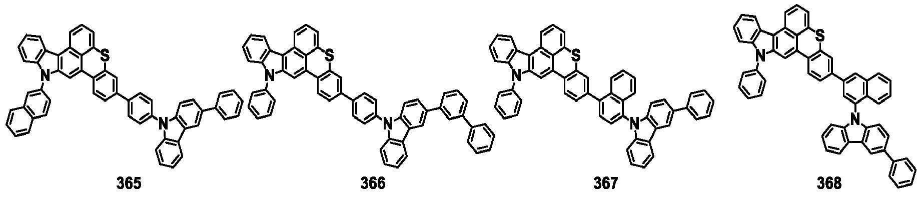

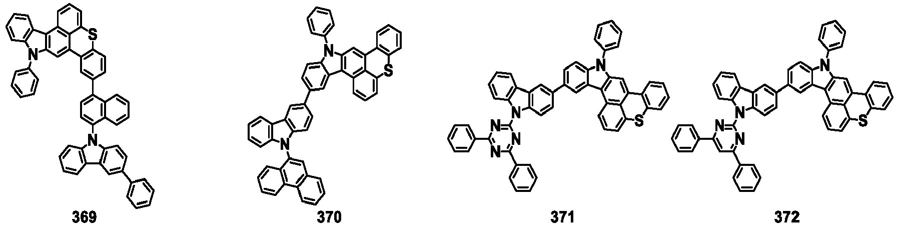

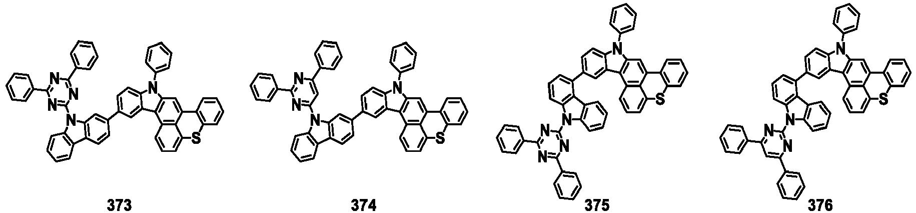

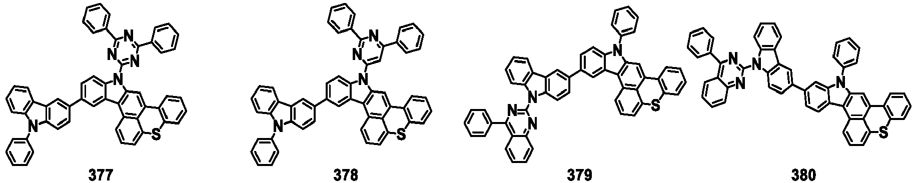

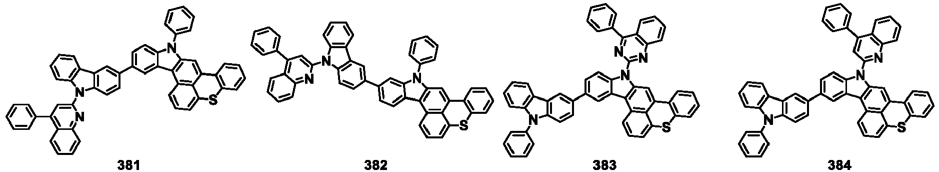

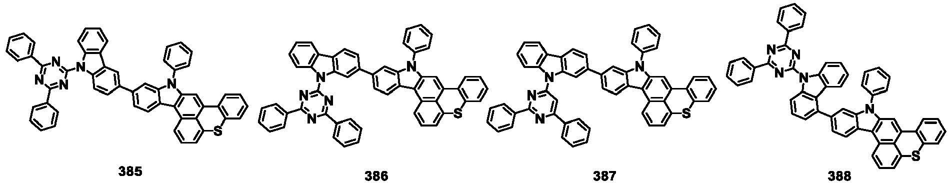

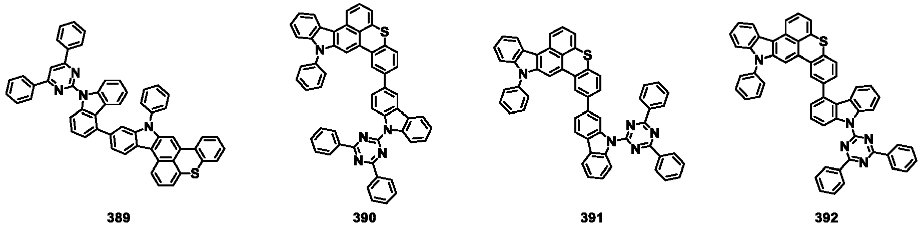

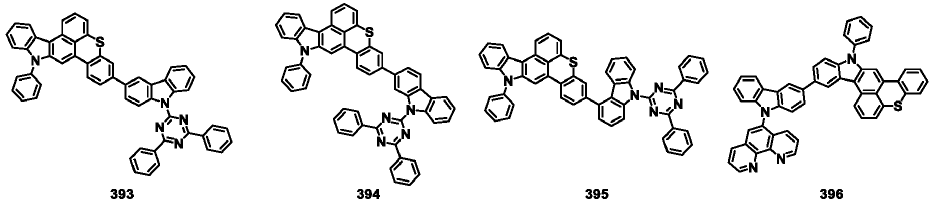

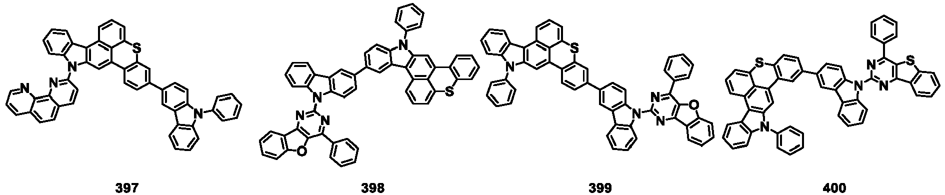

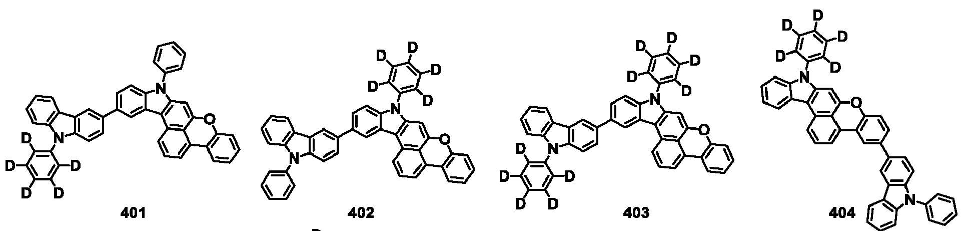

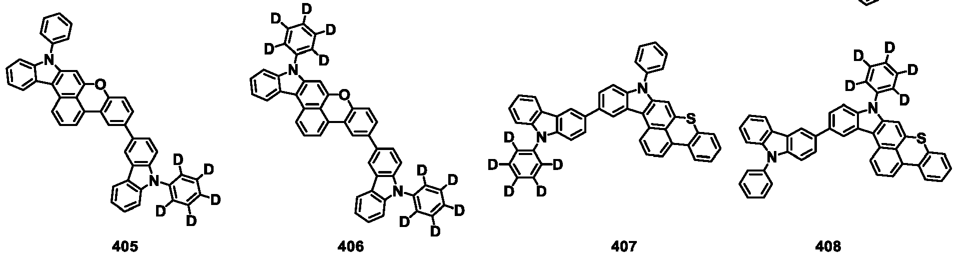

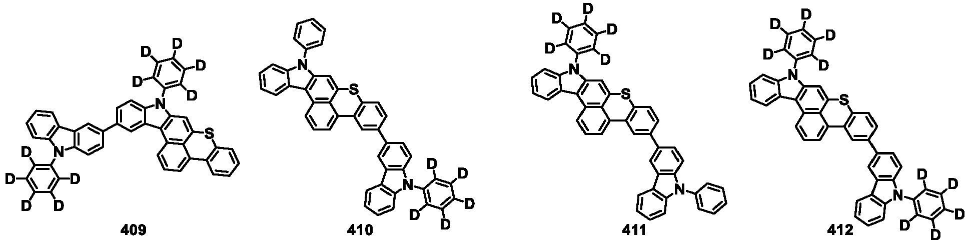

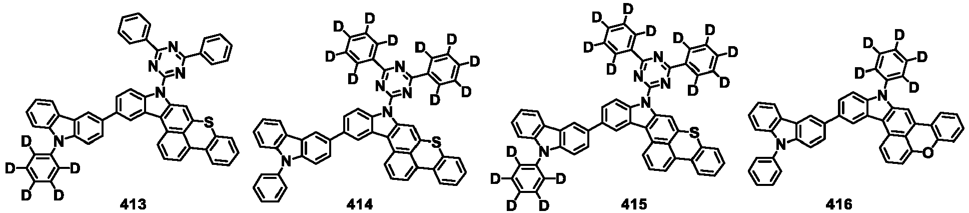

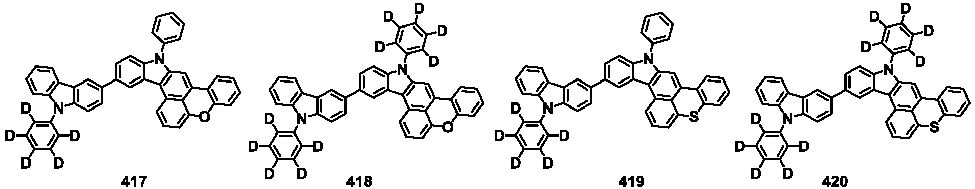

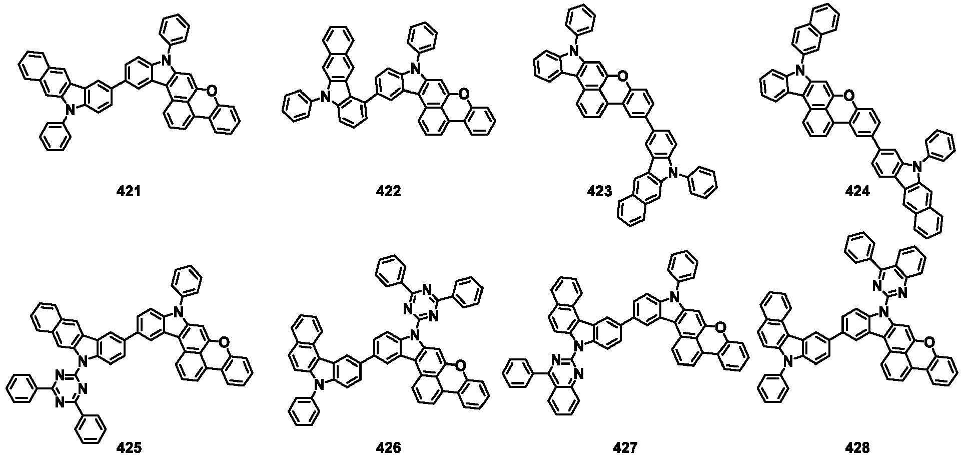

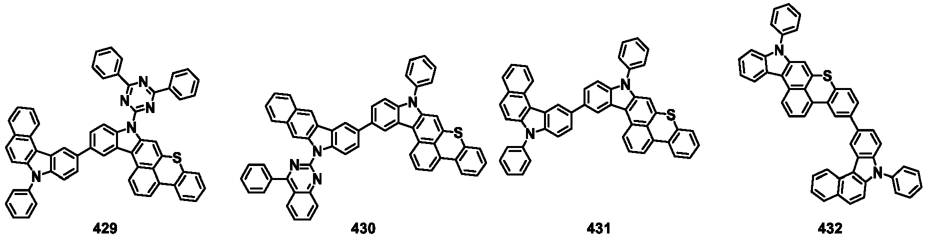

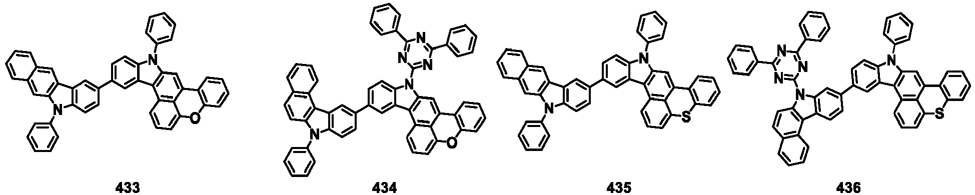

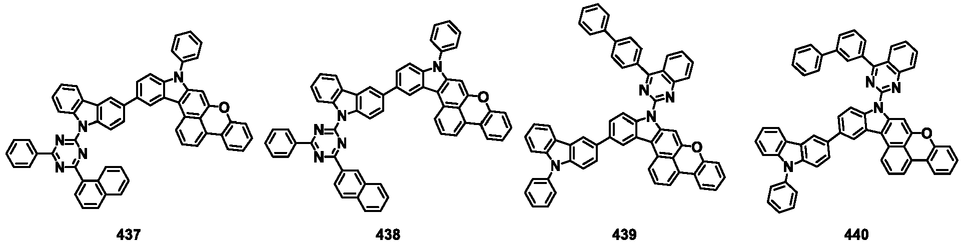

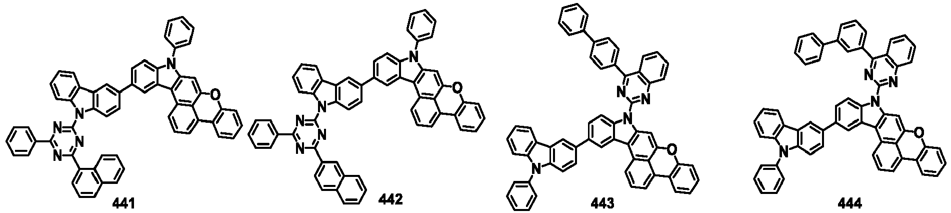

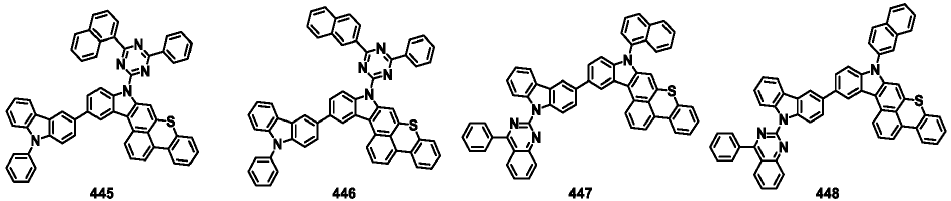

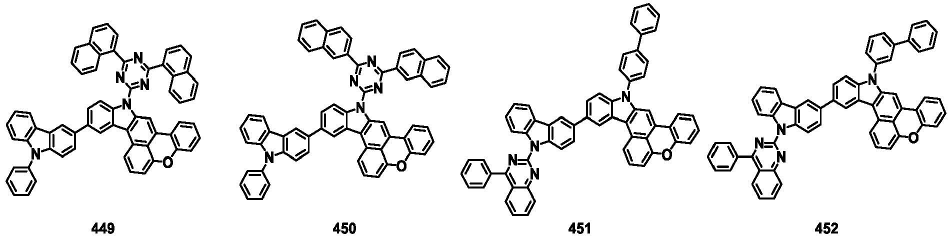

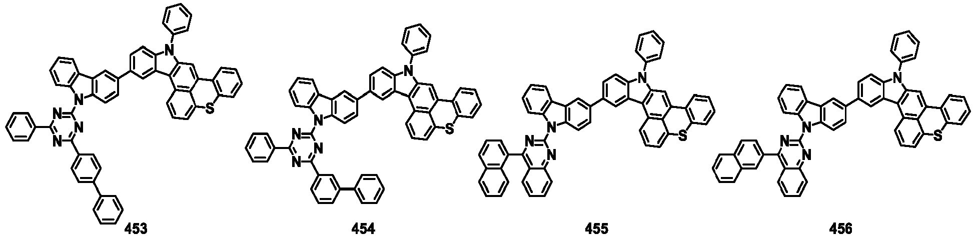

In addition, by introducing various substituents into the structure of Formula 1 above, compounds having intrinsic properties of the introduced substituents can be synthesized. For example, by introducing into the core structure a substituent mainly used in a material for a hole injection layer, a material for a hole transport layer, a material for a light emitting layer, a material for an electron transport layer, and a material for a charge generating layer used in manufacturing an organic light emitting device, substances that satisfy the requirements of each organic layer can be synthesized.

Also, by introducing various substituents into the structure of Formula 1, it is possible to finely control the energy bandgap, and on the other hand, to improve the properties at the interface between organic substances, and to diversify the use of the substances.

Also, in one embodiment of the present invention, the present invention provides an organic light emitting device comprising a first electrode; a second electrode provided to face the first electrode; and one or more organic layers provided between the first electrode and the second electrode, wherein at least one of the one or more organic layers comprises a heterocyclic compound represented by Formula 1 above.

In one embodiment of the present invention, the first electrode may be a positive electrode, and the second electrode may be a negative electrode.

In another embodiment, the first electrode may be a negative electrode, and the second electrode may be a positive electrode.

In one embodiment of the present invention, the organic light emitting device may be a blue organic light emitting device, and the heterocyclic compound represented by Formula 1 above may be used as a material of the blue organic light emitting device.

In one embodiment of the present invention, the organic light emitting device may be a green organic light emitting device, and the heterocyclic compound represented by Formula 1 may be used as a material of the green organic light emitting device.

In one embodiment of the present invention, the organic light emitting device may be a red organic light emitting device, and the heterocyclic compound represented by Formula 1 may be used as a material of the red organic light emitting device.

In one embodiment of the present invention, the organic light emitting device may be a blue organic light emitting device, and the heterocyclic compound represented by Formula 1 may be used as a material for a light emitting layer of the blue organic light emitting device.

In one embodiment of the present invention, the organic light emitting device may be a green organic light emitting device, and the heterocyclic compound represented by Formula 1 may be used as a material for a light emitting layer of the green organic light emitting device.

In one embodiment of the present invention, the organic light emitting device may be a red organic light emitting device, and the heterocyclic compound represented by Formula 1 may be used as a material for a light emitting layer of the red organic light emitting device.

Specific details of the heterocyclic compound represented by Formula 1 above are the same as described above.

The organic light emitting device of the present invention may be manufactured by a conventional method and material for manufacturing an organic light emitting device, except that one or more organic layers are formed using the aforementioned heterocyclic compound.

The heterocyclic compound may be formed as an organic layer by a solution coating method as well as a vacuum deposition method when manufacturing an organic light emitting device. In this case, the solution coating method means, but is not limited to, spin coating, dip coating, inkjet printing, screen printing, spraying, roll coating, and the like.

The organic layer of the organic light emitting device of the present invention may have a single-layer structure, and may also have a multi-layer structure formed by stacking two or more organic layers. For example, the organic light emitting device of the present invention may have a structure including a hole injection layer, a hole transport layer, a light emitting layer, an electron transport layer, an electron injection layer, etc. as an organic layer. However, the structure of the organic light emitting device is not limited thereto and may comprise a smaller number of organic layers.

In the organic light emitting device according to an embodiment of the present invention, an organic light emitting device is provided, wherein the organic layer including the heterocyclic compound represented by Formula 1 above further includes a heterocyclic compound represented by Formula 2 below: 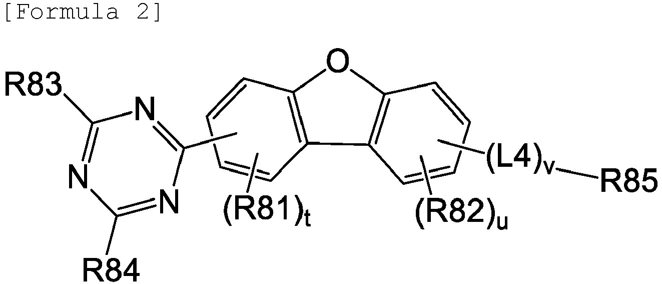 wherein,
L4 is a single bond; a substituted or unsubstituted C6 to C60 arylene group; or a substituted or unsubstituted C2 to C60 heteroarylene group,
R81 to R85 are the same as or different from each other, and are each independently selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; - P(=O)R101R102; -SiR101R102R103; and -NR101R102, or two or more groups adjacent to each other are combined with each other to form a substituted or unsubstituted C6 to C60 aromatic hydrocarbon ring or a substituted or unsubstituted C2 to C60 heterocycle, wherein R101, R102, and R103 are the same as or different from each other, and are each independently a substituted or unsubstituted C1 to C60 alkyl group; a substituted or unsubstituted C6 to C60 aryl group; or a substituted or unsubstituted C2 to C60 heteroaryl group,
t is an integer from 0 to 3, and when t is 2 or more, each R81 is the same as or different from each other, u is an integer from 0 to 3, and when u is 2 or more, each R82 is the same as or different from each other, v is an integer from 0 to 5, and when v is 2 or more, each L4 is the same as or different from each other.

When the compound represented by Formula 1 and the compound represented by Formula 2 are included at the same time, better efficiency and lifetime effect are exhibited. From this, it can be expected that the exciplex phenomenon occurs when both compounds are included at the same time.

The exciplex phenomenon is a phenomenon in which energy having the size of the HOMO energy level of the donor (p-host) and the LUMO energy level of the acceptor (n-host) is released through electron exchange between two molecules. If the exciplex phenomenon occurs between two molecules, Reverse Intersystem Crossing (RISC) occurs, and thus the internal quantum efficiency of fluorescence can be increased up to 100%. If a donor (p-host) with good hole transport ability and an acceptor (n-host) with good electron transport ability are used as hosts for the light emitting layer, since holes are injected into the p-host and electrons are injected into the n-host, the operating voltage can be lowered, thereby helping to improve lifetime. That is, if the compound represented by Formula 1 is used as the donor and the compound represented by Formula 2 is used as the acceptor, excellent device characteristics are exhibited.

In the organic light emitting device according to an embodiment of the present invention, L4 may be a single bond; a substituted or unsubstituted C6 to C60 arylene group; or a substituted or unsubstituted C2 to C60 heteroarylene group.

In other embodiment of the present invention, L4 may be a single bond, a substituted or unsubstituted C6 to C40 arylene group, or a substituted or unsubstituted C2 to C40 heteroarylene group.

In other embodiment of the present invention, L4 may be a single bond, a substituted or unsubstituted C6 to C20 arylene group, or a substituted or unsubstituted C2 to C20 heteroarylene group.

In other embodiment of the present invention, L4 may be a single bond.

In other embodiment of the present invention, L4 may be substituted or unsubstituted phenylene.

In the organic light emitting device according to an embodiment of the present invention, R81 to R85 may be the same as or different from each other, and may each independently be selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; -P(=O)R101R102; - SiR101R102R103; and -NR101R102, or two or more groups adjacent to each other may be combined with each other to form a substituted or unsubstituted C6 to C60 aromatic hydrocarbon ring or a substituted or unsubstituted C2 to C60 heterocycle, wherein R101, R102, and R103 may be the same as or different from each other, and may each independently be a substituted or unsubstituted C1 to C60 alkyl group; a substituted or unsubstituted C6 to C60 aryl group; or a substituted or unsubstituted C2 to C60 heteroaryl group.

In other embodiment of the present invention, R81 to R85 may be the same as or different from each other, and may each independently be selected from the group consisting of hydrogen; deuterium; halogen; a cyano group; a nitro group; a substituted or unsubstituted C1 to C40 alkyl group; a substituted or unsubstituted C2 to C40 alkenyl group; a substituted or unsubstituted C2 to C40 alkynyl group; a substituted or unsubstituted C3 to C40 cycloalkyl group; a substituted or unsubstituted C2 to C40 heterocycloalkyl group; a substituted or unsubstituted C6 to C40 aryl group; a substituted or unsubstituted C2 to C40 heteroaryl group; a substituted or unsubstituted C1 to C40 alkoxy group; a substituted or unsubstituted C6 to C40 aryloxy group; -P(=O)R101R102; - SiR101R102R103; and -NR101R102, or two or more groups adjacent to each other may be combined with each other to form a substituted or unsubstituted C6 to C40 aromatic hydrocarbon ring or a substituted or unsubstituted C2 to C40 heterocycle, wherein R101, R102, and R103 may be the same as or different from each other, and may each independently be a substituted or unsubstituted C1 to C40 alkyl group; a substituted or unsubstituted C6 to C40 aryl group; or a substituted or unsubstituted C2 to C40 heteroaryl group.

In other embodiment of the present invention, R81 to R85 may be the same as or different from each other, and may each independently be selected from the group consisting of hydrogen; deuterium; halogen; a cyano group; a nitro group; a substituted or unsubstituted C1 to C20 alkyl group; a substituted or unsubstituted C2 to C20 alkenyl group; a substituted or unsubstituted C2 to C20 alkynyl group; a substituted or unsubstituted C3 to C20 cycloalkyl group; a substituted or unsubstituted C2 to C20 heterocycloalkyl group; a substituted or unsubstituted C6 to C20 aryl group; a substituted or unsubstituted C2 to C20 heteroaryl group; a substituted or unsubstituted C1 to C20 alkoxy group; a substituted or unsubstituted C6 to C20 aryloxy group; -P(=O)R101R102; - SiR101R102R103; and -NR101R102, or two or more groups adjacent to each other may be combined with each other to form a substituted or unsubstituted C6 to C20 aromatic hydrocarbon ring or a substituted or unsubstituted C2 to C20 heterocycle, wherein R101, R102, and R103 may be the same as or different from each other, and may each independently be a substituted or unsubstituted C1 to C20 alkyl group; a substituted or unsubstituted C6 to C20 aryl group; or a substituted or unsubstituted C2 to C20 heteroaryl group.

In other embodiment of the present invention, R81 to R85 may be the same as or different from each other, and may each independently be hydrogen; deuterium; halogen; a cyano group; a nitro group; a substituted or unsubstituted C1 to C20 alkyl group; a substituted or unsubstituted C2 to C20 alkenyl group; a substituted or unsubstituted C2 to C20 alkynyl group; a substituted or unsubstituted C6 to C20 aryl group; or a substituted or unsubstituted C2 to C20 heteroaryl group.

In other embodiment of the present invention, R81 to R85 may be the same as or different from each other, and may each independently be hydrogen; deuterium; halogen; a cyano group; a nitro group; a substituted or unsubstituted C1 to C10 alkyl group; a substituted or unsubstituted C2 to C10 alkenyl group; a substituted or unsubstituted C2 to C10 alkynyl group; a substituted or unsubstituted C6 to C10 aryl group; or a substituted or unsubstituted C2 to C10 heteroaryl group.

In other embodiment of the present invention, R81 to R85 may be the same as or different from each other, and may each independently be hydrogen; deuterium; a substituted or unsubstituted C1 to C10 alkyl group; a substituted or unsubstituted C2 to C10 alkenyl group; a substituted or unsubstituted C2 to C10 alkynyl group; a substituted or unsubstituted C6 to C10 aryl group; or a substituted or unsubstituted C2 to C10 heteroaryl group.

In other embodiment of the present invention, R81 to R85 may be the same as or different from each other, and may each independently be hydrogen; deuterium; a substituted or unsubstituted C6 to C20 aryl group; or a substituted or unsubstituted C2 to C20 heteroaryl group.

In other embodiment of the present invention, R81 to R85 may be the same as or different from each other, and may each independently be hydrogen; deuterium; a substituted or unsubstituted C6 to C10 aryl group; or a substituted or unsubstituted C2 to C10 heteroaryl group.

In other embodiment of the present invention, R81 to R85 may be the same as or different from each other, and may each independently be hydrogen; deuterium; a substituted or unsubstituted phenyl, naphthyl, pyridinyl, anthracenyl, carbazolyl, dibenzothiophenyl, dibenzofuranyl, or phenanthrenyl group.

In other embodiment of the present invention, R81 to R85 may be the same as or different from each other, and may each independently be hydrogen; deuterium; a substituted or unsubstituted phenyl, carbazolyl, dibenzothiophenylor dibenzofuranyl group.

In other embodiment of the present invention, R81 to R85 may be the same as or different from each other, and may each independently be hydrogen; deuterium; a substituted or unsubstituted phenyl or carbazolyl group.

In the organic light emitting device according to another embodiment of the present invention, R81 to R85 may be the same as or different from each other, and may each independently be hydrogen; deuterium; a substituted or unsubstituted phenyl group; a biphenyl group; a carbazolyl group; or -NR101R102 group, wherein R101 and R102 may be the same as or different from each other, and may each independently be a substituted or unsubstituted C1 to C20 alkyl group; a substituted or unsubstituted C6 to C20 aryl group; or a substituted or unsubstituted C2 to C20 heteroaryl group.

In the organic light emitting device according to another embodiment of the present invention, L4 may be a single bond, R81 and R82 may be hydrogen, R83 and R84 may be a phenyl group or a biphenyl group, R85 may be a carbazolyl group or -NR101R102, wherein R101 and R102 may be the same as or different from each other, and may each independently be a substituted or unsubstituted C1 to C20 alkyl group; a substituted or unsubstituted C6 to C20 aryl group; or a substituted or unsubstituted C2 to C20 heteroaryl group.

In the organic light emitting device according to an embodiment of the present invention, t may be an integer from 0 to 3, and when t is 2 or more, each R81 may be the same as or different from each other, u may be an integer from 0 to 3, and when u is 2 or more, each R82 may be the same as or different from each other, v may be an integer from 0 to 5, and when v is 2 or more, each L4 may be the same as or different from each other.

In the organic light emitting device according to an embodiment of the present invention, Formula 2 above may be represented by Formula 2-1 or Formula 2-2 below: 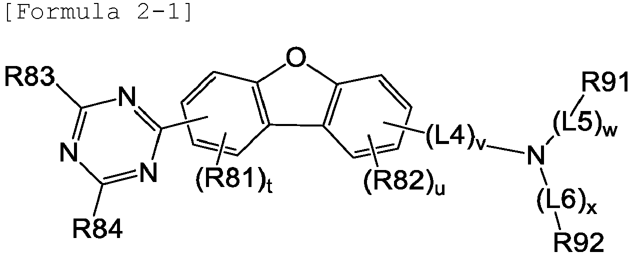 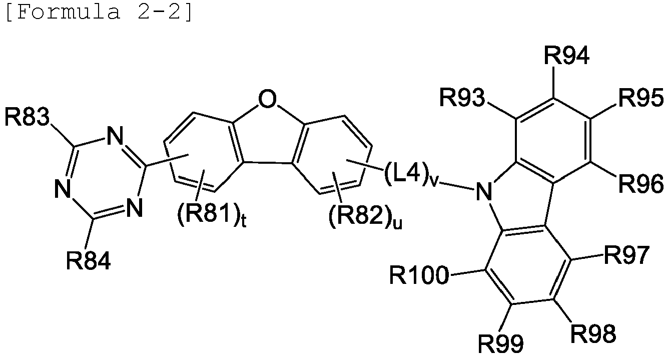 wherein,
L5 and L6 are the same as or different from each other, and are each independently a single bond; a substituted or unsubstituted C6 to C60 arylene group; or a substituted or unsubstituted C2 to C60 heteroarylene group,
w is an integer from 0 to 5, and when w is 2 or more, each L5 is the same as or different from each other, x is an integer from 0 to 5, and when x is 2 or more, each L6 is the same as or different from each other,
R91 to R100 are the same as or different from each other, and are each independently selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; - P(=O)R101R102; -SiR101R102R103; and -NR101R102, or two or more groups adjacent to each other are combined with each other to form a substituted or unsubstituted C6 to C60 aromatic hydrocarbon ring or a substituted or unsubstituted C2 to C60 heterocycle, wherein R101, R102, and R103 are the same as or different from each other, and are each independently a substituted or unsubstituted C1 to C60 alkyl group; a substituted or unsubstituted C6 to C60 aryl group; or a substituted or unsubstituted C2 to C60 heteroaryl group, and
R81 to R84, L4, t, u, and v are the same as defined in Formula 2 above.

In one embodiment of the present invention, L5 and L6 may be the same as or different from each other, and may each independently be a single bond; a substituted or unsubstituted C6 to C60 arylene group; or a substituted or unsubstituted C2 to C60 heteroarylene group.

In other embodiment of the present invention, L5 and L6 may be the same as or different from each other, and may each independently be a single bond, a substituted or unsubstituted C6 to C40 arylene group, or a substituted or unsubstituted C2 to C40 heteroarylene group.

In other embodiment of the present invention, L5 and L6 may be the same as or different from each other, and may each independently be a single bond, a substituted or unsubstituted C6 to C20 arylene group, or a substituted or unsubstituted C2 to C20 heteroarylene group.

In other embodiment of the present invention, L5 and L6 may be the same as or different from each other, and may each independently be selected from the group consisting of a single bond, substituted or unsubstituted phenylene, biphenylene, naphthalene, anthracenylene, pyrene, phenanthrene, pyridine, pyrimidine, triazine, quinoline, and quinazoline.

In other embodiment of the present invention, L5 and L6 may be the same as or different from each other, and may each independently be selected from the group consisting of a single bond, substituted or unsubstituted phenylene, pyrimidine, triazine, quinoline, and quinazoline.

In other embodiment of the present invention, L5 and L6 may be the same as or different from each other, and may each independently be a single bond.

In other embodiment of the present invention, L5 and L6 may be the same as or different from each other, and may each independently be substituted or unsubstituted phenylene.

In one embodiment of the present invention, w may be an integer from 0 to 5, and when w is 2 or more, each L5 may be the same as or different from each other, x may be an integer from 0 to 5, and when x is 2 or more, each L6 may be the same as or different from each other.

In one embodiment of the present invention, R91 to R100 may be the same as or different from each other, and may each independently be selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; -P(=O)R101R102; - S1R101R102R103; and -NR101R102, or two or more groups adjacent to each other may be combined with each other to form a substituted or unsubstituted C6 to C60 aromatic hydrocarbon ring or a substituted or unsubstituted C2 to C60 heterocycle, wherein R101, R102, and R103 may be the same as or different from each other, and may each independently be a substituted or unsubstituted C1 to C60 alkyl group; a substituted or unsubstituted C6 to C60 aryl group; or a substituted or unsubstituted C2 to C60 heteroaryl group.

In other embodiment of the present invention, R91 to R100 may be the same as or different from each other, and may each independently be selected from the group consisting of hydrogen; deuterium; halogen; a cyano group; a nitro group; a substituted or unsubstituted C1 to C40 alkyl group; a substituted or unsubstituted C2 to C40 alkenyl group; a substituted or unsubstituted C2 to C40 alkynyl group; a substituted or unsubstituted C3 to C40 cycloalkyl group; a substituted or unsubstituted C2 to C40 heterocycloalkyl group; a substituted or unsubstituted C6 to C40 aryl group; a substituted or unsubstituted C2 to C40 heteroaryl group; a substituted or unsubstituted C1 to C40 alkoxy group; a substituted or unsubstituted C6 to C40 aryloxy group; -P(=O)R101R102; - SiR101R102R103; and -NR101R102, or two or more groups adjacent to each other may be combined with each other to form a substituted or unsubstituted C6 to C40 aromatic hydrocarbon ring or a substituted or unsubstituted C2 to C40 heterocycle, wherein R101, R102, and R103 may be the same as or different from each other, and may each independently be a substituted or unsubstituted C1 to C40 alkyl group; a substituted or unsubstituted C6 to C40 aryl group; or a substituted or unsubstituted C2 to C40 heteroaryl group.

In other embodiment of the present invention, R91 to R100 may be the same as or different from each other, and may each independently be selected from the group consisting of hydrogen; deuterium; halogen; a cyano group; a nitro group; a substituted or unsubstituted C1 to C20 alkyl group; a substituted or unsubstituted C2 to C20 alkenyl group; a substituted or unsubstituted C2 to C20 alkynyl group; a substituted or unsubstituted C3 to C20 cycloalkyl group; a substituted or unsubstituted C2 to C20 heterocycloalkyl group; a substituted or unsubstituted C6 to C20 aryl group; a substituted or unsubstituted C2 to C20 heteroaryl group; a substituted or unsubstituted C1 to C20 alkoxy group; a substituted or unsubstituted C6 to C20 aryloxy group; -P(=O)R101R102; - S1R101R102R103; and -NR101R102, or two or more groups adjacent to each other may be combined with each other to form a substituted or unsubstituted C6 to C20 aromatic hydrocarbon ring or a substituted or unsubstituted C2 to C20 heterocycle, wherein R101, R102, and R103 may be the same as or different from each other, and may each independently be a substituted or unsubstituted C1 to C20 alkyl group; a substituted or unsubstituted C6 to C20 aryl group; or a substituted or unsubstituted C2 to C20 heteroaryl group.

In other embodiment of the present invention, R91 to R100 may be the same as or different from each other, and may each independently be hydrogen; deuterium; halogen; a cyano group; a nitro group; a substituted or unsubstituted C1 to C20 alkyl group; a substituted or unsubstituted C2 to C20 alkenyl group; a substituted or unsubstituted C2 to C20 alkynyl group; a substituted or unsubstituted C6 to C20 aryl group; or a substituted or unsubstituted C2 to C20 heteroaryl group, or two or more groups adjacent to each other may be combined with each other to form a substituted or unsubstituted C6 to C20 aromatic hydrocarbon ring or a substituted or unsubstituted C2 to C20 heterocycle.

In other embodiment of the present invention, R91 to R100 may be the same as or different from each other, and may each independently be selected from the group consisting of hydrogen; deuterium; halogen; a cyano group; a nitro group; a substituted or unsubstituted C1 to C10 alkyl group; a substituted or unsubstituted C2 to C10 alkenyl group; a substituted or unsubstituted C2 to C10 alkynyl group; a substituted or unsubstituted C6 to C10 aryl group; or a substituted or unsubstituted C2 to C10 heteroaryl group, or two or more groups adjacent to each other may be combined with each other to form a substituted or unsubstituted C6 to C10 aromatic hydrocarbon ring or a substituted or unsubstituted C2 to C10 heterocycle.

In other embodiment of the present invention, R91 to R100 may be the same as or different from each other, and may each independently be hydrogen; deuterium; a substituted or unsubstituted C1 to C10 alkyl group; a substituted or unsubstituted C2 to C10 alkenyl group; a substituted or unsubstituted C2 to C10 alkynyl group; a substituted or unsubstituted C6 to C10 aryl group; or a substituted or unsubstituted C2 to C10 heteroaryl group.

In other embodiment of the present invention, R91 to R100 may be the same as or different from each other, and may each independently be hydrogen; deuterium; a substituted or unsubstituted C6 to C20 aryl group; or a substituted or unsubstituted C2 to C20 heteroaryl group.

In other embodiment of the present invention, R91 to R100 may be the same as or different from each other, and may each independently be hydrogen; deuterium; a substituted or unsubstituted C6 to C10 aryl group; or a substituted or unsubstituted C2 to C10 heteroaryl group.

In other embodiment of the present invention, R91 to R100 may be the same as or different from each other, and may each independently be hydrogen; deuterium; substituted or unsubstituted phenyl, naphthyl, anthracenyl, or phenanthrenyl group.

In other embodiment of the present invention, the 'substitutions' of L1 to L6, R1 to R23, R31 to R46, R51 to R55, R61 to R75, R81 to R85, R91 to R100 and Ra to Re may each independently made at least one substituent selected from the group consisting of by deuterium; a C1 to C10 alkyl group; a C2 to C10 alkenyl group; a C2 to C10 alkynyl group; a C3 to C15 cycloalkyl group; a C2 to C20 heterocycloalkyl group; a C6 to C30 aryl group; a C2 to C30 heteroaryl group; a C1 to C10 alkylamine group; a C6 to C30 arylamine group; and a C2 to C30 heteroarylamine group.

In other embodiment of the present invention, the 'substitutions' of L1 to L6, R1 to R23, R31 to R46, R51 to R55, R61 to R75, R81 to R85, R91 to R100, and Ra to Re may each be independently made by at least one substituent selected from the group consisting of deuterium; a C1 to C10 alkyl group; a C6 to C30 aryl group; a C2 to C30 heteroaryl group.

In other embodiment of the present invention, the 'substitutions' of L1 to L6, R1 to R23, R31 to R46, R51 to R55, R61 to R75, R81 to R85, R91 to R100, and Ra to Re may each be independently made by at least one substituent selected from the group consisting of deuterium; a C1 to C5 alkyl group; a C6 to C20 aryl group; and a C2 to C20 heteroaryl group.

In other embodiment of the present invention, the 'substitutions' of L1 to L6, R1 to R23, R31 to R46, R51 to R55, R61 to R75, R81 to R85, R91 to R100, and Ra to Re may each be independently made by at least one substituent selected from the group consisting of deuterium, a methyl group, an ethyl group, a straight or branched propyl group, a straight or branched butyl group, a straight or branched pentyl group, a phenyl group, a naphthyl group, a pyridinyl group, an anthracenyl group, a carbazolyl group, a dibenzothiophenyl group, a dibenzofuranyl group, and a phenanthrenyl group.

In other embodiment of the present invention, the 'substitutions' of L1 to L6, R1 to R23, R31 to R46, R51 to R55, R61 to R75, R81 to R85, R91 to R100, and Ra to Re may each be independently made by at least one substituent selected from the group consisting of deuterium, a methyl group, an ethyl group, a straight or branched propyl group, a straight or branched butyl group, and a straight or branched pentyl group.

In one embodiment of the present invention, R81 to R84, L4, t, u, and v may be the same as defined in Formula 2 above.

In one embodiment of the present invention, the heterocyclic compound represented by Formula 2 may be at least one selected from compounds below:

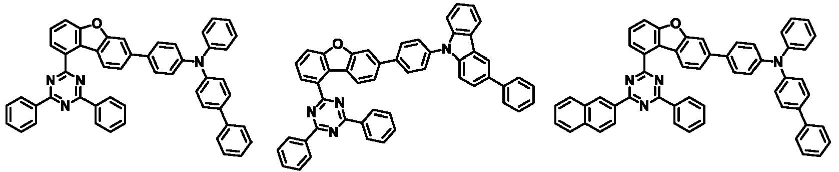

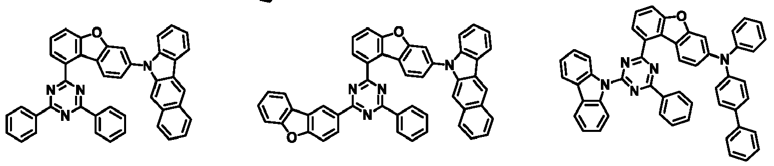

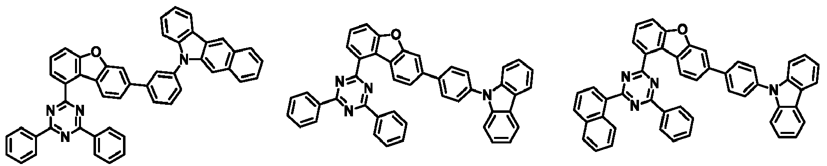

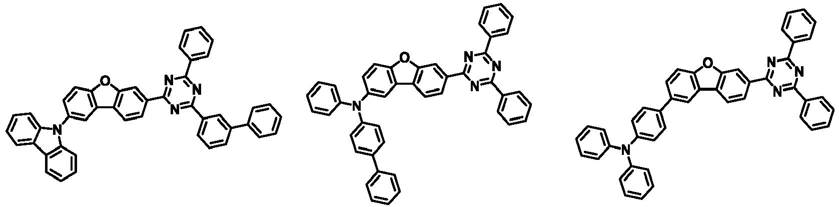

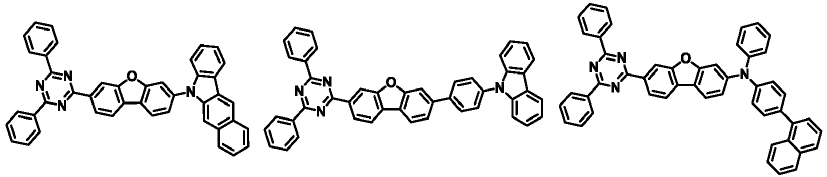

In addition, in the organic light emitting device according to an embodiment of the present invention, the organic layer may comprise a light emitting layer, the light emitting layer may comprise a host material, and the host material may comprise a heterocyclic compound represented by Formula 1 and a heterocyclic compound represented by Formula 2.

In addition, an embodiment of the present invention provides a composition for organic layer of an organic light emitting device comprising the heterocyclic compound represented by Formula 1 and the heterocyclic compound represented by Formula 2.

Specific details of the heterocyclic compound represented by Formula 1 and the heterocyclic compound represented by Formula 2 are the same as described above.

In one embodiment of the present invention, a weight ratio of the heterocyclic compound represented by Formula 1 and the heterocyclic compound represented by Formula 2 in the composition for organic layer of the organic light emitting device may be 1:10 to 10:1, 1:8 to 8:1, 1:5 to 5:1, and 1:2 to 2:1, but is not limited thereto.

The composition for organic layer of the organic light emitting device may be used when forming an organic material of the organic light emitting device, and in particular, may be more preferably used when forming a host of the light emitting layer.

In one embodiment of the present invention, the organic layer includes the heterocyclic compound represented by Formula 1 and the heterocyclic compound represented by Formula 2, and may be used together with a phosphorescent dopant.

The phosphorescent dopant material may be those known in the art. For example, phosphorescent dopant materials represented by LL'MX', LL'L"M, LMX'X", L2MX' and L3M may be used, but the scope of the present invention is not limited to these examples.

M may be iridium, platinum, osmium, or the like.

L is an anionic bidentate ligand coordinated to M by sp² carbon and a hetero atom, and X above may function to trap electrons or holes. Non-limiting examples of L comprise 2-(1-naphthyl)benzoxazole, (2-phenylbenzoxazole), (2-phenylbenzothiazole), (7,8-benzoquinoline), (thiophenyl pyridine), phenylpyridine, benzothiophenyl pyridine, 3-methoxy-2-phenylpyridine, thiophene group pyridine, tolyl pyridine and the like. Non-limiting examples of X' and X" comprises acetylacetonate (acac), hexafluoroacetylacetonate, salicylidene, picolinate, 8-hydroxyquinolinate, and the like.

Specific examples of the phosphorescent dopant are shown below, but are not limited thereto:

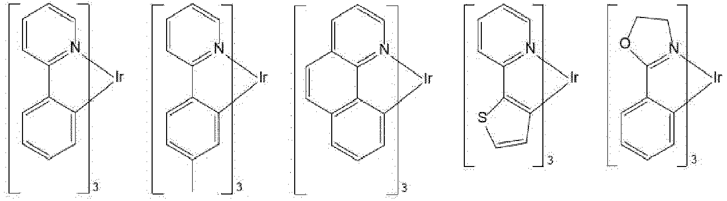

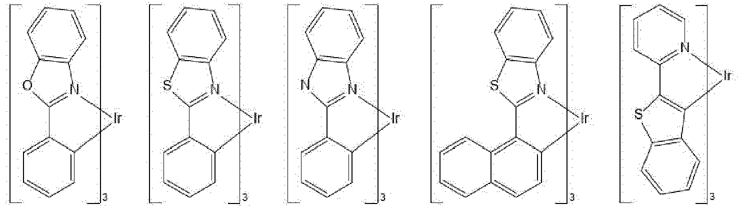

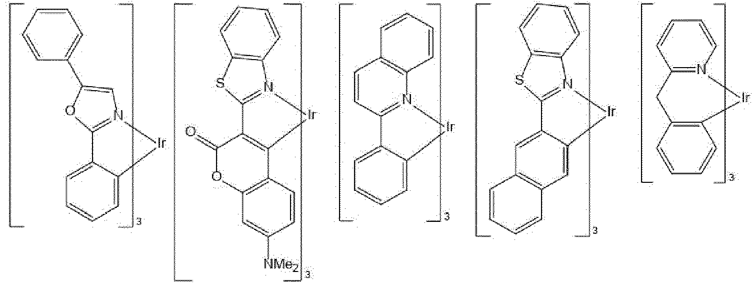

In one embodiment of the present invention, the organic layer comprises the heterocyclic compound represented by Formula 1 and the heterocyclic compound represented by Formula 2, and may be used together with an iridium-based dopant.

In one embodiment of the present invention, (piq)₂(Ir) (acac) which is a red phosphorescent dopant may be used as the iridium-based dopant.

In one embodiment of the present invention, Ir(ppy)₃, which is a green phosphorescent dopant, may be used as the iridium-based dopant.

In one embodiment of the present invention, the content of the dopant may be 1% to 15%, preferably 2% to 10%, more preferably 3% to 10%, based on the total of the light emitting layer.

In the organic light emitting device according to an embodiment of the present invention, the organic layer may comprise an electron injection layer or an electron transport layer, and the electron injection layer or the electron transport layer may comprise the heterocyclic compound.

In the organic light emitting device according to another embodiment, the organic layer may comprise an electron blocking layer or a hole blocking layer, and the electron blocking layer or the hole blocking layer may comprise the heterocyclic compound.

In the organic light emitting device according to another embodiment, the organic layer may comprise an electron transport layer, a light emitting layer, or a hole blocking layer, and the electron transport layer, the light emitting layer, or the hole blocking layer may comprise the heterocyclic compound.

The organic light emitting device according to an embodiment of the present invention may further comprise one layer or more layers selected from the group consisting of a light emitting layer, a hole injection layer, a hole transport layer, an electron injection layer, an electron transport layer, an electron blocking layer, and a hole blocking layer.

FIGs. 1 to 3 illustrate the stacking order of the electrode and the organic layer of the organic light emitting device according to an embodiment of the present invention. However, it is not intended that the scope of the present application be limited by these drawings, and the structure of the organic light emitting device known in the art may also be applied to the present application.

According to FIG. 1, an organic light emitting device formed by sequentially stacking a positive electrode 200, an organic layer 300, and a negative electrode 400 on a substrate 100 is shown. However, it is not limited to such a structure, and an organic light emitting device formed by sequentially stacking a negative electrode, an organic layer, and a positive electrode on a substrate may be implemented, as shown in FIG. 2.

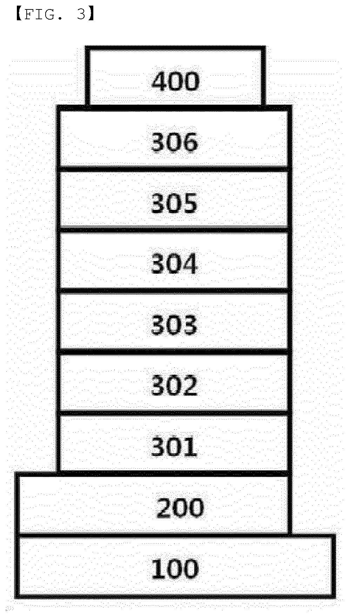

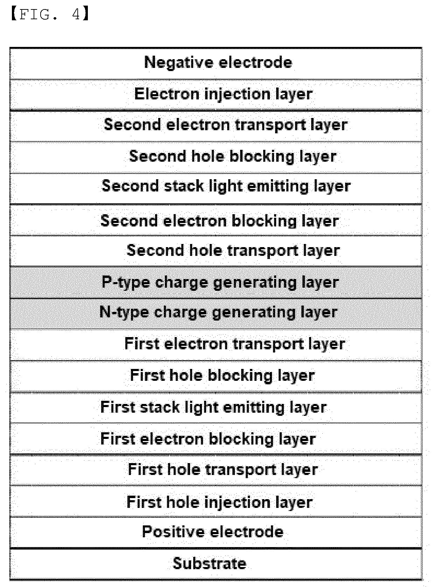

FIG. 3 illustrates a case in which the organic layer is composed of multiple layers. The organic light emitting device according to FIG. 3 comprises a hole injection layer 301, a hole transport layer 302, a light emitting layer 303, a hole blocking layer 304, an electron transport layer 305, and an electron injection layer 306. However, the scope of the present application is not limited by the stacked structure as described above, and if necessary, the remaining layers except for the light emitting layer may be omitted, and other necessary functional layers may be further added(e.g. as shown in Fig. 4).

In one embodiment of the present invention, the present invention provides a method of manufacturing an organic light emitting device comprising the steps of preparing a substrate; forming a first electrode on the substrate; forming one or more organic layers on the first electrode; and forming a second electrode on the one or more organic layers, wherein the step of forming the one or more organic layers comprises a step of forming the one or more organic layers using the composition for organic layer according to an embodiment of the present invention.

In one embodiment of the present invention, the step of forming the organic layer may comprise pre-mixing the heterocyclic compound represented by Formula 1 and the heterocyclic compound represented by Formula 2, and depositing it using a thermal vacuum deposition method.

The pre-mixing refers to first mixing the heterocyclic compound represented by Formula 1 and the heterocyclic compound represented by Formula 2 and putting them in one source to mix them, before deposition on the organic layer.

The pre-mixed material may be referred to as a composition for organic layer according to one embodiment of the present application.

The organic layer comprising the heterocyclic compound represented by Formula 1 may further comprise another material, if necessary.

The organic layer simultaneously comprising the heterocyclic compound represented by Formula 1 and Formula 2 may further comprise another material, if necessary.

In the organic light emitting device according to an embodiment of the present invention, the materials other than the heterocyclic compounds represented by Formula 1 or Formula 2 are exemplified below, but these are for illustrative purposes only and are not intended to limit the scope of the present application, and may be replaced by materials known in the art.

As the positive electrode material, materials having a relatively large work function can be used, and a transparent conductive oxide, metal, a conductive polymer or the like may be used. Specific examples of the positive electrode material may be, but is not limited to, metals such as vanadium, chromium, copper, zinc, and gold or alloys thereof; metal oxides such as zinc oxide, indium oxide, indium tin oxide (ITO), and indium zinc oxide (IZO); a combination of metal and oxide such as ZnO : Al or SnO₂ : Sb; conductive polymers such as poly(3-methylthiophene), poly[3,4-(ethylene-1,2-dioxy)thiophene] (PEDOT), polypyrrole, and polyaniline; and the like.

As the negative electrode material, materials having a relatively low work function may be used, and metal, metal oxide, a conductive polymer or the like may be used. Specific examples of the negative electrode material may be, but is not limited to, metals such as magnesium, calcium, sodium, potassium, titanium, indium, yttrium, lithium, gadolinium, aluminum, silver, tin, and lead or alloys thereof; multilayer-structured materials such as LiF/Al or LiO₂/Al; and the like.

As the hole injection layer material, a known material for the hole injection layer may be used, and for example, phthalocyanine compounds such as copper phthalocyanine disclosed in U.S. Patent No. 4,356,429, or starburst-type amine derivatives described in [Advanced Material, 6, p.677 (1994)], such as tris(4-carbazoyl-9-ylphenyl)amine(TCTA), 4,4',4"-tri[phenyl(m-tolyl)amino]triphenylamine(m-MTDATA), 1,3,5-tris[4-(3-methylphenylphenylamino)phenyl]benzene(m-MTDAPB), polyaniline/dodecylbenzenesulfonic acid which is a soluble conductive polymer, or poly(3,4-ethylenedioxythiophene)/poly(4-styrenesulfonate), polyaniline/camphor sulfonic acid, polyaniline/poly(4-styrene-sulfonate), or the like may be used.

As a material for the hole transport layer, a pyrazoline derivative, an arylamine-based derivative, a stilbene derivative, a triphenyldiamine derivative, or the like may be used, and a low-molecular or high-molecular material may be used.

As a material for the electron transport layer, metal complexes of oxadiazole derivatives, anthraquinodimethane and derivatives thereof, benzoquinone and derivatives thereof, naphthoquinone and its derivatives, anthraquinone and its derivatives, tetracyanoanthraquinodimethane and its derivatives, fluorenone derivatives, diphenyldicyanoethylene and its derivatives, diphenoquinone derivatives, 8-hydroxyquinoline and its derivatives, and the like may be used, and high-molecular materials as well as low-molecular materials may be also used.

As the electron injection layer material, for example, LiF is typically used in the art, but the present application is not limited thereto.

As a material for the light emitting layer, a red, green, or blue light emitting material may be used, and if necessary, two or more light emitting materials may be mixed and used. In this case, 2 or more luminescent materials may be used by deposition from separate sources, or may be pre-mixed and deposited from one source. In addition, as a material for the light emitting layer, a fluorescent material may be used, and a phosphorescent material may also be used. As a material for the light emitting layer, a material that emits light by combining holes and electrons respectively injected from the positive electrode and the negative electrode alone may be used, and materials in which the host material and the dopant material together participate in light emission may be also used.

If the hosts of the material for the light emitting layer are mixed and used, hosts of the same type may be mixed and used, or hosts of different types may be mixed and used. For example, any two or more types of n-type host material or p-type host material may be selected and used as a host material for the light emitting layer.

The organic light emitting device according to an embodiment of the present invention may be a top emission type, a bottom emission type, or a double side emission type depending on the material used.

The heterocyclic compound according to an embodiment of the present invention may also act in organic electronic devices including an organic solar cell, an organic photoreceptor, an organic transistor, and the like through the principle similar to that applied to the organic light emitting device.

Hereinafter, preferred examples are presented to help the understanding of the present invention. However, the following examples are provided only to make the present invention easier to understand, and the present invention is not limited thereto.

### <Preparation example>

### <Preparation Example 1> Preparation of intermediate A

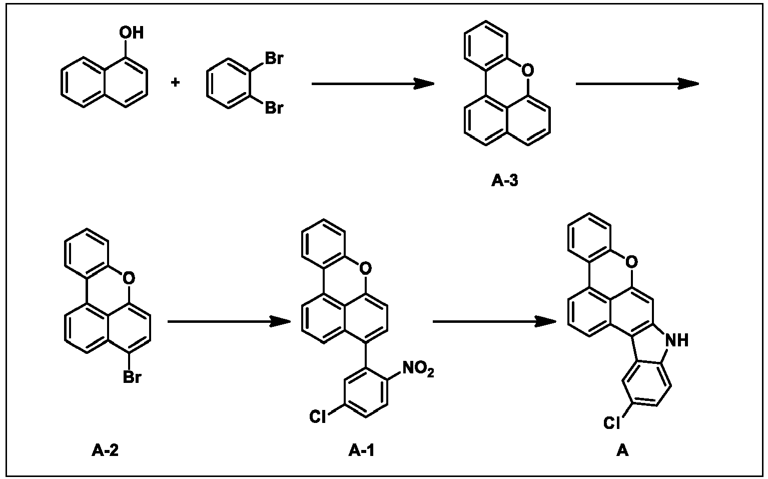

### 1) Preparation of Intermediate A-3

Naphthalene-1-ol (50.0g, 347mmol), 1,2-dibromobenzene (53.5mL, 416mmol), palladium acetate (Pd(OAc)₂) (15.6g, 17.4mmol), triphenylphosphine (PPh₃) (72.8g, 69.6mmol), CsCO₃(452g, 1390mmol), and dimethylformamide (DMF) (500mL) were placed in a 1 L round-bottom flask, and stirred at 140 °C under nitrogen condition for 24 hours. After the reaction was completed, the temperature was cooled to room temperature, and filtration was performed using a Celite filter. It was extracted with distilled water, brine, and methylene chloride (MC). The solution was dried over anhydrous MgSO₄ and concentrated. The concentrated reaction product was purified by column under the condition of MC:hexane=1:4 to obtain intermediate A-3 (57.5g, 264mmol, 76%).

### 2) Preparation of intermediate A-2

Intermediate A-3(57.5g, 264mmol) and N-Bromosuccinimide (NBS) (46.9g, 264mmol) were placed in a 1L round-bottom flask, dissolved in acetonitrile (570 mL) and then stirred at room temperature for 4 hours. After the reaction was completed, extraction was performed with MC, brine, sodium thiosulfate (aq), and distilled water. The solution was dried over anhydrous MgSO₄ and concentrated. The concentrated reaction product was purified by column under the condition of MC:hexane=1:4 to obtain intermediate A-2(72.8g, 245mmol, 93%).

### 3) Preparation of intermediate A-1

Intermediate A-2(20.0g, 67.3mmol) and (5-chloro-2-nitrophenyl)boronic acid (14.9g, 74.0mmol), tetrakis(triphenylhosphine)palladium(0) (Pd(PPh₃)₄) (3.89g, 3.37mmol), and K₂CO₃(18.6g, 135mmol) were placed in a 1L round-bottom flask, dissolved in H₂O (60 mL) and 1,4-dioxane (200 mL) and then refluxed for 4 hours. After the reaction was completed, it was cooled to room temperature, and washed with distilled water and methanol. The solid was dried, and then separated by column under the condition of MC:hexane=1:4 to obtain intermediate A-1(21.6g, 57.9mmol, 86%).

### 4) Preparation of intermediate A

Intermediate A-1(21.6g, 57.9mmol), triphenylphosphine (PPh₃) (30.4g, 116mmol), and 1,2-dichlorobenzene (1,2-DCB) (220mL) were placed in a 500 mL round-bottom flask, and refluxed for 8 hours. After the reaction was completed, it was cooled to room temperature, and filtration was performed using a Celite filter. The solution was dried with a rotary evaporator, and then separated by column under the condition of MC:hexane=1:3 to obtain intermediate A(16.4g, 48.0mmol, 83%).

### <Preparation Example 2> Preparation of intermediate B

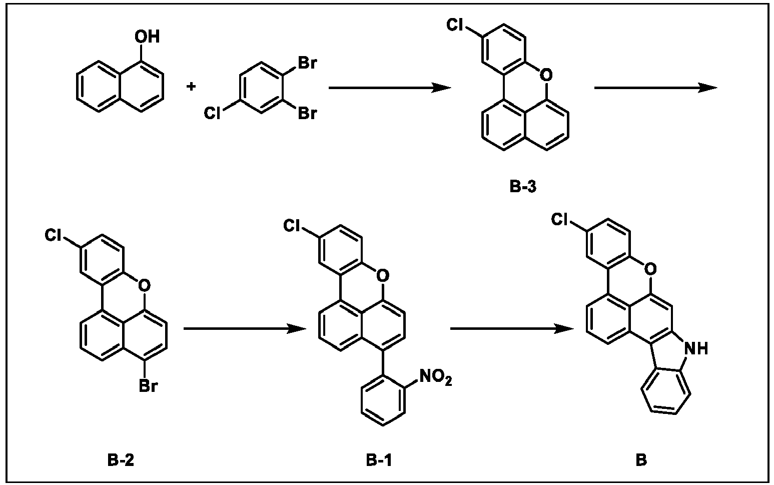

### 1) Preparation of intermediate B-3

Naphthalene-1-ol (30.0g, 208mmol), 1,2-dibromo-4-chlorobenzene (67.5mL, 250mmol), palladium acetate (Pd(OAc)₂) (2.33g, 10.4mmol), triphenylphosphine (PPh₃) (10.9g, 41.6mmol), CsCO₃ (271g, 832mmol), dimethylformamide (DMF) (300mL) were placed in a 1L round-bottom flask, and stirred at 140 °C under nitrogen condition for 24 hours. After the reaction was completed, the temperature was cooled to room temperature, and filtration was performed using a Celite filter. It was extracted with distilled water, brine, and MC. The solution was dried over anhydrous MgSO₄ and concentrated. The concentrated reaction product was purified by column under the condition of MC:hexane=1:4 to obtain intermediate B-3(34.7g, 137mmol, 66%).

### 2) Preparation of intermediate B-2

Intermediate B-3(34.7g, 137mmol) and N-Bromosuccinimide (NBS) (24.4g, 137mmol) were placed in a 1L round-bottom flask, and dissolved in acetonitrile (350 mL), and stirred at room temperature for 3 hours. After the reaction was completed, extraction was performed with MC, brine, sodium thiosulfate (aq), and distilled water. The solution was dried over anhydrous MgSO₄ and concentrated. The concentrated reaction product was purified by column under the condition of MC:hexane=1:4 to obtain intermediate B-2(72.8g, 130mmol, 95%).

### 3) Preparation of intermediate B-1

Intermediate B-2(72.8g, 130mmol) and (2-nitrophenyl)boronic acid (40.3g, 143mmol), tetrakis(triphenylhosphine)palladium(0) (Pd(PPh₃)₄) (7.51g, 6.5mmol), and K₂CO₃(35.9g, 260mmol) were placed in a 2L round-bottom flask, dissolved in H₂O (200 mL), and 1,4-dioxane (730 mL), and then refluxed for 5 hours. After the reaction was completed, it was cooled to room temperature, and washed with distilled water and methanol. The solid was dried, and then separated by column under the condition of MC:hexane=1:4 to obtain intermediate B-1(68.9g, 184mmol, 84%).

### 4) Preparation of intermediate B

Intermediate B-1(68.9g, 184mmol), triphenylphosphine (PPh₃) (96.5g, 368mmol), and 1,2-dichlorobenzene (1,2-DCB) (700mL) were placed in a 1L round-bottom flask, and refluxed for 15 hours. After the reaction was completed, it was cooled to room temperature, and filtration was performed using a Celite filter. The solution was dried with a rotary evaporator, and then separated by a column under the condition of MC:hexane=1:4 to obtain intermediate B(50.4g, 147mmol, 83%).

### <Preparation Example 3> Preparation of intermediate C

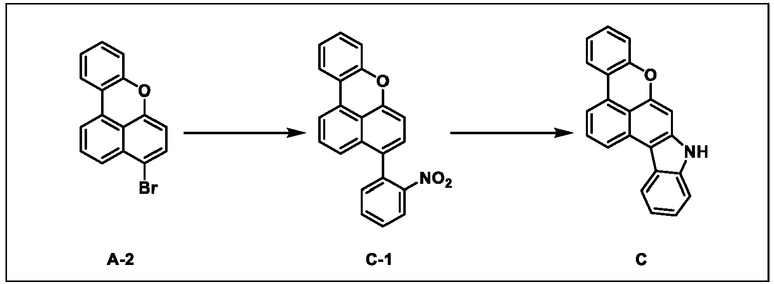

### 1) Preparation of intermediate C-1

Intermediate A-2(10.0g, 33.7mmol) and (2-nitrophenyl)boronic acid (6.18g, 37.0mmol), tetrakis(triphenylhosphine)palladium(0) (Pd(PPh₃)₄) (1.95g, 1. 69mmol), and K₂CO₃(9.32g, 67.4mmol) were placed in a 250 mL round-bottom flask, dissolved in H₂O (30 mL) and 1,4-dioxane (100 mL) and then refluxed for 3 hours. After the reaction was completed, it was cooled to room temperature, and washed with distilled water and methanol. The solid was dried, and then separated by column under the condition of MC:hexane=1:4 to obtain intermediate C-1(9.95g, 29.3mmol, 87%).

### 2) Preparation of intermediate C

Intermediate C-1 (9.95g, 29.3mmol), triphenylphosphine (PPh₃) (15.4g, 58.6mmol), and 1,2-dichlorobenzene (1,2-DCB) (100mL) were placed in a 250 mL round-bottom flask, and refluxed for 7 hours. After the reaction was completed, it was cooled to room temperature, and filtration was performed using a Celite filter. The solution was dried with a rotary evaporator, and then separated by a column under the condition of MC:hexane=1:4 to obtain intermediate C(8.01g, 26.1mmol, 89%).

### <Preparation Example 4> Preparation of intermediate D

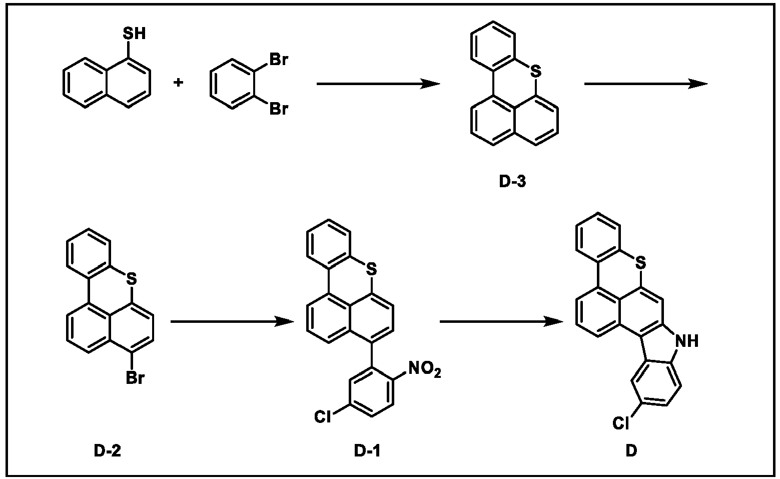

### 1) Preparation of intermediate D-3

Naphthalene-1-thiol (10.0g, 62.4mmol), 1,2-dibromobenzene (8.9mL, 74.9mmol), palladium acetate (Pd(OAc)₂) (0.70g, 3.12mmol), triphenylphosphine (PPh₃) (3.27g, 12.5mmol), CsCO₃ (81.3g, 250mmol), and dimethylformamide (DMF) (100mL) were placed in a 1L round-bottom flask, and stirred at 140 °C under nitrogen condition for 24 hours. After the reaction was completed, the temperature was cooled to room temperature, and filtration was performed using a Celite filter. It was extracted with distilled water, brine, and MC. The solution was dried over anhydrous MgSO₄ and concentrated. The concentrated reaction product was purified by column under the condition of MC:hexane=1:4 to obtain intermediate D-3(9.36g, 39.9mmol, 64%).

### 2) Preparation of intermediate D-2

Intermediate D-3(9.36g, 39.9mmol) and N-bromosuccinimide (NBS) (7.10g, 39.9mmol) were placed in a 250 mL round-bottom flask, dissolved in acetonitrile (95 mL), and stirred at room temperature for 3 hours. After the reaction was completed, extraction was performed with MC, brine, sodium thiosulfate (aq), and distilled water. The solution was dried over anhydrous MgSO₄ and concentrated. The concentrated reaction product was purified by column under the condition of MC:hexane=1:4 to obtain intermediate D-2(11.2g, 35.9mmol, 90%).

### 3) Preparation of intermediate D-1

Intermediate D-2(11.2g, 35.9mmol) and (5-chloro-2-nitrophenyl)boronic acid (7.95g, 39.5mmol), tetrakis(triphenylhosphine)palladium(0) (Pd(PPh₃)₄) (2.07g, 1.80mmol), and K₂CO₃ (9.92g, 71.8mmol) were placed in a 1L round-bottom flask, dissolved in H₂O (60 mL) and 1,4-dioxane (200 mL), and then refluxed for 4 hours. After the reaction was completed, it was cooled to room temperature, and washed with distilled water and methanol. The solid was dried, and then separated by column under the condition of MC:hexane=1:4 to obtain intermediate D-1(11.3g, 29.1mmol, 81%).

### 4) Preparation of intermediate D

Intermediate D-1(11.3g, 29.1mmol), triphenylphosphine (PPh₃) (15.3g, 58.2mmol), and 1,2-dichlorobenzene (1,2-DCB) (110mL) were placed in a 250 mL round-bottom flask, and refluxed for 9 hours. After the reaction was completed, it was cooled to room temperature, and filtered through Celite. The solution was dried with a rotary evaporator, and then separated by column under the condition of MC:hexane=1:3 to obtain intermediate D(8.12g, 22.7mmol,78%).

### <Preparation Example 5> Preparation of intermediate E

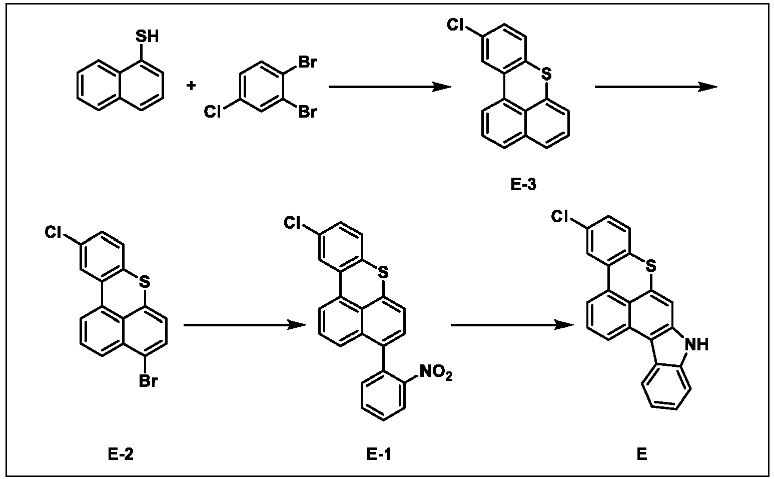

### 1) Preparation of intermediate E-3

Naphthalene-1-thiol (10.0g, 62.4mmol), 1,2-dibromo-4-chlorobenzene (10.1mL, 74.9mmol), palladium acetate (Pd(OAc)₂) (0.70g, 3.12mmol), triphenylphosphine (PPh₃) (3.27g, 12.5mmol), CsCO₃(81.3g, 250mmol), dimethylformamide (DMF) (100 mL) were placed in a 250 mL round-bottom flask, and stirred at 140 °C under nitrogen condition for 24 hours. After the reaction was completed, the temperature was cooled to room temperature, and filtration was performed using a Celite filter. It was extracted with distilled water, brine, and MC. The solution was dried over anhydrous MgSO₄ and concentrated. The concentrated reaction product was purified by column under the condition of MC:hexane=1:4 to obtain intermediate E-3(8.55g, 31.8mmol, 51%).

### 2) Preparation of intermediate E-2

Intermediate E-3 (8.55g, 31.8mmol) and N-bromosuccinimide (NBS) (5.66g, 31.8mmol) were placed in a 1L round-bottom flask, and dissolved in acetonitrile (85 mL), and then stirred at room temperature for 3 hours. After the reaction was completed, extraction was performed with MC, brine, sodium thiosulfate (aq), and distilled water. The solution was dried over anhydrous MgSO₄ and concentrated. The concentrated reaction product was purified by column under the condition of MC:hexane=1:4 to obtain intermediate E-2(9.73g, 28.0mmol, 88%).

### 3) Preparation of intermediate E-1

Intermediate E-2 (9.73g, 28.0mmol) and (2-nitrophenyl)boronic acid (5.14 g, 30.8 mmol), tetrakis(triphenylhosphine)palladium(0) (Pd(PPh₃)₄) (1.62g, 1.40mmol), and K₂CO₃ (7.74g, 56.0mmol) were placed in a 250 mL round-bottom flask, dissolved in H₂O (30 mL) and 1,4-dioxane (100 mL), and then refluxed for 6 hours. After the reaction was completed, it was cooled to room temperature, and washed with distilled water and methanol. The solid was dried, and then separated by column under the condition of MC:hexane=1:4 to obtain intermediate E-1(9.72g, 24.9mmol, 89%).

### 4) Preparation of intermediate E

Intermediate E-1(9.72g, 24.9mmol), PPh₃(13.1g, 49.8mmol), 1,2-dichlorobenzene (1,2-DCB) (250mL) were placed in a 500 mL round-bottom flask, and refluxed for 17 hours. After the reaction was completed, it was cooled to room temperature, and filtration was performed using a Celite filter. The solution was dried with a rotary evaporator, and then separated by a column under the condition of MC:hexane=1:4 to obtain intermediate E (7.04g, 19.7mmol, 79%).

### <Preparation Example 6> Preparation of intermediate F

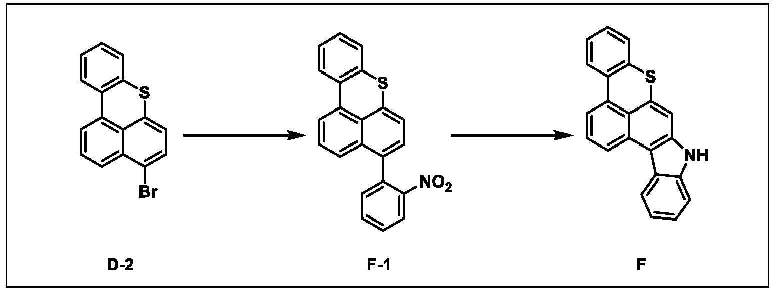

### 1) Preparation of intermediate F-1

Intermediate D-2 (10.0g, 31.9mmol) and (2-nitrophenyl)boronic acid (10.7g, 63.8mmol), tetrakis(triphenylhosphine)palladium(0) (Pd(PPh₃)₄) (1.85g, 1.60mmol), and K₂CO₃ (8.82g, 63.8mmol) were placed in a 250 mL round-bottom flask, dissolved in H₂O (30 mL) and 1,4-dioxane (100 mL), and the refluxed for 2 hours. After the reaction was completed, it was cooled to room temperature, and washed with distilled water and methanol. The solid was dried, and then separated by column under the condition of MC:hexane=1:4 to obtain intermediate F-1(10.4g, 29.3mmol, 92%).

### 2) Preparation of intermediate F

Intermediate F-1 (10.4g, 29.3mmol), triphenylphosphine (PPh₃) (15.4g, 58.6mmol), and 1,2-dichlorobenzene (1,2-DCB) (100mL) were placed in a 250 mL round-bottom flask, and refluxed for 4 hours. After the reaction was completed, it was cooled to room temperature, and filtration was performed using a Celite filter. The solution was dried with a rotary evaporator, and then separated by a column under the condition of MC:hexane=1:4 to obtain intermediate F(7.58g, 23.4mmol, 80%).

### <Preparation Example 7> Preparation of intermediate G

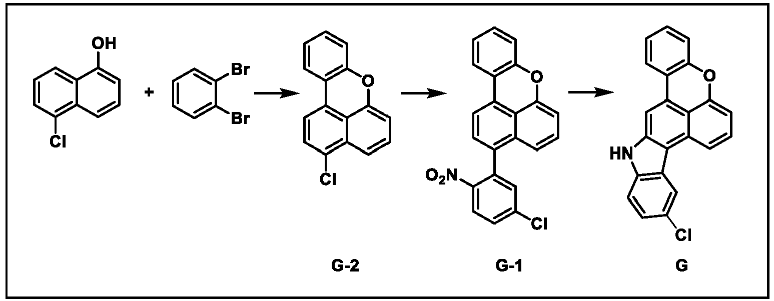

### 1) Preparation of intermediate G-2

5-chloronaphthalen-1-ol (10.0g, 56.6mmol), 1,2-dibromobenzene (8.0mL, 67.9mmol), palladium acetate (Pd(OAc)₂) (0.64g, 2.83mmol), PPh₃ (2.97g, 11.3mmol), CsCO₃ (73.6g, 226mmol), and dimethylformamide (DMF) (100mL) were placed in a 1L round-bottom flask, and stirred at 140 °C under nitrogen condition for 24 hours. After the reaction was completed, the temperature was cooled to room temperature, and filtration was performed using a Celite filter. It was extracted with distilled water, brine, and MC. The solution was dried over anhydrous MgSO₄ and concentrated. The concentrated reaction product was purified by column under the condition of MC:hexane=1:4 to obtain intermediate G-2(6.29g, 24.9mmol, 44%).

### 2) Preparation of intermediate G-1

Intermediate G-2 (6.29g, 24.9mmol) and (5-chloro-2-nitrophenyl)boronic acid (10.0g, 49.8mmol), tetrakis(triphenylhosphine)palladium(0) (Pd(PPh₃)₄) (1.44g, 1.25mmol), K₂CO₃ (6.88g, 49.8mmol) were placed in a 250 mL round-bottom flask, dissolved in H₂O (19 mL), 1,4-dioxane (63 mL), and then refluxed for 5 hours. After the reaction was completed, it was cooled to room temperature, and washed with distilled water and methanol. The solid was dried, and then separated by column under the condition of MC:hexane=1:4 to obtain intermediate G-1 (6. 79g, 18.2mmol, 73%) .

### 3) Preparation of intermediate G

Intermediate G-1 (6.79g, 18.2mmol), triphenylphosphine (PPh₃) (9.55 g, 36.4 mmol), and 1,2-dichlorobenzene (1,2-DCB) (68 mL) were placed in a 250 mL round-bottom flask, and refluxed for 10 hours. After the reaction was completed, it was cooled to room temperature, and filtration was performed using a Celite filter. The solution was dried with a rotary evaporator, and then separated by column under the condition of MC:hexane=1:3 to obtain intermediate G (4.35g, 12.7mmol,70%).

### <Preparation Example 8> Preparation of intermediate H

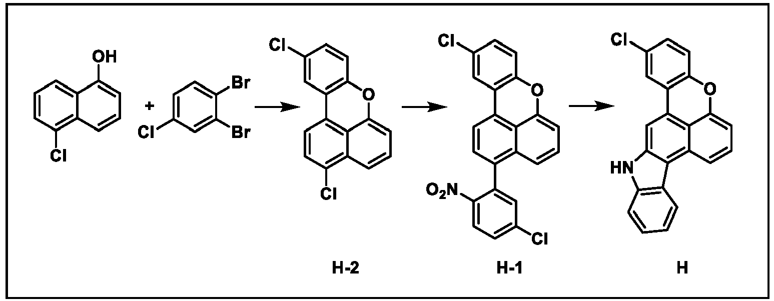

### 1) Preparation of intermediate H-2

5-chloronaphthalen-1-ol (10.0g, 56.6mmol), 1,2-dibromo-4-chlorobenzene (9.2mL, 67.9mmol), palladium acetate (Pd(OAc)₂) (0.64g, 2.83mmol), triphenylphosphine (PPh₃) (2.97g, 11.3mmol), CsCO₃(73.8g, 226mmol), dimethylformamide (DMF) (100 mL) were placed in a 250 mL round-bottom flask, and stirred for 25 hours at 140 °C under nitrogen condition. After the reaction was completed, the temperature was cooled to room temperature, and filtration was performed using a Celite filter. It was extracted with distilled water, brine, and MC. The solution was dried over anhydrous MgSO₄ and concentrated. The concentrated reaction product was purified by column under the condition of MC:hexane=1:4 to obtain intermediate H-2(6.99g, 24.3mmol, 43%).

### 2) Preparation of intermediate H-1

Intermediate H-2(6.99g, 24.3mmol) and (2-nitrophenyl)boronic acid (9.79g, 48.6mmol), tetrakis(triphenylhosphine)palladium(0) (Pd(PPh₃)₄) (1.40g, 1.22mmol), K₂CO₃ (6.72g, 48.6mmol) were placed in a 250 mL round-bottom flask, dissolved in H₂O (21 mL), and 1,4-dioxane (70 mL), and then refluxed for 5 hours. After the reaction was completed, it was cooled to room temperature, and washed with distilled water and methanol. The solid was dried, and then separated by column under the condition of MC:hexane=1:4 to obtain intermediate H-1(7.44g, 18.2mmol, 75%).

### 3) Preparation of intermediate H

Intermediate H-1 (7.44g, 18.2mmol), triphenylphosphine (PPh₃) (9.55g, 36.4mmol), and 1,2-Dichlorobenzene (1,2-DCB) (75mL) were placed in a 250 mL round-bottom flask, and refluxed for 15 hours. After the reaction was completed, it was cooled to room temperature, and filtration was performed using a Celite filter. The solution was dried with a rotary evaporator, and then separated by a column under the condition of MC:hexane=1:4 to obtain intermediate H(3.23g, 9.46mmol, 52%).

### <Preparation Example 9> Preparation of intermediate I

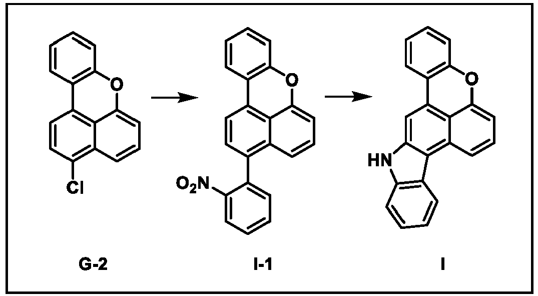

### 1) Preparation of intermediate I-1

Intermediate G-2 (10.0g, 39.6mmol) and (2-nitrophenyl)boronic acid (13.2g, 79.1mmol), tetrakis(triphenylhosphine)palladium(0) (Pd(PPh₃)₄) (2.29g, 1.98mmol), K₂CO₃ (10.9g, 79.2mmol) were placed in a 250 mL round-bottom flask, dissolved in H₂O (30 mL), 1,4-dioxane (100 mL), and then refluxed for 3 hours. After the reaction was completed, it was cooled to room temperature, and washed with distilled water and methanol. The solid was dried, and then separated by column under the condition of MC:hexane=1:4 to obtain intermediate I-1(12.8g, 37.6mmol, 95%).

### 2) Preparation of intermediate I

Intermediate I-1 (12.8g, 37.6mmol), triphenylphosphine (PPh₃) (19.7g, 75.2mmol), and 1,2-dichlorobenzene (1,2-DCB) (130mL) were placed in a 500 mL round-bottom flask, and refluxed for 6 hours. After the reaction was completed, it was cooled to room temperature, and filtration was performed using a Celite filter. The solution was dried with a rotary evaporator, and then separated by a column under the condition of MC:hexane=1:4 to obtain intermediate I(9.36g, 30.5mmol, 81%).

### <Preparation Example 10> Preparation of intermediate J

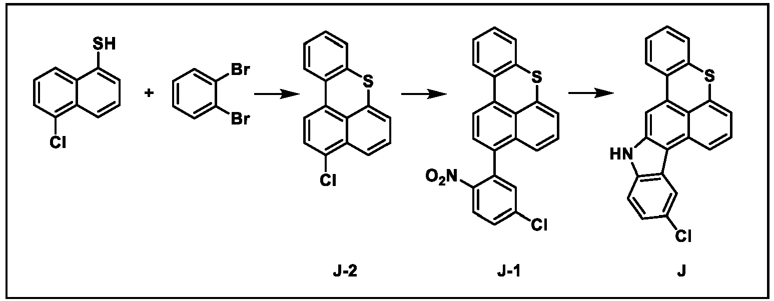

### 1) Preparation of intermediate J-2

5-chloronaphthalene-1-thiol (10.0g, 51.4mmol), 1,2-dibromobenzene (7.8mL, 61.6mmol), palladium acetate (Pd(OAc)₂) (0.58g, 2.57mmol), PPh₃ (2.70g, 10.3mmol), CsCO₃ (67.0g, 206mmol), and dimethylformamide (DMF) (100mL) were placed in a 1L round-bottom flask, and stirred at 140 °C for 26 hours under nitrogen condition. After the reaction was completed, the temperature was cooled to room temperature, and filtration was performed using a Celite filter. It was extracted with distilled water, brine, and MC. The solution was dried over anhydrous MgSO₄ and concentrated. The concentrated reaction product was purified by column under the condition of MC:hexane=1:4 to obtain intermediate J-2 (5.39g, 20.0mmol, 39%).

### 2) Preparation of intermediate J-1

Intermediate J-2(5.39g, 20.0mmol) and (5-chloro-2-nitrophenyl)boronic acid (8.05g, 40.0mmol), tetrakis(triphenylhosphine)palladium(0) (Pd(PPh₃)₄) (1.16g, 1.00mmol), and K₂CO₃ (5.53g, 40.0mmol) were placed in a 250 mL round-bottom flask, dissolved in H₂O (16 mL) and 1,4-dioxane (54 mL), and then refluxed for 4 hours. After the reaction was completed, it was cooled to room temperature, and washed with distilled water and methanol. The solid was dried, and then separated by column under the condition of MC:hexane=1:4 to obtain intermediate J-1(7.56g, 19.4mmol, 97%).

### 3) Preparation of intermediate J

Intermediate J-1(7.56g, 19.4mmol), triphenylphosphine (PPh₃) (10.2g, 38.8mmol), and 1,2-Dichlorobenzene (1,2-DCB) (75mL) were placed in a 250 mL round-bottom flask, and refluxed for 8 hours. After the reaction was completed, it was cooled to room temperature, and filtration was performed using a Celite filter. The solution was dried with a rotary evaporator, and then separated by column under the condition of MC:hexane=1:3 to obtain intermediate J(5.69g, 15.9mmol,82%).

### <Preparation Example 11> Preparation of intermediate K

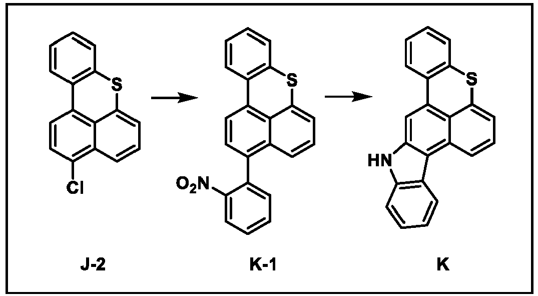

### 1) Preparation of intermediate K-1

Intermediate J-2 (10.0g, 37.2mmol) and (2-nitrophenyl)boronic acid (12.4g, 74.4mmol), tetrakis(triphenylhosphine)palladium(0) (Pd(PPh₃)₄) (2.15g, 1.86mmol), and K₂CO₃ (10.3g, 74.4mmol) were placed in a 250 mL round-bottom flask, were dissolved in H₂O (30 mL) and 1,4-dioxane (100 mL) and refluxed for 2 hours. After the reaction was completed, it was cooled to room temperature, and washed with distilled water and methanol. The solid was dried, and then separated by column under the condition of MC:hexane=1:4 to obtain intermediate K-1 (12.0g, 33.9mmol, 91%).

### 2) Preparation of intermediate K

Intermediate K-1 (12.0g, 33.9mmol), triphenylphosphine (PPh₃) (17.8g, 67.8mmol), and 1,2-dichlorobenzene (1,2-DCB) (120mL) were placed in a 500 mL round-bottom flask, and refluxed for 7 hours. After the reaction was completed, it was cooled to room temperature, and filtration was performed using a Celite filter. The solution was dried with a rotary evaporator, and then separated by a column under the condition of MC:hexane=1:4 to obtain intermediate K(8.55g, 26.4mmol, 78%).

### <Preparation Example 12> Preparation of compound 1

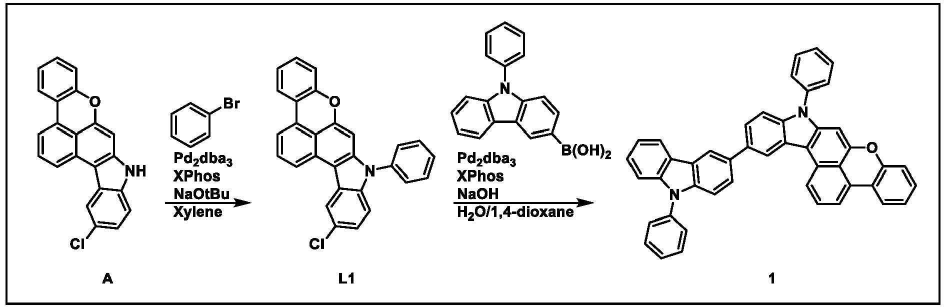

### 1) Preparation of intermediate L1

Intermediate A(7.00g, 20.5mmol), bromobenzene (2.6mL, 24.6mmol), tris(dibenzylideneacetone)dipalladium (Pd₂(dba)₃) (0.94g, 1.03mmol), XPhos (0.98g, 2.05mmol), sodium tert-butoxide (NaOtBu) (3.94g, 41.0mmol), and xylene (70mL) were placed in a 250 mL round-bottom flask, and stirred at 130 °C for 3 hours. After the reaction was completed, the temperature was cooled to room temperature, and filtration was performed using a Celite filter. It was extracted with distilled water, brine, and MC. The solution was dried over anhydrous MgSO₄ and concentrated. The concentrated reaction product was purified by a column under the condition of MC:hexane=1:3 to obtain intermediate L1 (8.40g, 20.1 mmol, 98%).

### 2) Preparation of compound 1

Intermediate L1 (8.40g, 20.1mmol), (9-phenyl-9H-carbazol-3-yl)boronic acid (6.35g, 22.1mmol), tris(dibenzylideneacetone)dipalladium (Pd₂(dba)₃) (0.92g, 1.01mmol), XPhos (0.96g, 2.01mmol), NaOH (16.1g, 40.2mmol), water (25mL), and xylene (85mL) were placed in a 250 mL round-bottom flask, and stirred at 130 °C for 4 hours. After the reaction was completed, the temperature was cooled to room temperature, and filtration was performed using a Celite filter. It was extracted with distilled water, brine, and MC. The solution was dried over anhydrous MgSO₄ and concentrated. The concentrated reaction product was purified by a column under the condition of MC:hexane=1:3 to obtain compound 1 (11.5g, 18.4mmol, 92%) .

The following target compounds (P) were prepared in the same manner as in Preparation Example 12, except that in Preparation Example 12, intermediates M and N in Table 1 below were used instead of intermediate A, bromobenzene, and (9-phenyl-9H-carbazol-3-yl)boronic acid.

**Table 1:**

| Intermediate | M | N | Target compound (P) | Yield |
|---|---|---|---|---|
| 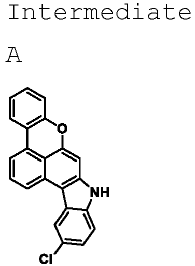 | 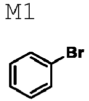 | 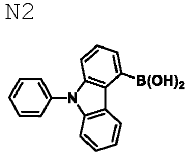 | 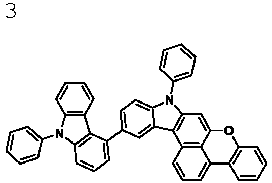 | 91% |
| 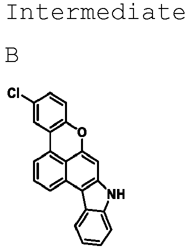 | 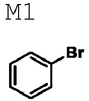 | 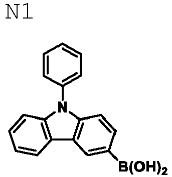 | 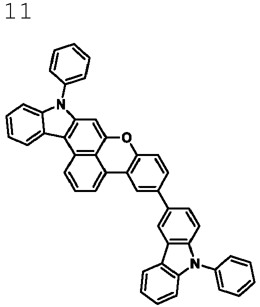 | 88% |
| 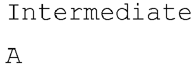 | 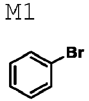 | 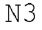 | 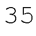 | 87% |
| 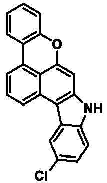 | | 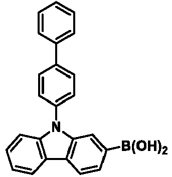 | 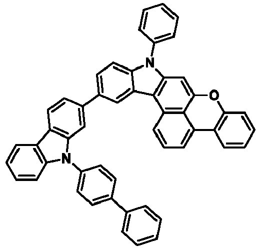 | |
| 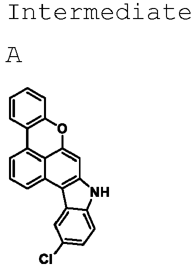 | 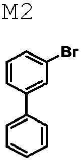 | 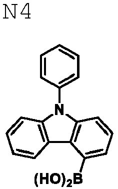 | 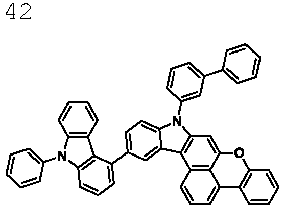 | 84% |
| 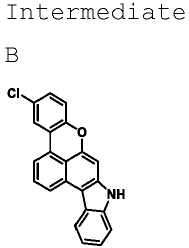 | 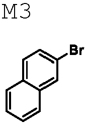 | 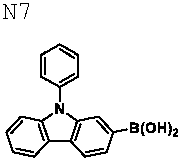 | 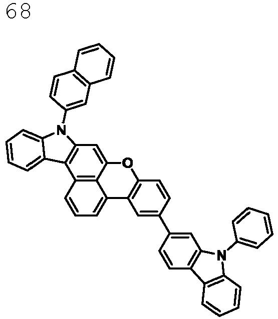 | 86% |
| 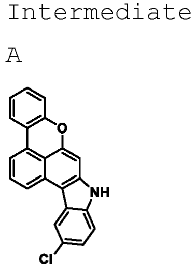 | 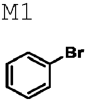 | 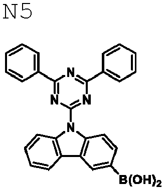 | 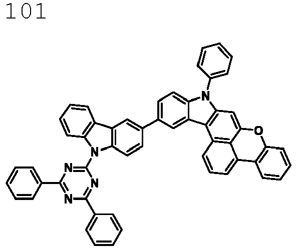 | 79% |
| 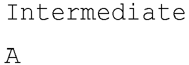 | 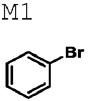 | 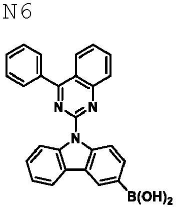 | 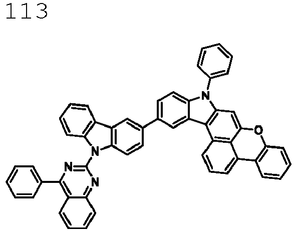 | 81% |
| 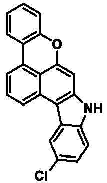 | | | | |
| 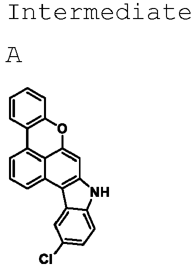 | 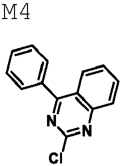 | 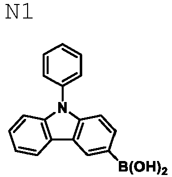 | 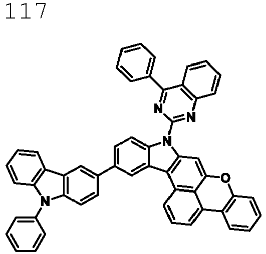 | 85% |
| 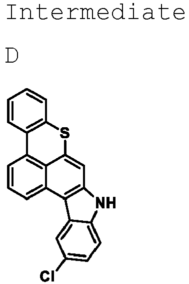 | 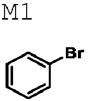 | 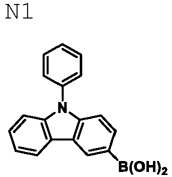 | 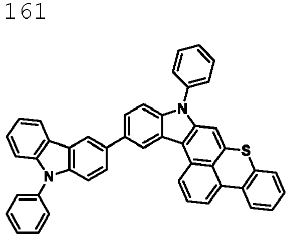 | 90% |
| 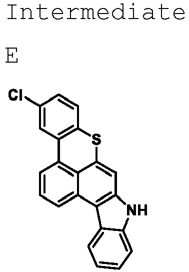 | 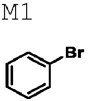 | 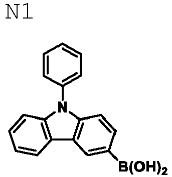 | 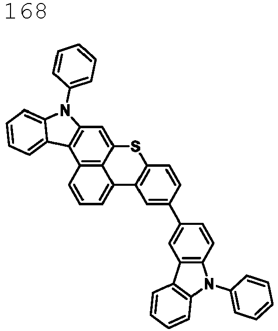 | 92% |
| 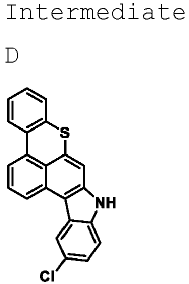 | 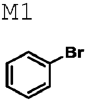 | 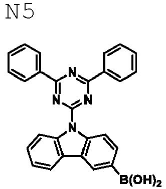 | 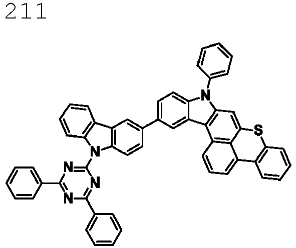 | 86% |
| 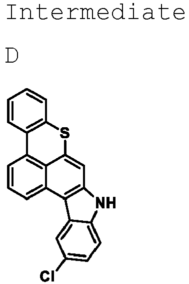 | 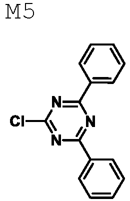 | 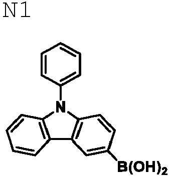 | 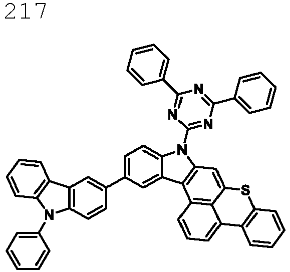 | 85% |
| 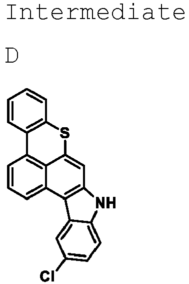 | 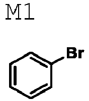 | 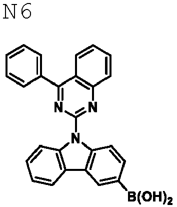 | 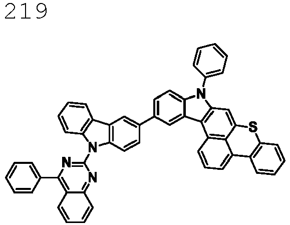 | 87% |
| 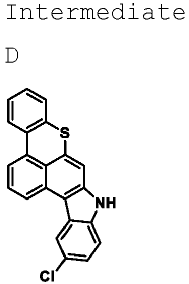 | 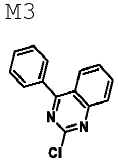 | 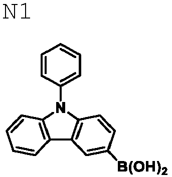 | 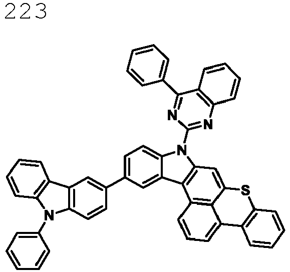 | 84% |
| 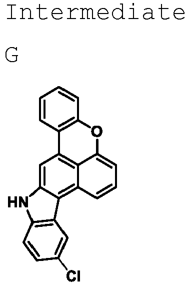 | 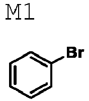 | 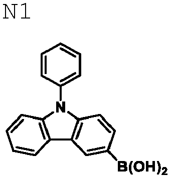 | 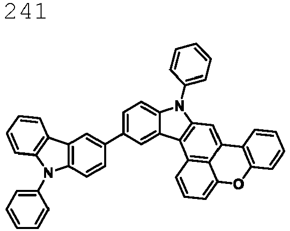 | 91% |
| 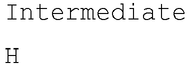 | 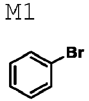 | 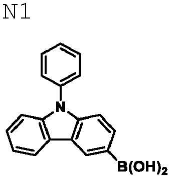 | 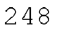 | 88% |
| 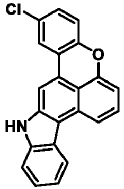 | | | 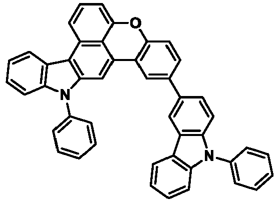 | |
| 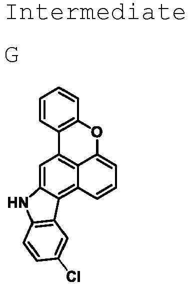 | 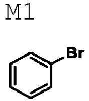 | 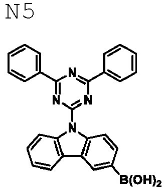 | 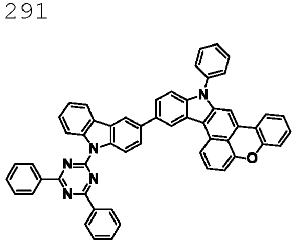 | 81% |
| 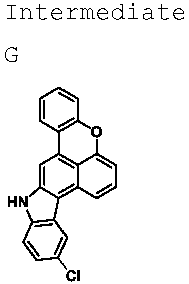 | 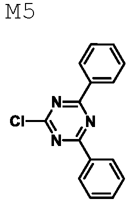 | 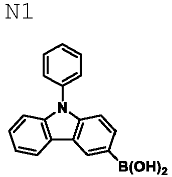 | 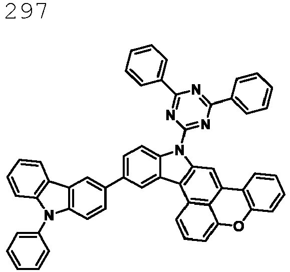 | 85% |
| 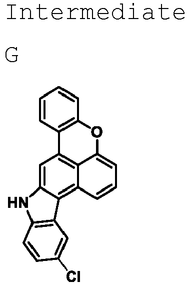 | 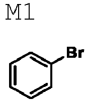 | 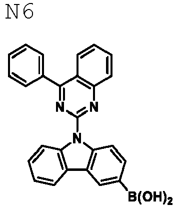 | 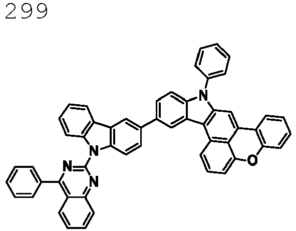 | 84% |
| 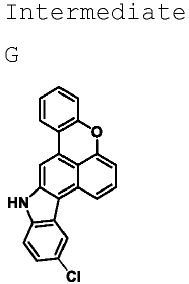 | 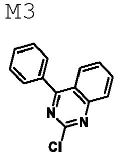 | 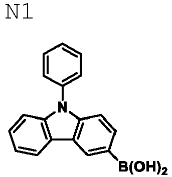 | 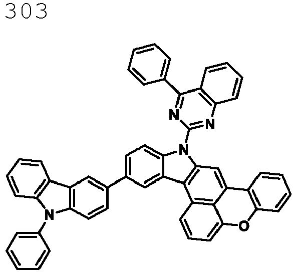 | 86% |
| 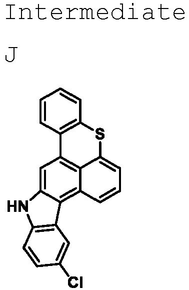 | 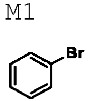 | 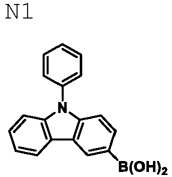 | 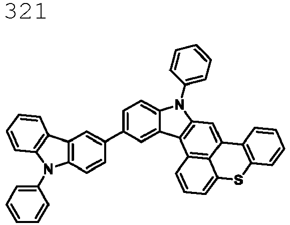 | 90% |
| 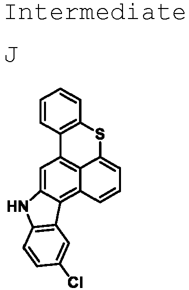 | 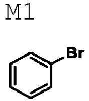 | 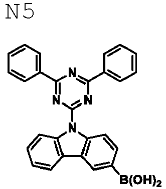 | 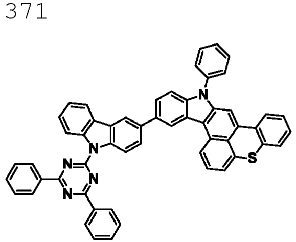 | 87% |
| 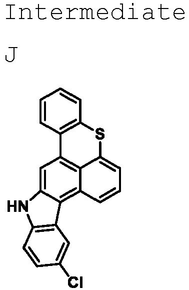 | 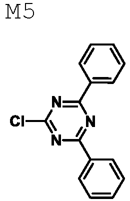 | 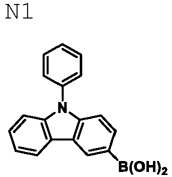 | 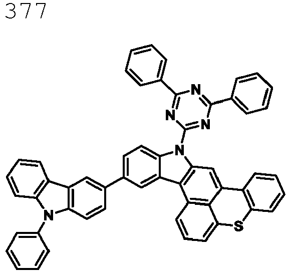 | 84% |
| 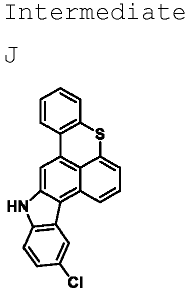 | 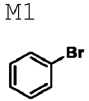 | 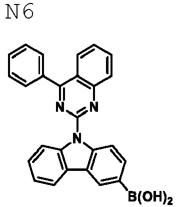 | 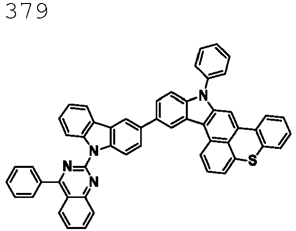 | 79% |
| 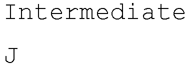 | 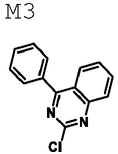 | 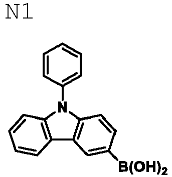 | 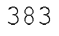 | 81% |
| 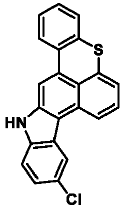 | | | 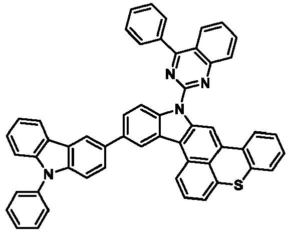 | |
| 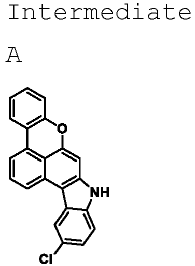 | 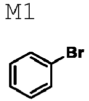 | 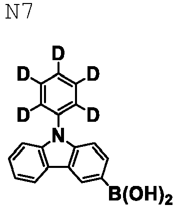 | 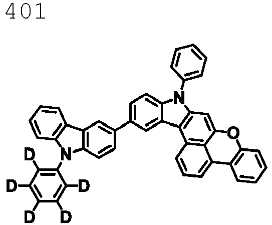 | 84% |

### <Preparation Example 13> Preparation of compound 18

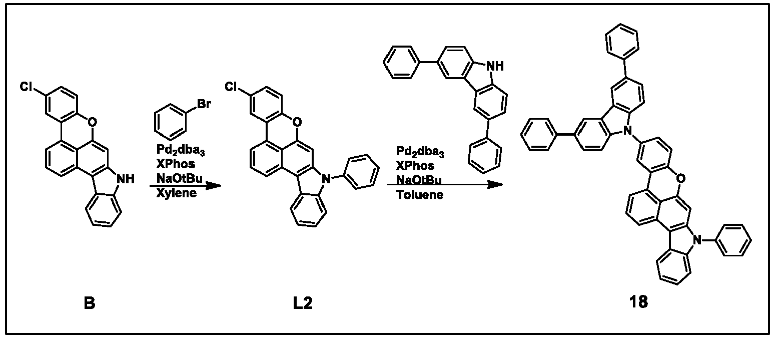

### 1) Preparation of intermediate L2

Intermediate B (7.00g, 20.5mmol), bromobenzene (2.6mL, 24.6mmol), tris(dibenzylideneacetone)dipalladium (Pd₂(dba)₃) (0.94g, 1.03mmol), XPhos (0.98g, 2.05mmol), sodium tert-butoxide (NaOtBu) (3.94g, 41.0mmol), and xylene (70mL) were placed in a 250 mL round-bottom flask, and stirred at 130 °C for 2 hours. After the reaction was completed, the temperature was cooled to room temperature, and filtration was performed using a Celite filter. It was extracted with distilled water, brine, and MC. The solution was dried over anhydrous MgSO₄ and concentrated. The concentrated reaction product was purified by a column under the condition of MC:hexane=1:3 to obtain intermediate L2(8.07g, 19.3mmol, 94%).

### 2) Preparation of compound 18

Intermediate L2 (8.07g, 19.3mmol), 3,6-diphenyl-9H-carbazole (6.79g, 21.2mmol), tris(dibenzylideneacetone)dipalladium (Pd₂(dba)₃) (0.88g, 0.97mmol), XPhos (0.92g, 1.93mmol), sodium tert-butoxide (NaOtBu) (3.56g, 38.6mmol), and toluene (80mL) were placed in a 250 mL round-bottom flask, and stirred at 120 °C for 3 hours. After the reaction was completed, the temperature was cooled to room temperature, and filtration was performed using a Celite filter. It was extracted with distilled water, brine, and MC. The solution was dried over anhydrous MgSO₄ and concentrated. The concentrated reaction product was purified by a column under the condition of MC:hexane=1:3 to obtain compound 18(12.3g, 17.6mmol, 91%) .

### <Preparation Example 14> Preparation of compound 22

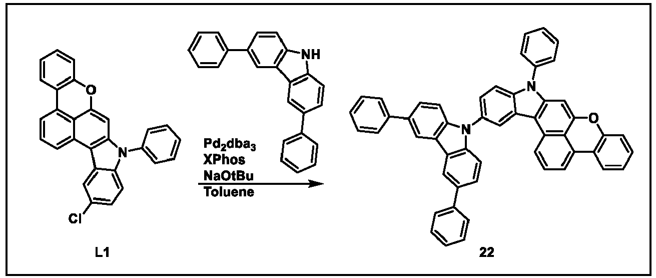

Intermediate L1 (7.0g, 16.8mmol), 3,6-diphenyl-9H-carbazole (5.89g, 18.4mmol), tris(dibenzylideneacetone)dipalladium (Pd₂(dba)₃) (0.77g, 0.84mmol), XPhos (0.80g, 1.68mmol), sodium tert-butoxide (NaOtBu) (3.23g, 33.6mmol), and toluene(70mL) were placed in a 250 mL round-bottom flask, and stirred at 120 °C for 4 hours. After the reaction was completed, the temperature was cooled to room temperature, and filtration was performed using a Celite filter. It was extracted with distilled water, brine, and MC. The solution was dried over anhydrous MgSO₄ and concentrated. The concentrated reaction product was purified by a column under the condition of MC:hexane=1:3 to obtain compound 22 (10.5g, 15.0mmol, 89%).

### <Preparation Example 15> Preparation of compound 27

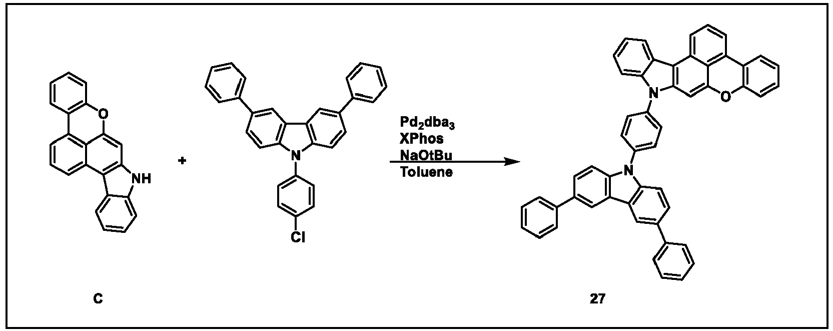

Intermediate C (7.0g, 22.8mmol), 3,6-diphenyl-9H-carbazole (10.8g, 25.1mmol), tris(dibenzylideneacetone)dipalladium (Pd₂(dba)₃) (1.04g, 1.14mmol), XPhos (1.09g, 2.28mmol), sodium tert-butoxide (NaOtBu) (4.38g, 45.6mmol), and toluene (70mL) were placed in a 250 mL round-bottom flask, and stirred at 120 °C for 4 hours. After the reaction was completed, the temperature was cooled to room temperature, and filtration was performed using a Celite filter. It was extracted with distilled water, brine, and MC. The solution was dried over anhydrous MgSO₄ and concentrated. The concentrated reaction product was purified by a column under the condition of MC:hexane=1:3 to obtain compound 27(13.6g, 19.4mmol, 85%) .

Compounds 177, 257, and 337 were prepared in the same manner as in Preparation Example 15, except that in Preparation Example 15, intermediates of Table 2 below were used instead of intermediate C.

**Table 2:**

| Intermediate | Target compound (P) | Yield |
|---|---|---|
| 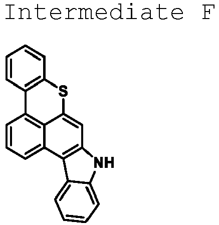 | 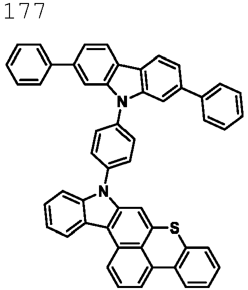 | 84% |
| 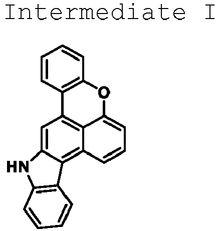 | 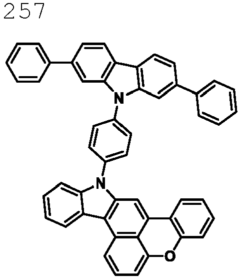 | 89% |
| 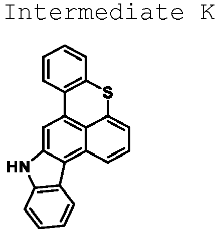 | 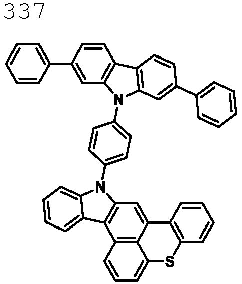 | 88% |

The compounds described herein were prepared in the same manner as in the above Preparation Examples, and the results of the synthesis of the prepared compounds are shown in Tables 3 and 4 below. Table 3 below is the measurement value of ¹H NMR (CDCl₃, 400Mz), and Table 4 below is the measurement value of the FD-mass spectrometer (FD-MS: Field desorption mass spectrometry).

**Table 3:**

| **Compound** | **¹H NMR (CDCl₃, 400Mz)** |
|---|---|
| 1 | δ = 9.02~9.00 (d, 1H), 8.83~8.80 (d, 2H), 8.56~8.54 (d, 1H), 8.35~8.33 (d, 1H), 8.19~8.10 (m, 5H), 7.98~7.96 (d, 2H), 7.51~7.33 (m, 11H), 7.31~7.29 (t, 1H), 7.17~7.15 (m, 3H), 6.98 (s, 1H) |
| 3 | δ = 9.07~9.05 (d, 1H), 8.86~8.84 (d, 2H), 8.58~8.56 (d, 1H), 8.31~8.29 (d, 1H), 8.18~8.09 (m, 5H), 8.00~7.97 (d, 2H), 7.56~7.34 (m, 12H), 7.21~7.16 (m, 3H), 6.96 (s, 1H) |
| 11 | δ = 9.11~9.09 (d, 1H), 8.87~8.83 (m, 3H), 8.08~7.99 (m, 5H), 7. 96-7. 93 (m, 3H), 7.61~7.52 (m, 10H), 7.36~7.31 (m, 3H), 7.16~7.13 (t, 2H), 6.87 (s, 1H) |
| 18 | δ = 9.09~9.07 (d, 1H), 8.81~8.78 (m, 2H), 8.42~8.40 (d, 1H), 8.28~8.26 (m, 2H), 8.05~7.97 (m, 4H), 7.93~7.83 (m, 7H), 7.66~7.51 (m, 13H), 7.24~7.22 (t, 1H), 6.89 (s, 1H) |
| 22 | δ = 9.03~9.01 (d, 1H), 8.88~8.86 (d, 1H), 8.82~8.80 (d, 1H), 8.43∼8.41 (d, 1H), 8.37~8.35 (d, 1H), 8.30~8.28 (d, 1H), 8.17~8.15 (m, 2H), 8.01~7.59 (m, 21H), 7.20~7.18 (m, 2H), 6.89 (s, 1H) |
| 27 | δ = 8.99~8.97 (d, 1H), 8.89~8.87 (d, 1H), 8.85~8.82 (m, 2H), 8.48~8.46 (d, 1H), 8.35~8.33 (d, 1H), 8.10~7.98 (m, 7H), 7.85~7.72 (m, 7H), 7.43~7.29 (m, 8H), 7.22~7.19 (m, 3H), 6.85 (s, 1H) |
| 35 | δ = 9.05~9.03 (d, 1H), 8.81~8.79 (d, 2H), 8.53~8.51 (d, 1H), 8.32~8.29 (d, 1H), 8.13~7.98 (m, 8H), 7.96~7.89 (m, 4H), 7.55~7.38 (m, 11H), 7.21~7.16 (m, 3H), 6.99 (s, 1H) |
| 42 | δ = 9.06∼9.04 (d, 1H), 8.84~8.80 (d, 2H), 8.43~8.41 (d, 1H), 8.37 (s, 1H), 8.33~8.31 (d, 1H), 8.16~8.09 (m, 8H), 7.90~7.63 (m, 14H), 7.20~7.15 (m, 3H), 6.97 (s, 1H) |
| 68 | δ = 9.10~9.08 (d, 1H), 8.75~8.70 (m, 3H), 8.38~8.86 (d, 1H), 8.12~7.92 (m, 11H), 7.81~7.68 (m, 7H), 7.54~7.46 (m, 4H), 7.15~7.13 (t, 2H), 6.86 (s, 1H) |
| 101 | δ = 9.11~9.09 (d, 1H), 8.88~8.86 (d, 2H), 8.76~8.68 (m, 5H), 8.46~8.44 (d, 1H), 8.34~8.25 (m, 5H), 8.10~8.07 (m, 2H), 7.85~7.60 (m, 13H), 7.35~7.30 (m, 3H), 7.05 (s, 1H) |
| 113 | δ = 9.13~9.11 (d, 1H), 8.85~8.83 (d, 2H), 8.74~8.72 (d, 1H), 8.57~8.55 (d, 2H), 8.31~8.08 (m, 11H), 8.01~7.89 (m, 11H), 7.35~7.30 (m, 3H), 7.03 (s, 1H) |
| 117 | δ = 9.13~9.11 (d, 1H), 8.87~8.84 (d, 2H), 8.75~8.73 (d, 1H), 8.55~8.53 (d, 2H), 8.28~8.03 (m, 11H), 7.98~7.76 (m, 11H), 7.38~7.33 (m, 3H), 7.04 (s, 1H) |
| 161 | δ = 9.01~8.98 (d, 1H), 8.79~8.77 (d, 2H), 8.53~8.51 (d, 1H), 8.37~8.35 (d, 1H), 8.17~8.08 (m, 5H), 7.95~7.93 (d, 2H), 7.55~7.35 (m, 11H), 7.29~7.27 (t, 1H), 7.15~7.08 (m, 4H) |
| 168 | δ = 9.08~9.06 (d, 1H), 8.89~8.85 (m, 3H), 8.03~7.96 (m, 4H), 7. 90-7. 85 (m, 3H), 7.63~7.53 (m, 10H), 7.35~7.31 (m, 2H), 7.28~7.23 (m, 3H), 7.16~7.14 (t, 2H) |
| 177 | δ = 8.98~8.96 (d, 1H), 8.90~8.88 (d, 1H), 8.83~8.81 (m, 2H), 8.46~8.44 (d, 1H), 8.38~8.36 (d, 1H), 8.11~7.96 (m, 6H), 7.80~7.68 (m, 7H), 7.42~7.16 (m, 13H) |
| 211 | δ = 9.10~9.08 (d, 1H), 8.85~8.82 (d, 2H), 8.75~8.67 (m, 5H), 8.44~8.42 (d, 1H), 8.33~8.25 (m, 4H), 8.10~8.08 (m, 2H), 7.84~7.62 (m, 13H), 7. 42-7. 33 (m, 5H) |
| 217 | δ = 9.12~9.10 (d, 1H), 8.82~8.80 (d, 2H), 8.74~8.66 (m, 5H), 8.41~8.39 (d, 1H), 8.33~8.25 (m, 4H), 8.08~8.06 (m, 2H), 7.85~7.60 (m, 13H), 7.40~7.31 (m, 5H) |
| 219 | δ = 9.13~9.11 (d, 1H), 8.88~8.85 (d, 2H), 8.74~8.72 (d, 1H), 8.55~8.53 (d, 2H), 8.26~8.04 (m, 11H), 7.95~7.74 (m, 11H), 7.35~7.28 (m, 4H) |
| 223 | δ = 9.10~9.08 (d, 1H), 8.84~8.82 (d, 2H), 8.75~8.73 (d, 1H), 8.56~8.53 (d, 2H), 8.29~8.08 (m, 11H), 7.91~7.69 (m, 11H), 7.31~7.24 (m, 4H) |
| 241 | δ = 9.32~9.30 (d, 1H), 9.08~9.06 (d, 1H), 8.85~8.83 (d, 1H), 8.64~8.62 (d, 1H), 8.34~8.25 (m, 5H), 8.17~8.15 (d, 1H), 8.02~7.76 (m, 12H), 7.50~7.46 (m, 2H), 7.35~7.29 (m, 3H), 6.64 (s, 1H) |
| 248 | δ = 9.29~9.27 (d, 1H), 9.02~9.00 (d, 2H), 8.63~8.54 (m, 5H), 8.34~8.25 (m, 5H), 8.15~8.13 (d, 2H), 8.01~7.75 (m, 11H), 7.52~7.46 (m, 4H), 7.34~7.31 (m, 2H), 6.60 (s, 1H) |
| 257 | δ = 9.27~9.25 (d, 1H), 9.07~9.05 (d, 1H), 8.89~8.87 (d, 1H), 8.84~8.82 (d, 1H), 8.44~8.35 (m, 7H), 8.27~8.18 (m, 6H), 7.94~7.73 (m, 11H), 7.42~7.36 (m, 3H), 6.59 (s, 1H) |
| 291 | δ = 9.33~9.31 (d, 1H), 9.14~9.12 (d, 1H), 8.85~8.76 (m, 5H), 8.49~8.47 (d, 1H), 8.24~8.15 (m, 5H), 8.11~8.09 (d, 1H), 8.05~8.80 (m, 13H), 7.72~7.70 (m, 2H), 7.43~7.37 (m, 3H), 6.69 (s, 1H) |
| 297 | δ = 9.31~9.29 (d, 1H), 9.13~9.11 (d, 1H), 8.90~8.81 (m, 5H), 8.52~8.50 (d, 1H), 8.23~8.14 (m, 5H), 8.09∼8.07 (d, 1H), 8.00~8.74 (m, 13H), 7.69~7.63 (m, 2H), 7.35~7.30 (m, 3H), 6.65 (s, 1H) |
| 299 | δ = 9.30~9.28 (d, 1H), 9.15~9.14 (d, 1H), 8.92~8.83 (m, 5H), 8.55~8.53 (d, 1H), 8.20~8.12 (m, 5H), 8.01~7.76 (m, 13H), 7.41~7.37 (m, 2H), 7.30~7.26 (m, 3H), 6.68 (s, 1H) |
| 303 | δ = 9.29~9.27 (d, 1H), 9.12~9.10 (d, 1H), 8.91~8.83 (m, 5H), 8.52~8.50 (d, 1H), 8.25~8.17 (m, 5H), 8.08~7.84 (m, 13H), 7.38~7.34 (m, 2H), 7.28~7.23 (m, 3H), 6.70 (s, 1H) |
| 321 | δ = 9.33~9.31 (d, 1H), 9.12~9.10 (d, 1H), 8.88~8.86 (d, 1H), 8.60~8.58 (d, 2H), 8.38~8.29 (m, 5H), 8.12~8.10 (d, 2H), 7.98~7.68 (m, 16H) |
| 337 | δ = 9.28~9.26 (d, 1H), 9.09~9.07 (d, 1H), 8.85~8.83 (d, 1H), 8.80~8.78 (d, 1H), 8.40~8.27 (m, 7H), 8.20~8.08 (m, 7H), 7.86~7.59 (m, 14H) |
| 371 | δ = 9.31~9.29 (d, 1H), 9.13~9.11 (d, 1H), 8.84~8.75 (m, 5H), 8.45~8.43 (d, 1H), 8.22~8.13 (m, 5H), 8.01~7.99 (d, 1H), 7.82~8.57 (m, 13H) |
| 377 | δ = 9.30~9.28 (d, 1H), 9.15~9.13 (d, 1H), 8.83~8.74 (m, 5H), 8.47~8.45 (d, 1H), 8.29~8.20 (m, 5H), 8.12~8.10 (d, 1H), 7.86~7.60 (m, 13H) |
| 379 | δ = 9.33~9.31 (d, 1H), 9.16~9.14 (d, 1H), 8.91~8.82 (m, 5H), 8.54~8.53 (d, 1H), 8.22~8.13 (m, 5H), 8.03~7.78 (m, 13H), 7.40~7.29 (m, 6H) |
| 383 | δ = 9.32~9.30 (d, 1H), 9.14~9.12 (d, 1H), 8.90~8.81 (m, 5H), 8.56~8.54 (d, 1H), 8.25~8.16 (m, 5H), 8.07~7.81 (m, 13H), 7.43~7.31 (m, 6H) |
| 401 | δ = 9.03~9.01 (d, 1H), 8.84~8.81 (d, 2H), 8.57~8.55 (d, 1H), 8.34~8.32 (d, 1H), 8.18~8.09 (m, 5H), 7.53~7.39 (m, 8H), 7.32~7.29 (t, 1H), 7.18~7.14 (m, 3H), 6.97 (s, 1H) |

**Table 4:**

| **Comp ound** | **FD-Mass** | **Comp ound** | **FD-Mass** |
|---|---|---|---|
| 1 | m/z= 624.2281(C46H28N2O, 624.2202) | 2 | m/z= 624.2194(C46H28N2O, 624.2202) |
| 3 | m/z= 624.2286(C46H28N2O, 624.2202) | 4 | m/z= 624.2189(C46H28N2O, 624.2202) |
| 5 | m/z= 624.2245(C46H28N2O, 624.2202) | 6 | m/z= 624.2275(C46H28N2O, 624.2202) |
| 7 | m/z= 624.2215(C46H28N2O, 624.2202) | 8 | m/z= 624.2202 (C46H28N2O, 624.2202) |
| 9 | m/z= 624.2185(C46H28N2O, 624.2202) | 10 | m/z= 624.2199(C46H28N2O, 624.2202) |
| 11 | m/z= 624.2225(C46H28N2O, 624.2202) | 12 | m/z= 624.2249(C46H28N2O, 624.2202) |
| 13 | m/z= 624.2251(C46H28N2O, 624.2202) | 14 | m/z= 700.2518(C52H32N2O, 700.2515) |
| 15 | m/z= 700.2508(C52H32N2O, 700.2515) | 16 | m/z= 700.2531(C52H32N2O, 700.2515) |
| 17 | m/z= 624.2085(C46H28N2O, 624.2202) | 18 | m/z= 700.2552(C52H32N2O, 700.2515) |
| 19 | m/z= 700.2538(C52H32N2O, 700.2515) | 20 | m/z= 700.2508(C52H32N2O, 700.2515) |
| 21 | m/z= 624.2209(C46H28N2O, 624.2202) | 22 | m/z= 700.2497(C52H32N2O, 700.2515) |
| 23 | m/z= 700.2584(C52H32N2O, 700.2515) | 24 | m/z= 700.2528(C52H32N2O, 700.2515) |
| 25 | m/z= 624.2295(C46H28N2O, 624.2202) | 26 | m/z= 700.2521(C52H32N2O, 700.2515) |
| 27 | m/z=700.2521(C52H32N2O, 700.2515) | 28 | m/z=700.2519(C52H32N2O, 700.2515) |
| 29 | m/z=625.2148 (C45H27N3O, 625.2154) | 30 | m/z=625.2161(C45H27N3O, 625.2154) |
| 31 | m/z=674.2318 (C5OH3ON20, 674.2358) | 32 | m/z=674.2363(C50H30N2O, 674.2358) |
| 33 | m/z=674.2327 (C5OH3ON20, 674.2358) | 34 | m/z=674.2360(C50H30N2O, 674.2358) |
| 35 | m/z=700.2531(C52H32N2O, 700.2515) | 36 | m/z=700.2532 (C52H32N2O, 700.2515) |
| 37 | m/z=674.2309(C50H30N2O, 674.2358) | 38 | m/z=674.2364 (C5OH3ON20, 674.2358) |
| 39 | m/z=674.2384 (C5OH3ON20, 674.2358) | 40 | m/z=674.2355 (C5OH3ON20, 674.2358) |
| 41 | m/z=700.2522 (C52H32N2O, 700.2515) | 42 | m/z=700.2538 (C52H32N2O, 700.2515) |
| 43 | m/z=674.2368 (C5OH3ON20, 674.2358) | 44 | m/z=674.2361(C50H30N2O, 674.2358) |
| 45 | m/z=674.2340(C50H30N2O, 674.2358) | 46 | m/z=674.2370(C50H30N2O, 674.2358) |
| 47 | m/z=700.2510(C52H32N2O, 700.2515) | 48 | m/z=700.2534 (C52H32N2O, 700.2515) |
| 49 | m/z=674.2381(C50H30N2O, 674.2358) | 50 | m/z=674.2361(C50H30N2O, 674.2358) |
| 51 | m/z=674.2343(C50H30N2O, 674.2358) | 52 | m/z=674.2382 (C5OH3ON20, 674.2358) |
| 53 | m/z=700.2508 (C52H32N2O, 700.2515) | 54 | m/z=700.2488 (C52H32N2O, 700.2515) |
| 55 | m/z=700.2500(C52H32N20, 700.2515) | 56 | m/z=700.2531(C52H32N2O, 700.2515) |
| 57 | m/z=674.2365 (C5OH3ON20, 674.2358) | 58 | m/z=674.2319(C50H30N2O, 674.2358) |
| 59 | m/z=674.2348 (C5OH3ON20, 674.2358) | 60 | m/z=674.2350(C50H30N2O, 674.2358) |
| 61 | m/z=700.2531(C52H32N2O, 700.2515) | 62 | m/z=700.2516(C52H32N20, 700.2515) |
| 63 | m/z=674.2344 (C5OH3ON20, 674.2358) | 64 | m/z=674.2358 (C5OH3ON20, 674.2358) |
| 65 | m/z=700.2538 (C52H32N2O, 700.2515) | 66 | m/z=700.2519(C52H32N20, 700.2515) |
| 67 | m/z=674.2368 (C5OH3ON20, 674.2358) | 68 | m/z=674.2368 (C5OH3ON20, 674.2358) |
| 69 | m/z=674.2312 (C5OH3ON20, 674.2358) | 70 | m/z=674.2354 (C5OH3ON20, 674.2358) |
| 71 | m/z=700.2492 (C52H32N2O, 700.2515) | 72 | m/z=700.2510(C52H32N2O, 700.2515) |
| 73 | m/z=674.2365 (C5OH3ON20, 674.2358) | 74 | m/z=674.2355 (C5OH3ON20, 674.2358) |
| 75 | m/z=674.2369(C50H30N2O, 674.2358) | 76 | m/z=674.2367 (C5OH3ON20, 674.2358) |
| 77 | m/z=674.2338 (C5OH3ON20, 674.2358) | 78 | m/z=674.2319(C50H30N2O, 674.2358) |
| 79 | m/z=674.2341(C50H30N2O, 674.2358) | 80 | m/z=674.2335 (C5OH3ON20, 674.2358) |
| 81 | m/z=700.2497 (C52H32N2O, 700.2515) | 82 | m/z=700.2531(C52H32N2O, 700.2515) |
| 83 | m/z=700.2531(C52H32N2O, 700.2515) | 84 | m/z=674.2328 (C5OH3ON20, 674.2358) |
| 85 | m/z=750.2670(C56H34N2O, 750.2671) | 86 | m/z=724.2322 (C54H32N2O, 724.2515) |
| 87 | m/z=776.2832 (C58H36N2O, 776.2828) | 88 | m/z=750.2654 (C56H34N2O, 750.2671) |
| 89 | m/z=776.2861(C58H36N2O, 776.2828) | 90 | m/z=750.2670(C56H34N2O, 750.2671) |
| 91 | m/z=776.2835 (C58H36N2O, 776.2828) | 92 | m/z=750.2695 (C56H34N2O, 750.2671) |
| 93 | m/z=750.2677 (C56H34N2O, 750.2671) | 94 | m/z=750.2646(C56H34N2O, 750.2671) |
| 95 | m/z=750.2673(C56H34N2O, 750.2671) | 96 | m/z=750.2692 (C56H34N2O, 750.2671) |
| 97 | m/z=724.2508 (C54H32N2O, 724.2515) | 98 | m/z=724.2531(C54H32N2O, 724.2515) |
| 99 | m/z=724.2513(C54H32N2O, 724.2515) | 100 | m/z=724.2538 (C54H32N2O, 724.2515) |
| 101 | m/z=779.2688 (C55H33N5O, 779.2685) | 102 | m/z=778.2720(C56H34N4O, 778.2733) |
| 103 | m/z=778.2730(C56H34N4O, 778.2733) | 104 | m/z=779.2702 (C55H33N5O, 779.2685) |
| 105 | m/z=778.2739(C56H34N4O, 778.2733) | 106 | m/z=778.2736(C56H34N4O, 778.2733) |
| 107 | m/z=779.2702 (C55H33N5O, 779.2685) | 108 | m/z=778.2741(C56H34N4O, 778.2733) |
| 109 | m/z=778.2736(C56H34N4O, 778.2733) | 110 | m/z=779.2781(C55H33N5O, 779.2685) |
| 111 | m/z=778.2729(C56H34N4O, 778.2733) | 112 | m/z=778.2739(C56H34N4O, 778.2733) |
| 113 | m/z=752.8801(C54H32N4O, 752.8770) | 114 | m/z=752.8787 (C54H32N4O, 752.8770) |
| 115 | m/z=751.2654 (C55H33N3O, 751.2624) | 116 | m/z=751.2641(C55H33N3O, 751.2624) |
| 117 | m/z=752.8787 (C54H32N4O, 752.8770) | 118 | m/z=751.2632 (C55H33N3O, 751.2624) |
| 119 | m/z=779.2702 (C55H33N5O, 779.2685) | 120 | m/z=778.2716(C56H34N4O, 778.2733) |
| 121 | m/z=778.2751(C56H34N4O, 778.2733) | 122 | m/z=77 9.2680(C55H33N5O, 779.2685) |
| 123 | m/z=778.2730(C56H34N4O, 778.2733) | 124 | m/z=778.2761(C56H34N4O, 778.2733) |
| 125 | m/z=779.2702 (C55H33N5O, 779.2685) | 126 | m/z=778.2730(C56H34N4O, 778.2733) |
| 127 | m/z=778.2735 (C56H34N4O, 778.2733) | 128 | m/z=779.2695 (C55H33N5O, 779.2685) |
| 129 | m/z=778.2761(C56H34N4O, 778.2733) | 130 | m/z=778.2741(C56H34N4O, 778.2733) |
| 131 | m/z=779.2692 (C55H33N5O, 779.2685) | 132 | m/z=778.2739(C56H34N4O, 778.2733) |
| 133 | m/z=778.2738 (C56H34N4O, 778.2733) | 134 | m/z=779.2644 (C55H33N5O, 779.2685) |
| 135 | m/z=778.2741(C56H34N4O, 778.2733) | 136 | m/z=778.2752 (C56H34N4O, 778.2733) |
| 137 | m/z=779.2716(C55H33N5O, 779.2685) | 138 | m/z=778.2745 (C56H34N4O, 778.2733) |
| 139 | m/z=778.2736(C56H34N4O, 778.2733) | 140 | m/z=779.2691(C55H33N5O, 779.2685) |
| 141 | m/z=778.2730(C56H34N4O, 778.2733) | 142 | m/z=778.2748 (C56H34N4O, 778.2733) |
| 143 | m/z=77 9.27 00(C55H33N5O, 779.2685) | 144 | m/z=778.2731(C56H34N4O, 778.2733) |
| 145 | m/z=778.7217 (C56H34N4O, 778.2733) | 146 | m/z=726.2410(C52H30N4O, 726.2420) |
| 147 | m/z=726.2428 (C52H30N4O, 726.2420) | 148 | m/z=726.2447 (C52H30N4O, 726.2420) |
| 149 | m/z=726.2439(C52H30N4O, 726.2420) | 150 | m/z=726.2411(C52H30N4O, 726.2420) |
| 151 | m/z=726.2435 (C52H30N4O, 726.2420) | 152 | m/z=726.2408 (C52H30N4O, 726.2420) |
| 153 | m/z=726.2415 (C52H30N4O, 726.2420) | 154 | m/z=792.2497 (C56H32N4O2, 792.2525) |
| 155 | m/z=792.2519(C56H32N4O2, 792.2525) | 156 | m/z=792.2531(C56H32N4O2, 792.2525) |
| 157 | m/z=792.2518 (C56H32N4O2, 792.2525) | 158 | m/z=808.2315 (C56H32N4OS, 808.2297) |
| 159 | m/z=808.2308 (C56H32N4OS, 808.2297) | 160 | m/z=808.2281(C56H32N4OS, 808.2297) |
| 161 | m/z=640.1994(C46H28N2S, 640.1973) | 162 | m/z=640.1955(C46H28N2S, 640.1973) |
| 163 | m/z=640.1970(C46H28N2S, 640.1973) | 164 | m/z=640.1964(C46H28N2S, 640.1973) |
| 165 | m/z=640.1982(C46H28N2S, 640.1973) | 166 | m/z=640.1998(C46H28N2S, 640.1973) |
| 167 | m/z=640.1958(C46H28N2S, 640.1973) | 168 | m/z=640.1981(C46H28N2S, 640.1973) |
| 169 | m/z=716.2251(C52H32N2S, 716.2286) | 170 | m/z=716.2279(C52H32N2S, 716.2286) |
| 171 | m/z=7l6.2295 (C52H32N2S, 716.2286) | 172 | m/z=716.2272 (C52H32N2S, 716.2286) |
| 173 | m/z=716.2290(C52H32N2S, 716.2286) | 174 | m/z=716.2277 (C52H32N2S, 716.2286) |
| 175 | m/z=716.2312 (C52H32N2S, 716.2286) | 176 | m/z=716.2291(C52H32N2S, 716.2286) |
| 177 | m/z=716.2293(C52H32N2S, 716.2286) | 178 | m/z=641.1934 (C45H27N3S, 641.1926) |
| 179 | m/z=690.2145 (C50H30N2S, 690.2130) | 180 | m/z=690.2144 (C50H30N2S, 690.2130) |
| 181 | m/z=716.2291(C52H32N2S, 716.2286) | 182 | m/z=690.2115 (C50H30N2S, 690.2130) |
| 183 | m/z=690.2115 (C50H30N2S, 690.2130) | 184 | m/z=716.2290(C52H32N2S, 716.2286) |
| 185 | m/z=716.2294 (C52H32N2S, 716.2286) | 186 | m/z=690.2138 (C50H30N2S, 690.2130) |
| 187 | m/z=690.2142 (C50H30N2S, 690.2130) | 188 | m/z=716.2291(C52H32N2S, 716.2286) |
| 189 | m/z=690.2121(C50H30N2S, 690.2130) | 190 | m/z=716.2270(C52H32N2S, 716.2286) |
| 191 | m/z=690.2128 (C50H30N2S, 690.2130) | 192 | m/z=690.2133(C50H30N2S, 690.2130) |
| 193 | m/z=690.2146(C50H30N2S, 690.2130) | 194 | m/z=690.2145 (C50H30N2S, 690.2130) |
| 195 | m/z=690.2145 (C50H30N2S, 690.2130) | 196 | m/z=690.2155 (C50H30N2S, 690.2130) |
| 197 | m/z=716.2292 (C52H32N2S, 716.2286) | 198 | m/z=716.2291(C52H32N2S, 716.2286) |
| 199 | m/z=716.2275 (C52H32N2S, 716.2286) | 200 | m/z=690.2149(C50H30N2S, 690.2130) |
| 201 | m/z=766.2438 (C56H34N2S, 766.2443) | 202 | m/z=740.2298 (C54H32N2S, 740.2286) |
| 203 | m/z=792.2618 (C58H36N2S, 792.2599) | 204 | m/z=792.2618 (C58H36N2S, 792.2599) |
| 205 | m/z=766.2457 (C56H34N2S, 766.2443) | 206 | m/z=792.2620(C58H36N2S, 792.2599) |
| 207 | m/z=766.2450(C56H34N2S, 766.2443) | 208 | m/z=766.2430(C56H34N2S, 766.2443) |
| 209 | m/z=766.2431(C56H34N2S, 766.2443) | 210 | m/z=740.2296(C54H32N2S, 740.2286) |
| 211 | m/z=765.2466(C55H33N5S, 795.2457) | 212 | m/z=794.2486(C56H34N4S, 794.2504) |
| 213 | m/z=795.2465 (C55H33N5S, 795.2457) | 214 | m/z=794.2501(C56H34N4S, 794.2504) |
| 215 | m/z=795.2462 (C55H33N5S, 795.2457) | 216 | m/z=794.2483(C56H34N4S, 794.2504) |
| 217 | m/z=795.2450(C55H33N5S, 795.2457) | 218 | m/z=794.2591(C56H34N4S, 794.2504) |
| 219 | m/z=768.2329(C54H32N4S, 768.2348) | 220 | m/z=768.2356(C54H32N4S, 768.2348) |
| 221 | m/z=767.2418 (C55H33N3S, 767.2395) | 222 | m/z=767.2399(C55H33N3S, 767.2395) |
| 223 | m/z=768.2351(C54H32N4S, 768.2348) | 224 | m/z=767.2414 (C55H33N3S, 767.2395) |
| 225 | m/z=795.2455 (C55H33N5S, 795.2457) | 226 | m/z=795.2462 (C55H33N5S, 795.2457) |
| 227 | m/z=794.2512 (C56H34N4S, 794.2504) | 228 | m/z=795.2480(C55H33N5S, 795.2457) |
| 229 | m/z=794.2503(C56H34N4S, 794.2504) | 230 | m/z=795.2461(C55H33N5S, 795.2457) |
| 231 | m/z=795.2483(C55H33N5S, 795.2457) | 232 | m/z=795.2429(C55H33N5S, 795.2457) |
| 233 | m/z=795.2450(C55H33N5S, 795.2457) | 234 | m/z=795.2466(C55H33N5S, 795.2457) |
| 235 | m/z=795.2461(C55H33N5S, 795.2457) | 236 | m/z=742.2232 (C52H30N4S, 742.2191) |
| 237 | m/z=742.2218 (C52H30N4S, 742.2191) | 238 | m/z=808.2318 (C56H32N4OS, 808.2297) |
| 239 | m/z=808.2314 (C56H32N4OS, 808.2297) | 240 | m/z=824.2100(C56H32N4S2, 824.2068) |
| 241 | m/z=624.2281(C46H28N2O, 624.2202) | 242 | m/z=624.2192 (C46H28N2O, 624.2202) |
| 243 | m/z=624.2206(C46H28N2O, 624.2202) | 244 | m/z=624.2184 (C46H28N2O, 624.2202) |
| 245 | m/z=624.2211(C46H28N2O, 624.2202) | 246 | m/z=624.2191(C46H28N2O, 624.2202) |
| 247 | m/z=624.2216(C46H28N2O, 624.2202) | 248 | m/z=624.2240(C46H28N2O, 624.2202) |
| 249 | m/z=700.2519(C52H32N2O, 700.2515) | 250 | m/z=700.2511(C52H32N2O, 700.2515) |
| 251 | m/z=700.2509(C52H32N2O, 700.2515) | 252 | m/z=700.2532 (C52H32N2O, 700.2515) |
| 253 | m/z=700.2531(C52H32N2O, 700.2515) | 254 | m/z=700.2508(C52H32N2O, 700.2515) |
| 255 | m/z=700.2492 (C52H32N2O, 700.2515) | 256 | m/z=700.2522 (C52H32N2O, 700.2515) |
| 257 | m/z=700.2514 (C52H32N2O, 700.2515) | 258 | m/z=625.2162 (C45H27N3O, 625.2154) |
| 259 | m/z=674.2350(C50H30N2O, 674.2358) | 260 | m/z=674.2350(C50H30N2O, 674.2358) |
| 261 | m/z=700.2519(C52H32N2O, 700.2515) | 262 | m/z=674.2366(C50H30N2O, 674.2358) |
| 263 | m/z=674.2366(C50H30N2O, 674.2358) | 264 | m/z=700.2519(C52H32N2O, 700.2515) |
| 265 | m/z=700.2548 (C52H32N2O, 700.2515) | 266 | m/z=674.2348 (C5OH3ON20, 674.2358) |
| 267 | m/z=674.2364 (C5OH3ON20, 674.2358) | 268 | m/z=700.2523(C52H32N2O, 700.2515) |
| 269 | m/z=674.2339(C50H30N2O, 674.2358) | 270 | m/z=700.2531(C52H32N2O, 700.2515) |
| 271 | m/z=674.2361(C50H30N2O, 674.2358) | 272 | m/z=674.2364 (C5OH3ON20, 674.2358) |
| 273 | m/z=674.2370(C50H30N2O, 674.2358) | 274 | m/z=674.2332 (C5OH3ON20, 674.2358) |
| 275 | m/z=674.2329(C50H30N2O, 674.2358) | 276 | m/z=674.2343(C50H30N2O, 674.2358) |
| 277 | m/z=700.2511(C52H32N2O, 700.2515) | 278 | m/z=700.2511(C52H32N2O, 700.2515) |
| 279 | m/z=700.2530(C52H32N2O, 700.2515) | 280 | m/z=674.2370(C50H30N2O, 674.2358) |
| 281 | m/z=750.2709(C56H34N2O, 750.2671) | 282 | m/z=724.2519(C54H32N2O, 724.2515) |
| 283 | m/z=766.2838 (C58H36N2O, 776.2828) | 284 | m/z=776.2839(C58H36N2O, 776.2828) |
| 285 | m/z=750.2682 (C56H34N2O, 750.2671) | 286 | m/z=776.2798 (C58H36N2O, 776.2828) |
| 287 | m/z=750.2683(C56H34N2O, 750.2671) | 288 | m/z=750.2682 (C56H34N2O, 750.2671) |
| 289 | m/z=750.2688 (C56H34N2O, 750.2671) | 290 | m/z=724.2533(C54H32N2O, 724.2515) |
| 291 | m/z=779.2659(C55H33N5O, 779.2685) | 292 | m/z=778.2741(C56H34N4O, 778.2733) |
| 293 | m/z=779.2700(C55H33N5O, 779.2685) | 294 | m/z=778.2717 (C56H34N4O, 778.2733) |
| 295 | m/z=779.2691(C55H33N5O, 779.2685) | 296 | m/z=778.2749(C56H34N4O, 778.2733) |
| 297 | m/z=779.2692 (C55H33N5O, 779.2685) | 298 | m/z=778.2728 (C56H34N4O, 778.2733) |
| 299 | m/z=752.8805(C54H32N4O, 752.8770) | 300 | m/z=752.8818 (C54H32N4O, 752.8770) |
| 301 | m/z=751.2638 (C55H33N3O, 751.2624) | 302 | m/z=751.2607 (C55H33N3O, 751.2624) |
| 303 | m/z=752.8787 (C54H32N4O, 752.8770) | 304 | m/z=751.2620(C55H33N3O, 751.2624) |
| 305 | m/z=779.2693(C55H33N5O, 779.2685) | 306 | m/z=779.2691(C55H33N5O, 779.2685) |
| 307 | m/z=778.2745 (C56H34N4O, 778.2733) | 308 | m/z=779.2695 (C55H33N5O, 779.2685) |
| 309 | m/z=778.2736(C56H34N4O, 778.2733) | 310 | m/z=779.2712 (C55H33N5O, 779.2685) |
| 311 | m/z=779.2691(C55H33N5O, 779.2685) | 312 | m/z=779.2721(C55H33N5O, 779.2685) |
| 313 | m/z=779.2693(C55H33N5O, 779.2685) | 314 | m/z=77 9.2680(C55H33N5O, 779.2685) |
| 315 | m/z=779.2677 (C55H33N5O, 779.2685) | 316 | m/z=726.2433(C52H30N4O, 726.2420) |
| 317 | m/z=726.2448 (C52H30N4O, 726.2420) | 318 | m/z=792.2538 (C56H32N4O2, 792.2525) |
| 319 | m/z=792.2518 (C56H32N4O2, 792.2525) | 320 | m/z=808.2259(C56H32N4OS, 808.2297) |
| 321 | m/z=690.2145 (C50H30N2S, 690.2130) | 322 | m/z=690.2144 (C50H30N2S, 690.2130) |
| 323 | m/z=690.2112 (C50H30N2S, 690.2130) | 324 | m/z=690.2127 (C50H30N2S, 690.2130) |
| 325 | m/z=690.2144 (C50H30N2S, 690.2130) | 326 | m/z=690.2154 (C50H30N2S, 690.2130) |
| 327 | m/z=690.2150(C50H30N2S, 690.2130) | 328 | m/z=690.2163(C50H30N2S, 690.2130) |
| 329 | m/z=716.2294 (C52H32N2S, 716.2286) | 330 | m/z=716.2270(C52H32N2S, 716.2286) |
| 331 | m/z=716.2298 (C52H32N2S, 716.2286) | 332 | m/z=716.2259(C52H32N2S, 716.2286) |
| 333 | m/z=716.2313(C52H32N2S, 716.2286) | 334 | m/z=716.2291(C52H32N2S, 716.2286) |
| 335 | m/z=716.2290(C52H32N2S, 716.2286) | 336 | m/z=716.2299(C52H32N2S, 716.2286) |
| 337 | m/z=716.2270(C52H32N2S, 716.2286) | 338 | m/z=641.1923(C45H27N3S, 641.1926) |
| 339 | m/z=690.2145 (C50H30N2S, 690.2130) | 340 | m/z=690.2148 (C50H30N2S, 690.2130) |
| 341 | m/z=716.2292 (C52H32N2S, 716.2286) | 342 | m/z=690.2143(C50H30N2S, 690.2130) |
| 343 | m/z=690.2151(C50H30N2S, 690.2130) | 344 | m/z=716.2291(C52H32N2S, 716.2286) |
| 345 | m/z=716.2290(C52H32N2S, 716.2286) | 346 | m/z=690.2147 (C50H30N2S, 690.2130) |
| 347 | m/z=690.2156(C50H30N2S, 690.2130) | 348 | m/z=716.2291(C52H32N2S, 716.2286) |
| 349 | m/z=690.2111(C50H30N2S, 690.2130) | 350 | m/z=716.2282 (C52H32N2S, 716.2286) |
| 351 | m/z=690.2133(C50H30N2S, 690.2130) | 352 | m/z=690.2145 (C50H30N2S, 690.2130) |
| 353 | m/z=690.2108 (C50H30N2S, 690.2130) | 354 | m/z=690.2138 (C50H30N2S, 690.2130) |
| 355 | m/z=690.2147 (C50H30N2S, 690.2130) | 356 | m/z=690.2150(C50H30N2S, 690.2130) |
| 357 | m/z=716.2293(C52H32N2S, 716.2286) | 358 | m/z=716.2612 (C52H32N2S, 716.2286) |
| 359 | m/z=716.2288 (C52H32N2S, 716.2286) | 360 | m/z=690.2118 (C50H30N2S, 690.2130) |
| 361 | m/z=766.2431(C56H34N2S, 766.2443) | 362 | m/z=740.2293(C54H32N2S, 740.2286) |
| 363 | m/z=792.2621(C58H36N2S, 792.2599) | 364 | m/z=792.2602 (C58H36N2S, 792.2599) |
| 365 | m/z=766.2440(C56H34N2S, 766.2443) | 366 | m/z=792.2573(C58H36N2S, 792.2599) |
| 367 | m/z=766.2453(C56H34N2S, 766.2443) | 368 | m/z=766.2430(C56H34N2S, 766.2443) |
| 369 | m/z=766.2462 (C56H34N2S, 766.2443) | 370 | m/z=740.2292 (C54H32N2S, 740.2286) |
| 371 | m/z=795.2451(C55H33N5S, 795.2457) | 372 | m/z=794.2524 (C56H34N4S, 794.2504) |
| 373 | m/z=795.2468 (C55H33N5S, 795.2457) | 374 | m/z=794.2538 (C56H34N4S, 794.2504) |
| 375 | m/z=795.2482 (C55H33N5S, 795.2457) | 376 | m/z=794.2489(C56H34N4S, 794.2504) |
| 377 | m/z=795.2475 (C55H33N5S, 795.2457) | 378 | m/z=794.2483(C56H34N4S, 794.2504) |
| 379 | m/z=768.2344 (C54H32N4S, 768.2348) | 380 | m/z=768.2368 (C54H32N4S, 768.2348) |
| 381 | m/z=767.2418 (C55H33N3S, 767.2395) | 382 | m/z=767.2424 (C55H33N3S, 767.2395) |
| 383 | m/z=768.2371(C54H32N4S, 768.2348) | 384 | m/z=767.2431(C55H33N3S, 767.2395) |
| 385 | m/z=795.2462 (C55H33N5S, 795.2457) | 386 | m/z=795.2462 (C55H33N5S, 795.2457) |
| 387 | m/z=794.2485 (C56H34N4S, 794.2504) | 388 | m/z=795.2466(C55H33N5S, 795.2457) |
| 389 | m/z=794.2498 (C56H34N4S, 794.2504) | 390 | m/z=795.2468 (C55H33N5S, 795.2457) |
| 391 | m/z=795.2460(C55H33N5S, 795.2457) | 392 | m/z=795.2452 (C55H33N5S, 795.2457) |
| 393 | m/z=795.2469(C55H33N5S, 795.2457) | 394 | m/z=795.2475 (C55H33N5S, 795.2457) |
| 395 | m/z=795.2454 (C55H33N5S, 795.2457) | 396 | m/z=742.2223(C52H30N4S, 742.2191) |
| 397 | m/z=74.2188(C52H30N4S, 742.2191) | 398 | m/z=808.2317 (C56H32N4OS, 808.2297) |
| 399 | m/z=808.2325 (C56H32N4OS, 808.2297) | 400 | m/z=824.2050(C56H32N4S2, 824.2068) |
| 401 | m/z=629.2515 (C46H23D5N2O, 629.2522) | 402 | m/z=629.2515 (C46H23D5N2O, 629.2514) |
| 403 | m/z=634.2829(C46H18D10N2O, 634.2813) | 404 | m/z=629.2515 (C46H23D5N2O, 629.2531) |
| 405 | m/z=629.2515 (C46H23D5N2O, 629.2518) | 406 | m/z=634.2829(C46H18D10N2O, 634.2798) |
| 407 | m/z=645.2287 (C46H23D5N2S, 645.2305) | 408 | m/z=645.2287 (C46H23D5N2S, 645.2291) |
| 409 | m/z=650.2601 (C46H18D10N2S, 650.2612) | 410 | m/z=645. 2287 (C46H23D5N2S, 645.2295) |
| 411 | m/z=645.2287 (C46H23D5N2S, 645.2291) | 412 | m/z=650.2601 (C46H18D10N2S, 650.2618) |
| 413 | m/z=800.2771(C55H28D5N5S, 800.2786) | 414 | m/z=805.3084 (C55H23D10N5S, 805.3092) |
| 415 | m/z=810.3398 (C55H18D15N5S, 810.3418) | 416 | m/z=629.2515 (C46H23D5N2O, 629.2531) |
| 417 | m/z=629.2515 (C46H23D5N2O, 629.2531) | 418 | m/z=634.2829(C46H18D10N2O, 634.2935) |
| 419 | m/z=645.2287 (C46H23D5N2S, 645.2291) | 420 | m/z=650.2601 (C46H18D10N2S, 650.2617) |
| 421 | m/z=674.2358 (C50H30N2O, 674.2363) | 422 | m/z=674.2358 (C50H30N2O, 674.2368) |
| 423 | m/z=674.2358 (C50H30N2O, 674.2348) | 424 | m/z=724.2515 (C54H32N2O, 724.2526) |
| 425 | m/z=829.2842 (C59H35N5O, 829.2844) | 426 | m/z=829.2842 (C59H35N5O, 829.2844) |
| 427 | m/z=802.2733(C58H34N4O, 802.2730) | 428 | m/z=802.2733(C58H34N4O, 802.2741) |
| 429 | m/z=845.2613(C59H35N5S, 845.2618) | 430 | m/z=818.2504(C58H34N4S, 818.2508) |
| 431 | m/z=690.2130(C50H30N2S, 690.2137) | 432 | m/z=690.2130(C50H30N2S, 690.2145) |
| 433 | m/z=674.2358 (C50H30N2O, 674.2368) | 434 | m/z=829.2842 (C59H35N5O, 829.2830) |
| 435 | m/z=690.2130(C50H30N2S, 690.2145) | 436 | m/z=845.2613(C59H35N5S, 845.2635) |
| 437 | m/z=829.2842 (C59H35N5O, 829.2848) | 438 | m/z=829.2842 (C59H35N5O, 829.2845) |
| 439 | m/z=828.2889(C60H36N4O, 828.2905) | 440 | m/z=828.2889(C60H36N4O, 828.2915) |
| 441 | m/z=829.2842 (C59H35N5O, 829.2846) | 442 | m/z=829.2842 (C59H35N5O, 829.2844) |
| 443 | m/z=828. 2889 (C60H36N4O, 828.8907) | 444 | m/z=828. 2889 (C60H36N4O, 828.2891) |
| 445 | m/z=845.2613(C59H35N5S, 845.2611) | 446 | m/z=845.2613(C59H35N5S, 845.2642) |
| 447 | m/z=818.2504(C58H34N4S, 818.2531) | 448 | m/z=818.2504(C58H34N4S, 818.2503) |
| 449 | m/z=879.2998 (C63H37N5O, 879.3014) | 450 | m/z=879.2998 (C63H37N5O, 879.3000) |
| 451 | m/z=828.2889(C60H36N4O, 828.2951) | 452 | m/z=828.2889(C60H36N4O, 828.2905) |
| 453 | m/z=871.2770(C61H37N5S,871.27 91) | 454 | m/z=871.2770(C61H37N5S,871.276 4) |
| 455 | m/z=818.2504(C58H34N4S, 818.2543) | 456 | m/z=818.2504(C58H34N4S, 818.2497) |

### <Experimental Example 1>

### (1) Manufacturing of organic light emitting device (red host)

A glass substrate coated with a thin film of indium tin oxide (ITO) with a thickness of 1,500Å was ultrasonically washed with distilled water. After washing with distilled water, the substrate was ultrasonically washed with a solvent such as acetone, methanol, isopropyl alcohol, etc., dried, and then treated with ultraviolet ozone (UVO) for 5 minutes using ultraviolet (UV) in a UV cleaner. Thereafter, the substrate was transferred to a plasma cleaner (PT), and then plasma-treated in a vacuum to increase the work function of ITO and remove the remaining film, and transferred to a thermal deposition equipment for organic deposition.

A hole injection layer of 4,4',4"-tris[2-naphthyl(phenyl)amino]triphenylamine (2-TNATA) and a hole transport layer of N,N'-Di(1-naphthyl)-N,N'-diphenyl-(1,1'-biphenyl)-4,4'-diamine (NPB) as common layers were formed on the ITO transparent electrode (positive electrode).

On top of that, a light emitting layer was thermally deposited in the vacuum as follows. The light emitting layer was formed by pre-mixing one of the compounds of Formula 1 in Table 5 below as a P-type host and the compounds (G, H) of Formula 2 in Table 5 below as a N-type host, and doping the host with [(piq)₂(Ir)(acac)] in an amount of 3 wt.% of the deposition thickness of the light emitting layer while using [(piq)₂(Ir)(acac)] as the red phosphorescent dopant, thereby depositing with a thickness of 500 Å. Thereafter, bathocuproine (BCP) was deposited with a thickness of 60 Å as a hole blocking layer, and Alq₃ was deposited with a thickness of 200 Å as an electron transport layer on it.

Finally, on the electron transport layer, lithium fluoride (LiF) was deposited with a thickness of 10 Å to form an electron injection layer, and then aluminum (Al) was deposited with a thickness of 1,200 Å on the electron injection layer to form a negative electrode, thereby manufacturing organic light emitting devices of Examples 1 to 33.

Organic light emitting devices of Comparative Examples 1 to 12 were additionally manufactured in the same manner as in Examples, except that in the manufacturing process of the organic light emitting devices of Examples 1 to 33, compounds A to C and F below were used instead of using the compound of Formula 1 as the P-type host.

On the other hand, all organic compounds necessary for the manufacture of OLED devices were purified by vacuum sublimation under 10⁻⁸ to 10⁻⁶ torr for each material and then used in the manufacture of the organic light-emitting devices.

### (2) Operating voltage and luminous efficiency of organic light emitting device

For the organic light emitting devices of Examples 1 to 33 and Comparative Examples 1 to 12 manufactured as described above, electroluminescence (EL) characteristics were measured with M7000 from McScience Co. With the measurement results, T₉₀ of the red phosphorescent device was measured at a reference luminance of 6,000 cd/m² through the lifetime measuring device (M6000) manufactured by McScience Co.

In the above, T₉₀ means a lifetime (unit: hour (h)) that is a time at which the luminance becomes 90% compared to the initial luminance.

The results of measuring the operating voltage, luminous efficiency, color coordinate (CIE), and lifetime of the organic light emitting devices manufactured according to the present invention are shown in Table 5 below.

**Table 5:**

| | Light emitting layer Compound | Ratio | Operating voltage (V) | Efficiency (cd/A) | Color coordinate (x, y) | Lifetime (T₉₀) |
|---|---|---|---|---|---|---|
| Comparative Example 1 | A : G | 1 : 2 | 4.82 | 46.8 | (0.685, 0.315) | 85 |
| Comparative Example 2 | | 1 : 1 | 4.98 | 48.9 | (0.685, 0.315) | 72 |
| Comparative Example 3 | | 2 : 1 | 4.77 | 51.5 | (0.686, 0.314) | 49 |
| Comparative Example 4 | B : G | 1 : 2 | 5.02 | 38.1 | (0.685, 0.315) | 45 |
| Comparative Example 5 | | 1 : 1 | 4.99 | 41.4 | (0.685, 0.315) | 51 |
| Comparative Example 6 | | 2 : 1 | 4.75 | 46.8 | (0.685, 0.315) | 30 |
| Comparative Example 7 | C : H | 1 : 2 | 5.13 | 44.7 | (0.685, 0.315) | 76 |
| Comparative Example 8 | | 1 : 1 | 5.27 | 52.9 | (0.685, 0.315) | 54 |
| Comparative Example 9 | | 2 : 1 | 4.84 | 51.1 | (0.685, 0.315) | 48 |
| Comparative Example 10 | F : H | 1 : 2 | 5.19 | 35.4 | (0.684, 0.316) | 71 |
| Comparative Example 11 | | 1 : 1 | 4.81 | 39.5 | (0.685, 0.315) | 78 |
| Comparative Example 12 | | 2 : 1 | 4.93 | 41.6 | (0.685, 0.315) | 67 |
| Example 1 | 3 : G | 1 : 2 | 4.42 | 92.8 | (0.685, 0.315) | 120 |
| Example 2 | | 1 : 1 | 4.33 | 99.2 | (0.685, 0.315) | 134 |
| Example 3 | | 2 : 1 | 4.28 | 93.9 | (0.685, 0.315) | 122 |
| Example 4 | 18 : G | 1 : 8 | 4.17 | 101.2 | (0.685, 0.315) | 261 |
| Example 5 | | 1 : 5 | 4.05 | 104.9 | (0.685, 0.315) | 249 |
| Example 6 | | 1 : 2 | 3.96 | 105.7 | (0.685, 0.315) | 224 |
| Example 7 | | 1 : 1 | 3.99 | 108.5 | (0.685, 0.315) | 182 |
| Example 8 | | 2 : 1 | 3.81 | 110.8 | (0.685, 0.315) | 157 |
| Example 9 | | 5 : 1 | 3.86 | 106.9 | (0.685, 0.315) | 138 |
| Example 10 | | 8 : 1 | 4.01 | 103.1 | (0.686, 0.314) | 119 |
| Example 11 | 68 : H | 1 : 2 | 4.12 | 98.5 | (0.685, 0.315) | 255 |
| Example 12 | | 1 : 1 | 3.95 | 101.8 | (0.685, 0.315) | 235 |
| Example 13 | | 2 : 1 | 3.89 | 105.7 | (0.685, | 214 |
| | | | | | 0.315) | |
| Example 14 | 101 : G | 1 : 8 | 4.36 | 99.5 | (0.684, 0.316) | 189 |
| Example 15 | | 1 : 5 | 4.25 | 104.2 | (0.685, 0.315) | 182 |
| Example 16 | | 1 : 2 | 4.05 | 109.5 | (0.685, 0.315) | 208 |
| Example 17 | | 1 : 1 | 4.19 | 108.6 | (0.685, 0.315) | 198 |
| Example 18 | | 2 : 1 | 4.12 | 112.2 | (0.684, 0.316) | 173 |
| Example 19 | | 5 : 1 | 3.91 | 113.8 | (0.685, 0.315) | 147 |
| Example 20 | | 8 : 1 | 3.87 | 115.7 | (0.686, 0.314) | 126 |
| Example 21 | 113 : H | 1 : 8 | 4.15 | 101.2 | (0.685, 0.315) | 180 |
| Example 22 | | 1 : 5 | 4.09 | 109.5 | (0.686, 0.314) | 183 |
| Example 23 | | 1 : 2 | 4.18 | 115.1 | (0.685, 0.315) | 202 |
| Example 24 | | 1 : 1 | 4.24 | 118.9 | (0.685, 0.315) | 188 |
| Example 25 | | 2 : 1 | 3.79 | 121.8 | (0.685, 0.315) | 142 |
| Example 26 | | 5 : 1 | 3.85 | 114.2 | (0.685, 0.315) | 133 |
| Example 27 | | 8 : 1 | 3.94 | 100.5 | (0.684, 0.316) | 128 |
| Example 28 | 241 : H | 1 : 2 | 3.92 | 102.5 | (0.685, 0.315) | 260 |
| Example 29 | | 1 : 1 | 3.80 | 103.6 | (0.685, 0.315) | 251 |
| Example 30 | | 2 : 1 | 3.99 | 108.9 | (0.685, 0.315) | 228 |
| Example 31 | 383 : G | 1 : 2 | 4.12 | 112.8 | (0.685, 0.315) | 149 |
| Example 32 | | 1 : 1 | 4.38 | 110.9 | (0.685, 0.315) | 133 |
| Example 33 | | 2 : 1 | 4.29 | 121.7 | (0.685, 0.315) | 127 |

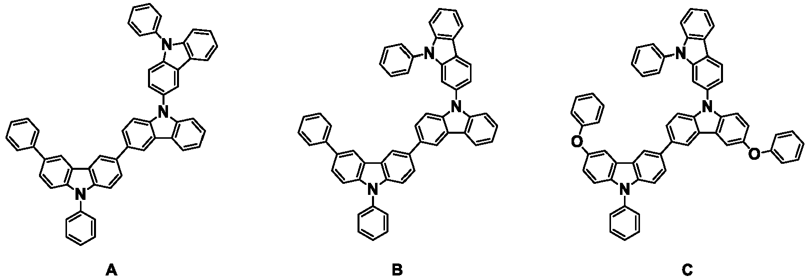

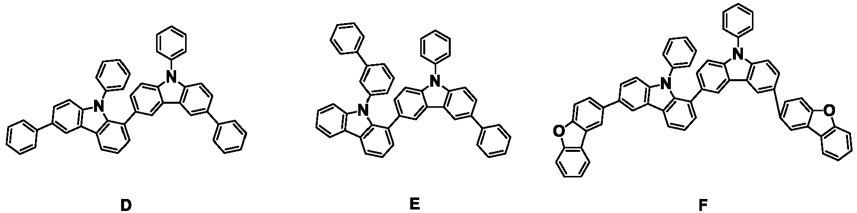

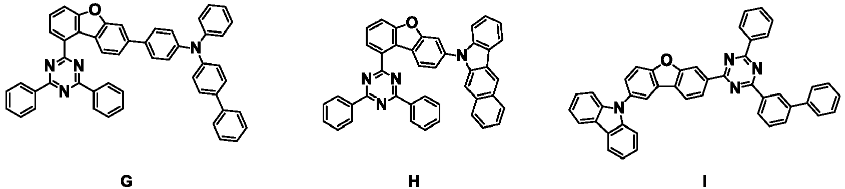

### <Experimental Example 2>

### (1) Manufacturing of organic light emitting device (green host)

A glass substrate coated with a thin film of indium tin oxide (ITO) with a thickness of 1,500Å was ultrasonically washed with distilled water. After washing with distilled water, the substrate was ultrasonically washed with a solvent such as acetone, methanol, isopropyl alcohol, etc., dried, and then treated with ultraviolet ozone (UVO) for 5 minutes using ultraviolet (UV) in a UV cleaner. Thereafter, the substrate was transferred to a plasma cleaner (PT), and then plasma-treated in a vacuum to increase the work function of ITO and remove the remaining film, and transferred to a thermal deposition equipment for organic deposition.

Subsequently, after evacuating the chamber until the vacuum level reaches 10⁻⁶ torr, a hole injection layer having a thickness of 600 Å was deposited on the ITO transparent electrode (a positive electrode) substrate by applying a current to the cell to evaporate 4,4',4"-Tris[2-naphthyl(phenyl)amino]triphenylamine (2-TNATA). N,N'-Di(1-naphthyl)-N,N'-diphenyl-(1,1'-biphenyl)-4,4'-diamine (NPB) was put into another cell in the vacuum deposition equipment, and a current was applied to the cell to evaporate it and thus deposit a hole transport layer with a thickness of 300 Å on the hole injection layer.

On top of that, a light emitting layer was thermally vacuum deposited as follows. The light emitting layer was formed by pre-mixing one of the compounds of Formula 1 in Table 6 below as a P-type host and the compound (I) of Formula 2 in Table 6 below as a N-type host, and doping the host with [Ir(ppy)₃] in an amount of 7 wt.% of the deposition thickness of the light emitting layer while using [Ir(ppy)₃] as the green phosphorescent dopant, thereby depositing with a thickness of 700 Å. Thereafter, bathocuproine (BCP) was deposited with a thickness of 60 Å as a hole blocking layer, and Alq₃ was deposited with a thickness of 200 Å as an electron transport layer on it.

Finally, on the electron transport layer, lithium fluoride (LiF) was deposited with a thickness of 10 Å to form an electron injection layer, and then aluminum (Al) was deposited with a thickness of 1,200 Å on the electron injection layer to form a negative electrode, thereby manufacturing organic light emitting devices of Examples 34 to 61.

Organic light emitting devices of Comparative Examples 13 to 18 were additionally manufactured in the same manner as in Examples, except that in the manufacturing process of the organic light emitting devices of Examples 34 to 61, compounds D and E above were used instead of using the compound of Formula 1 as the P-type host.

On the other hand, all organic compounds necessary for the manufacture of OLED devices were purified by vacuum sublimation under 10⁻⁸ to 10⁻⁶ torr for each material and then used in the manufacture of the organic light-emitting devices.

### (2) Operating voltage and luminous efficiency of organic light emitting device

For the organic light emitting devices of Examples 34 to 61 and Comparative Examples 13 to 18 manufactured as described above, electroluminescence (EL) characteristics were measured with M6000 from McScience Co. With the measurement results, T₉₀ of the green phosphorescent device was measured at a reference luminance of 6,000 cd/m² through the lifetime measuring device (M6000) manufactured by McScience Co.

In the above, T₉₀ means a lifetime (unit: hour (h)) that is a time at which the luminance becomes 90% compared to the initial luminance.

The results of measuring the operating voltage, luminous efficiency, color coordinate (CIE), and lifetime of the organic light emitting devices manufactured according to the present invention are shown in Table 6 below.

**Table 6:**

| | Light emitting layer compound | Ratio | Operating voltage (V) | Efficiency (cd/A) | Color coordinate (x, y) | Lifetime (T₉₀) |
|---|---|---|---|---|---|---|
| Comparative Example 13 | | 1 : 2 | 4.92 | 145.2 | (0.245, 0.717) | 49 |
| Comparative Example 14 | | 1 : 1 | 5.13 | 128.9 | (0.245, 0.717) | 41 |
| Comparative Example 15 | | 2 : 1 | 5.38 | 114.8 | (0.246, 0.718) | 53 |
| Comparative Example 16 | E : I | 1 : 2 | 5.42 | 125.8 | (0.246, 0.718) | 39 |
| Comparative Example 17 | | 1 : 1 | 5.28 | 131.0 | (0.245, 0.717) | 78 |
| Comparative Example 18 | | 2 : 1 | 5.07 | 115.7 | (0.245, 0.717) | 57 |
| Example 34 | 1 : I | 1 : 8 | 4.45 | 271.8 | (0.241, 0.721) | 108 |
| Example 35 | | 1 : 5 | 4.31 | 300.97 | (0.245, 0.717) | 112 |
| Example 36 | | 1 : 2 | 4.28 | 303.73 | (0.246, 0.718) | 132 |
| Example 37 | | 1 : 1 | 4.25 | 309.35 | (0.245, 0.717) | 154 |
| Example 38 | | 2 : 1 | 4.33 | 301.19 | (0.245, 0.717) | 221 |
| Example 39 | | 5 : 1 | 4.38 | 299.90 | (0.241, 0.721) | 198 |
| Example 40 | 42 : I | 1 : 2 | 4.41 | 303.12 | (0.245, 0.717) | 193 |
| Example 41 | | 1 : 1 | 4.32 | 306.02 | (0.245, 0.717) | 188 |
| Example 42 | | 2 : 1 | 4.24 | 308.18 | (0.245, 0.717) | 208 |
| Example 43 | 161 : I | 1 : 2 | 4.26 | 292.99 | (0.245, 0.717) | 289 |
| Example 44 | | 1 : 1 | 4.29 | 276.40 | (0.241, 0.721) | 308 |
| Example 45 | | 2 : 1 | 4.38 | 280.46 | (0.246, 0.718) | 299 |
| Example 46 | 219 : I | 1 : 2 | 4.47 | 271.87 | (0.245, 0.717) | 173 |
| Example 47 | | 1 : 1 | 4.42 | 294.14 | (0.245, 0.717) | 141 |
| Example 48 | | 2 : 1 | 4.35 | 288.83 | (0.245, 0.717) | 122 |
| Example 49 | 291 : I | 1 : 8 | 4.72 | 293.78 | (0.245, 0.717) | 180 |
| Example 50 | | 1 : 5 | 4.63 | 295.52 | (0.241, 0.721) | 208 |
| Example 51 | | 1 : 2 | 4.56 | 305.46 | (0.245, 0.717) | 197 |
| Example 52 | | 1 : 1 | 4.51 | 307.69 | (0.245, 0.717) | 171 |
| Example 53 | | 2 : 1 | 4.45 | 306.87 | (0.245, 0.717) | 167 |
| Example 54 | | 5 : 1 | 4.34 | 300.91 | (0.245, 0.717) | 125 |
| Example 55 | | 8 : 1 | 4.22 | 298.15 | (0.246, 0.718) | 148 |
| Example 56 | 377 : I | 1 : 2 | 4.38 | 297.19 | (0.245, 0.717) | 171 |
| Example 57 | | 1 : 1 | 4.37 | 294.35 | (0.245, 0.717) | 148 |
| Example 58 | | 2 : 1 | 4.42 | 299.71 | (0.246, 0.718) | 123 |
| Example 59 | 401: I | 1 : 2 | 3.81 | 108.8 | (0.685, 0.315) | 159 |
| Example 60 | | 1 : 1 | 3.79 | 104.9 | (0.685, 0.315) | 163 |
| Example 61 | | 2 : 1 | 3.84 | 101.5 | (0.685, 0.315) | 155 |

From Experimental Examples 1 and 2, it was confirmed that if the heterocyclic compound of Formula 1 is used as a host of an organic layer of an organic light emitting device, particularly, a light emitting layer, the operating voltage, the efficiency, and the lifetime may be improved.

Specifically, it was confirmed that in the case of an embodiment using the heterocyclic compound of Formula 1 of the present invention as a P-type host, it has better operating voltage, efficiency, and lifetime in both the red host and the green host, as compared to the case of the comparative example using the compounds of compounds A to F as the P-type host.

It was confirmed that in the case of Examples 1 to 61 using the heterocyclic compound of Formula 1, since it has a steric configuration by fixing the substituent and spatially separates the Highest Occupied Molecular Orbital (HOMO) and Lowest Unoccupied Molecular Orbital (LUMO) to enable strong charge transfer, it is suitable as a red host and a green host, and high efficiency can be expected when used as an organic material in an organic light emitting device.

In addition, conventional Bis-Carbazole used as a P-type host was not effective in terms of energy transfer when applied to a red host, but it was confirmed that in the present invention, the heterocyclic compound of Formula 1 was introduced, and it was suitable for use as a red host by reducing the bandgap and thus improving the stability of the molecule, effectively lowering T1 (triplet energy level), and improving the stability of excited electrons.

All simple modifications and variations of the present invention fall within the scope of the present invention, and the specific protection scope of the present invention will be clarified by the appended claims.

### [Description of Symbol]

- 100:: Substrate
- 200:: Positive electrode
- 300:: Organic layer
- 301:: Hole injection layer
- 302:: Hole transport layer
- 303:: Light emitting layer
- 304:: Hole blocking layer
- 305:: Electron transport layer
- 306:: Electron injection layer
- 400:: Negative electrode

## Claims

1. A heterocyclic compound represented by Formula 1 below: 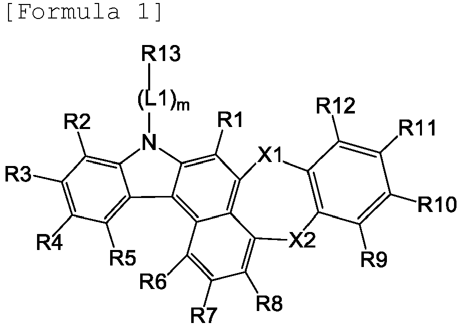 wherein,
one of X1 and X2 is O or S, and the other is a direct bond,
L1 is a single bond; a substituted or unsubstituted C6 to C60 arylene group; or a substituted or unsubstituted C2 to C60 heteroarylene group,
m is an integer from 0 to 5, and when m is 2 or more, each L1 is the same as or different from each other,
R1 to R13 are the same as or different from each other, and are each independently selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; - P(=O)R101R102; -SiR101R102R103; and -NR101R102, or two or more groups adjacent to each other are combined with each other to form a substituted or unsubstituted C6 to C60 aromatic hydrocarbon ring or a substituted or unsubstituted C2 to C60 heterocycle, wherein R101, R102, and R103 are the same as or different from each other, and are each independently a substituted or unsubstituted C1 to C60 alkyl group; a substituted or unsubstituted C6 to C60 aryl group; or a substituted or unsubstituted C2 to C60 heteroaryl group.

2. The heterocyclic compound according to claim 1, wherein Formula 1 is represented by Formula 1-1 or Formula 1-2 below: 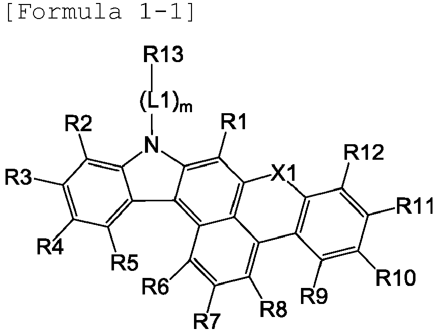 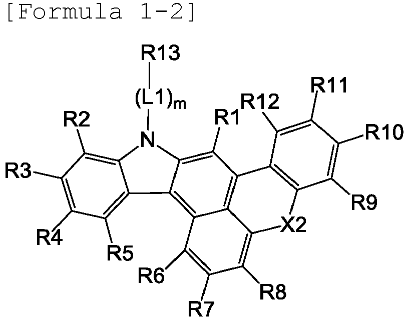 wherein X1, X2, L1, R1 to R13, and m are the same as defined in Formula 1 above.

3. The heterocyclic compound according to claim 1, wherein any one of R1 to R13 is represented by Structural Formula A or Structural Formula B below: 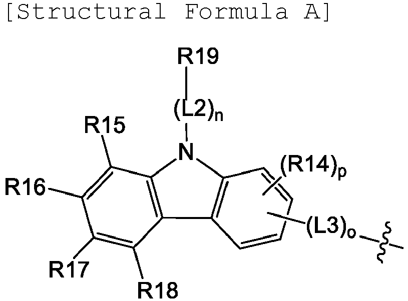 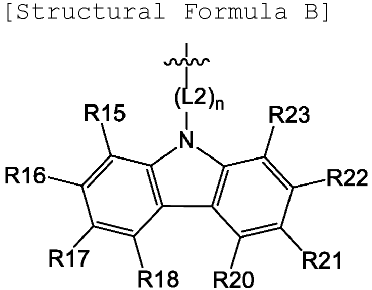 wherein,
L2 and L3 are the same as or different from each other, and are each independently a single bond; a substituted or unsubstituted C6 to C60 arylene group; or a substituted or unsubstituted C2 to C60 heteroarylene group,
n is an integer from 0 to 5, and when n is 2 or more, each L2 is the same as or different from each other, o is an integer from 0 to 5, and when o is 2 or more, each L3 is the same as or different from each other, p is an integer from 0 to 3, and when p is 2 or more, each R14 is the same as or different from each other,
R14 to R23 are the same as or different from each other, and are each independently selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; - P(=0)R101R102; -SiR101R102R103; and -NR101R102, or two or more groups adjacent to each other are combined with each other to form a substituted or unsubstituted C6 to C60 aromatic hydrocarbon ring or a substituted or unsubstituted C2 to C60 heterocycle, wherein R101, R102, and R103 are the same as or different from each other, and are each independently a substituted or unsubstituted C1 to C60 alkyl group; a substituted or unsubstituted C6 to C60 aryl group; or a substituted or unsubstituted C2 to C60 heteroaryl group.

4. The heterocyclic compound according to claim 3, wherein Structural Formula A is represented by any one of Structural Formula A-1 to Structural Formula A-4 below: 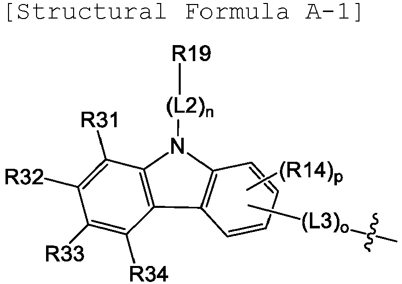 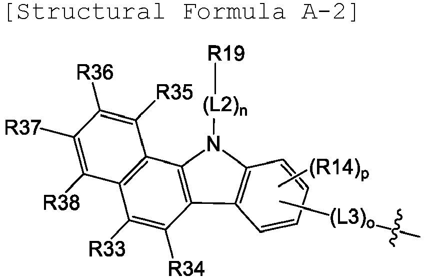 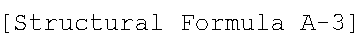 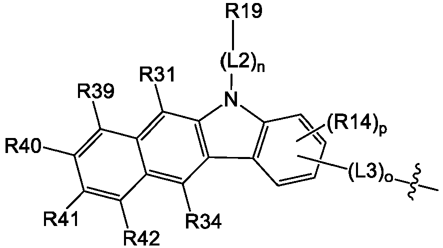 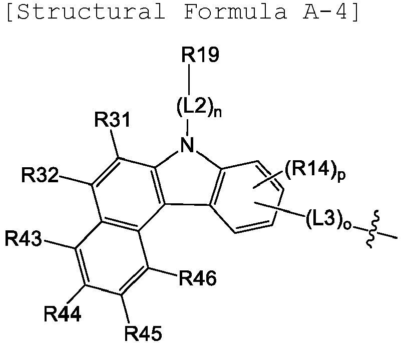 wherein,
R31 to R46 are the same as or different from each other, and are each independently selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; - P(=0)R101R102; -SiR101R102R103; and -NR101R102, or two or more groups adjacent to each other are combined with each other to form a substituted or unsubstituted C6 to C60 aromatic hydrocarbon ring or a substituted or unsubstituted C2 to C60 heterocycle, wherein R101, R102, and R103 are the same as or different from each other, and are each independently a substituted or unsubstituted C1 to C60 alkyl group; a substituted or unsubstituted C6 to C60 aryl group; or a substituted or unsubstituted C2 to C60 heteroaryl group, and
L2, L3, R14, R19, n, o, and p are the same as defined in Structural Formula A above.

5. The heterocyclic compound according to claim 3, wherein R19 is represented by any one of Structural Formula A-5-1 to Structural Formula A-5-5 below: 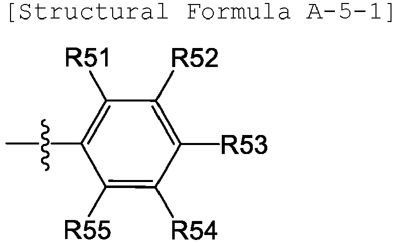 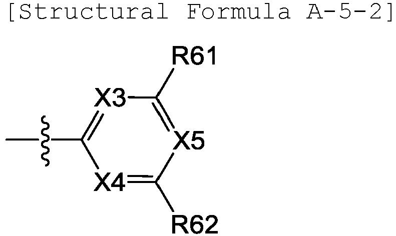 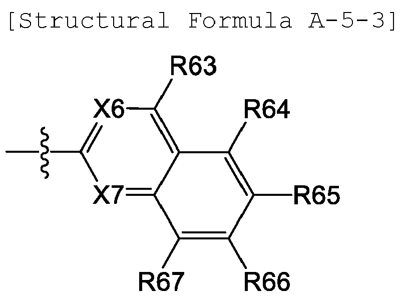 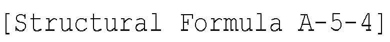 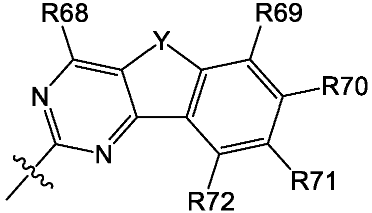 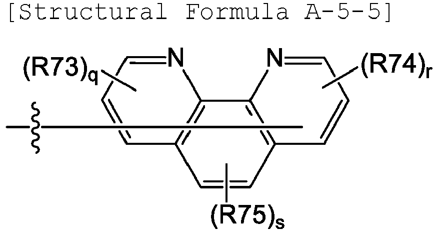 wherein,
X3 is N or CRa, X4 is N or CRb, X5 is N or CRc, X6 is N or CRd, and X7 is N or CRe,
Y is O or S,
R51 to R55, R61 to R75, and Ra to Re are the same as or different from each other, and are each independently selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; - P(=0)R101R102; -SiR101R102R103; and -NR101R102, or two or more groups adjacent to each other are combined with each other to form a substituted or unsubstituted C6 to C60 aromatic hydrocarbon ring or a substituted or unsubstituted C2 to C60 heterocycle, wherein R101, R102, and R103 are the same as or different from each other, and are each independently a substituted or unsubstituted C1 to C60 alkyl group; a substituted or unsubstituted C6 to C60 aryl group; or a substituted or unsubstituted C2 to C60 heteroaryl group,
q is an integer from 0 to 3, and when q is 2 or more, each R73 is the same as or different from each other, r is an integer of 0 to 3, and when r is 2 or more, each R74 is the same as or different from each other, s is an integer from 0 to 2, and when s is 2, each R75 is the same as or different from each other.

6. The heterocyclic compound according to claim 3, wherein any one of R3, R4, R10, R11, and R13 of Formula 1 is represented by Structural Formula A or Structural Formula B above.

7. The heterocyclic compound according to claim 1, wherein Formula 1 is represented by any one of the compounds below:

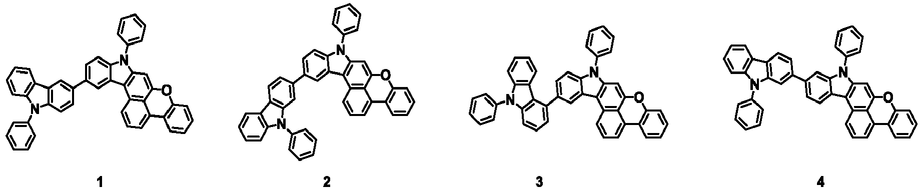

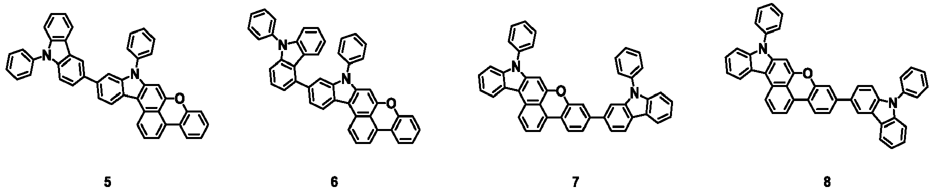

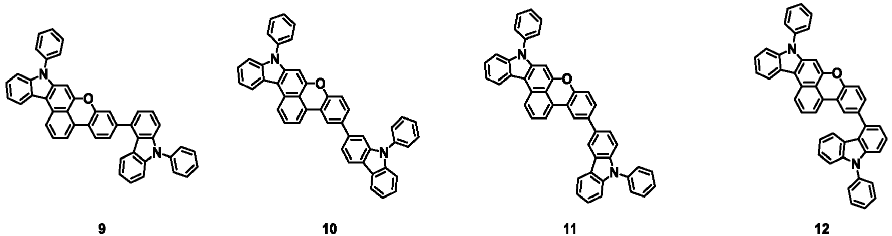

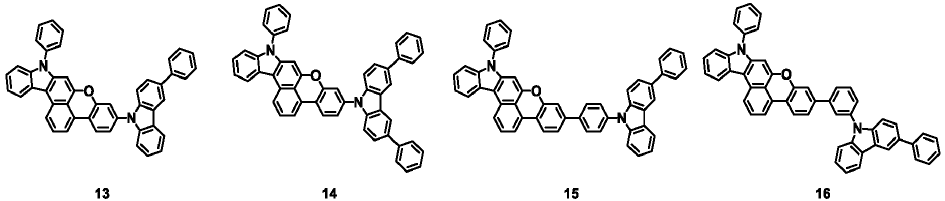

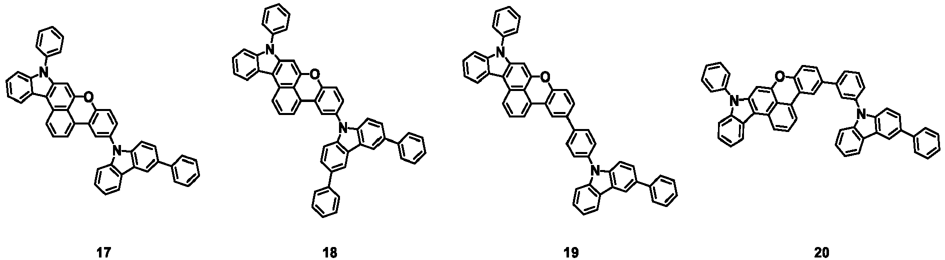

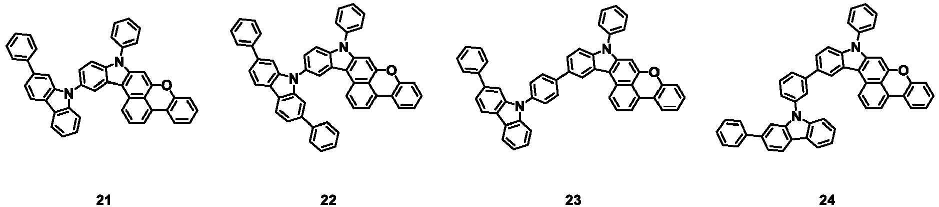

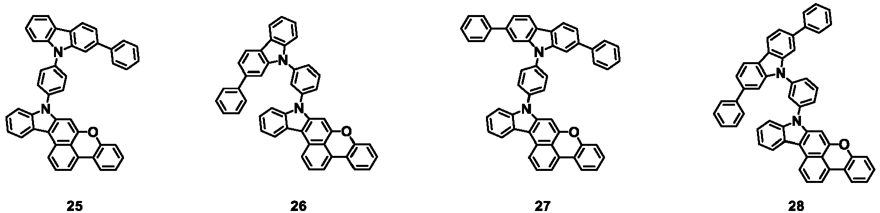

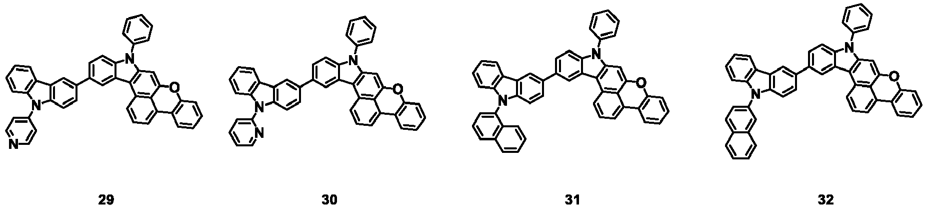

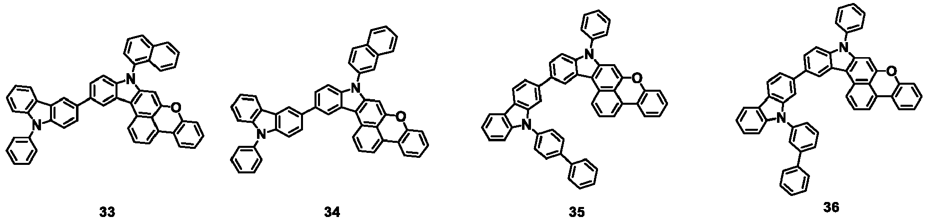

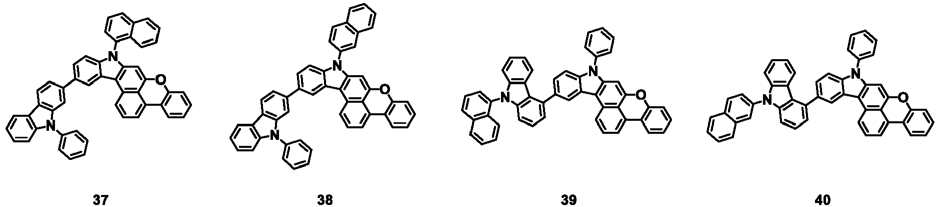

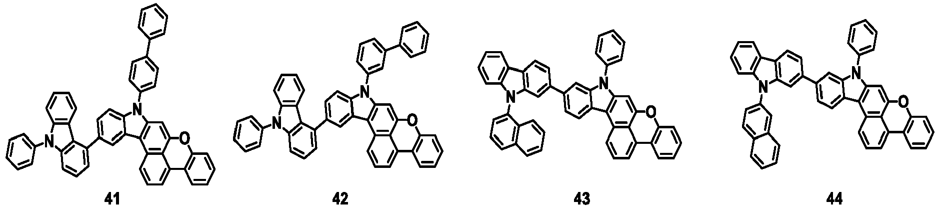

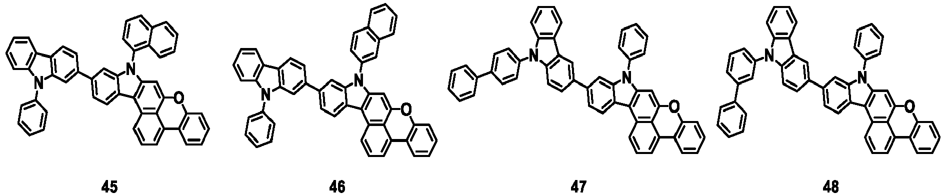

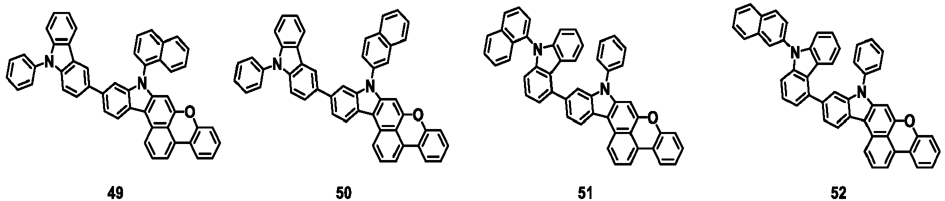

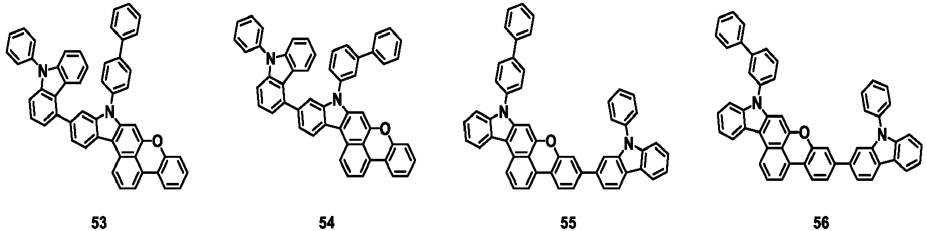

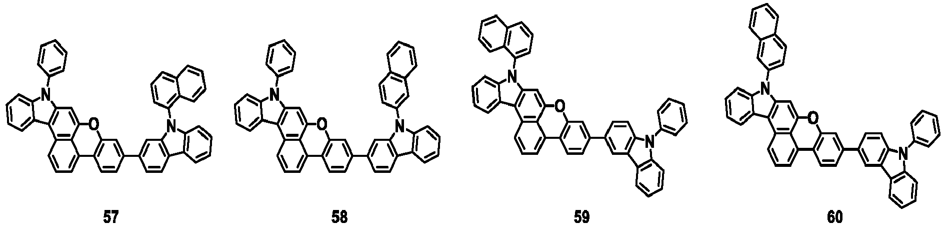

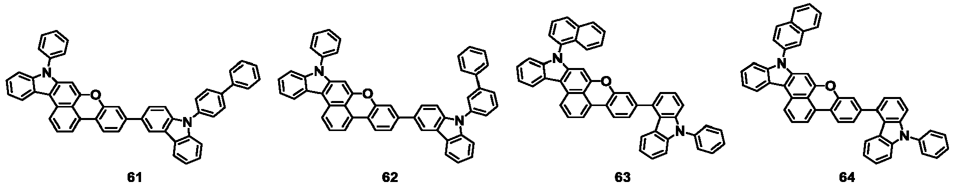

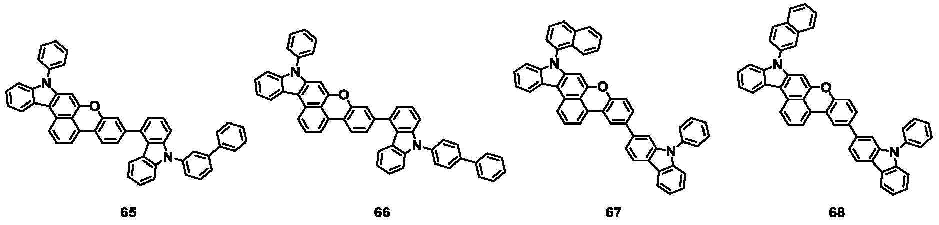

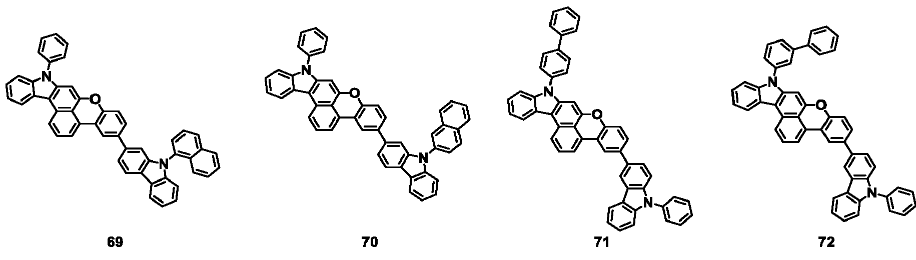

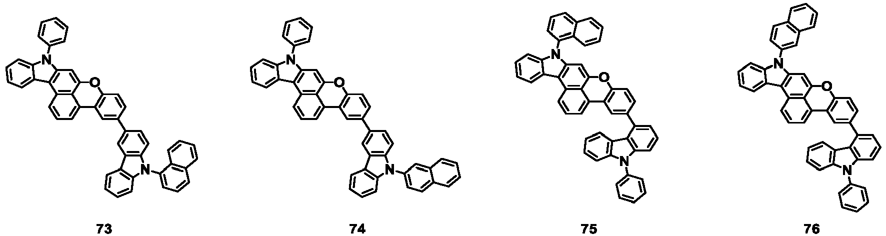

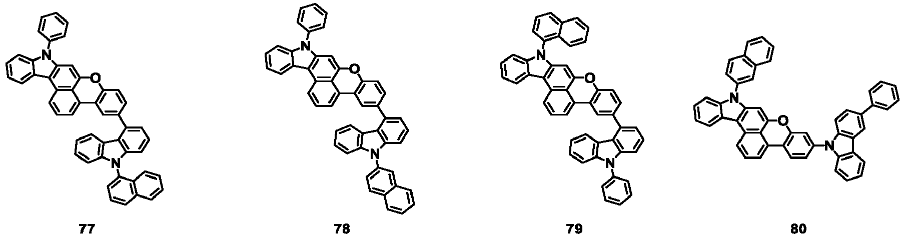

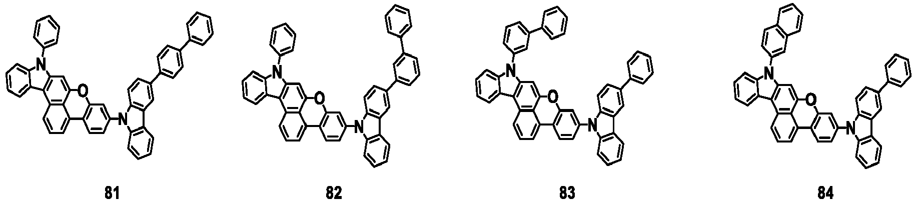

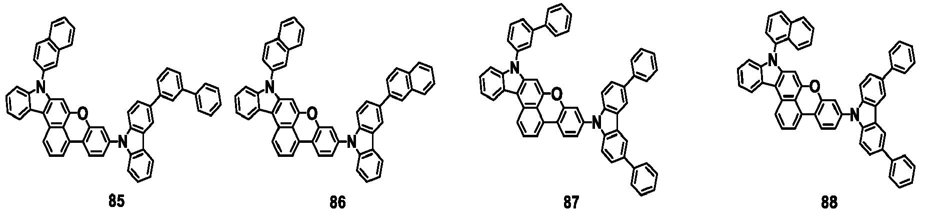

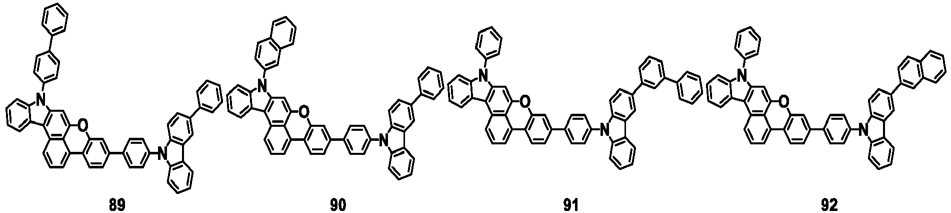

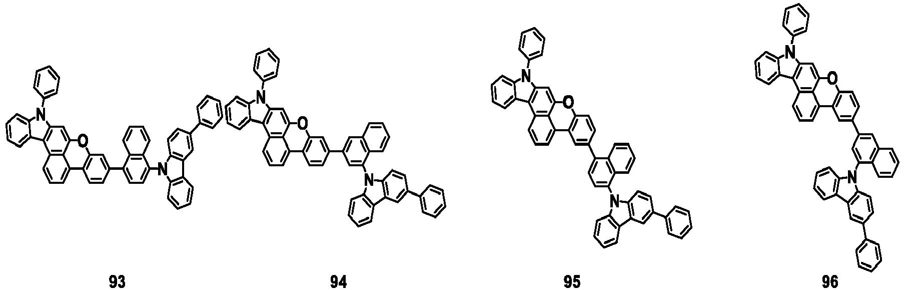

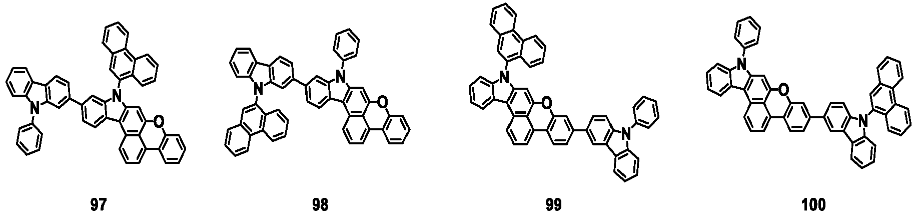

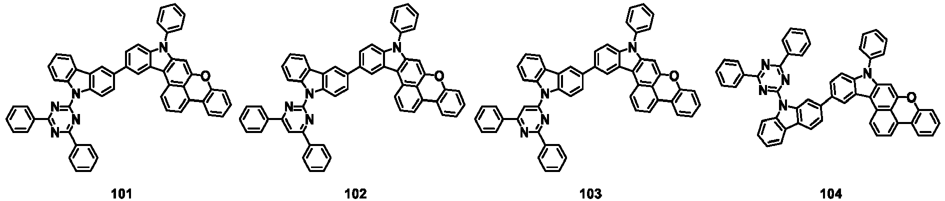

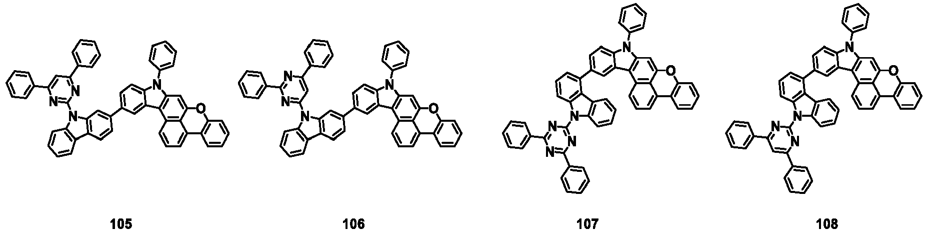

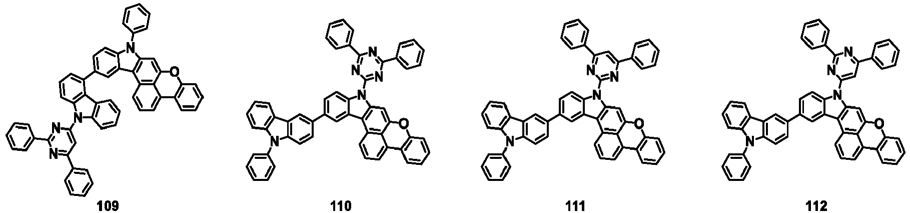

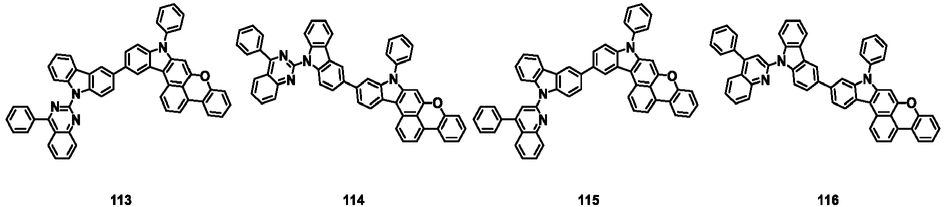

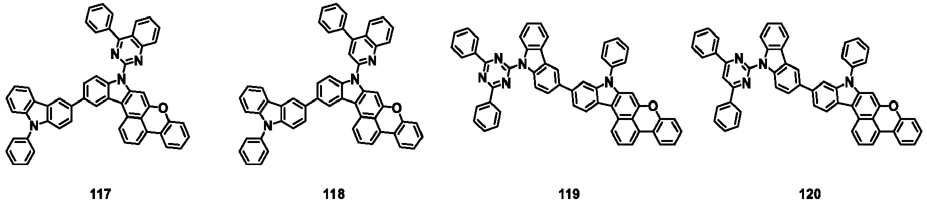

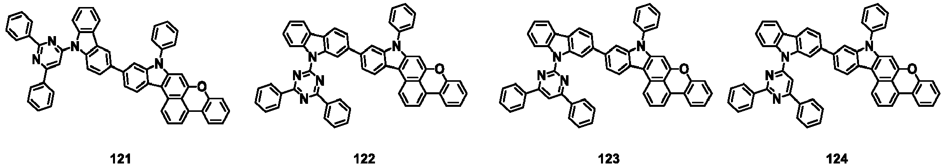

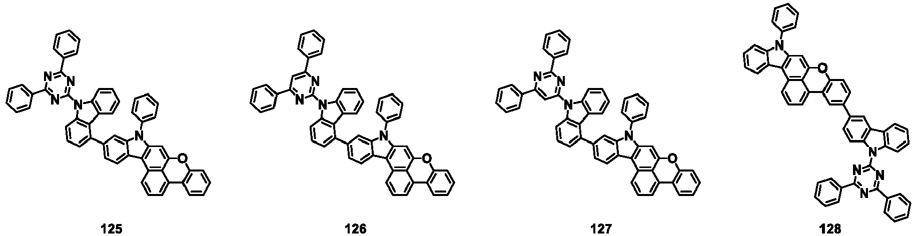

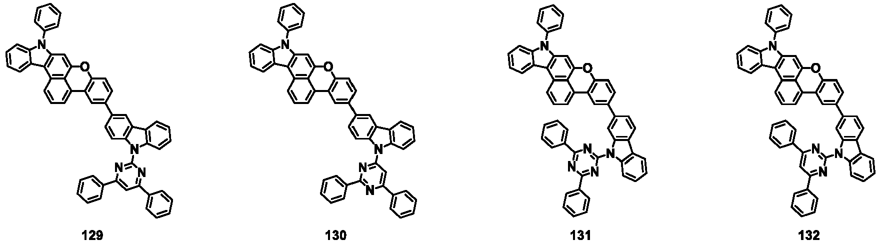

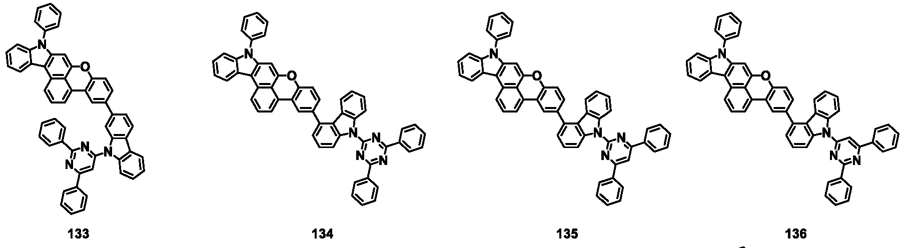

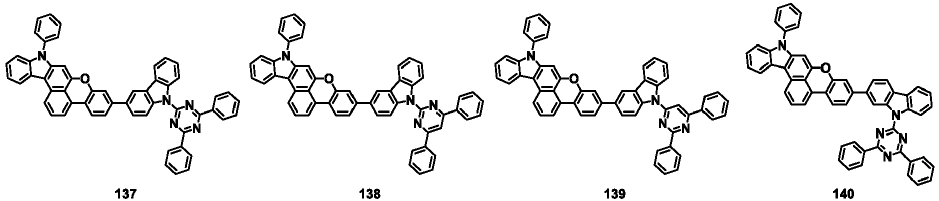

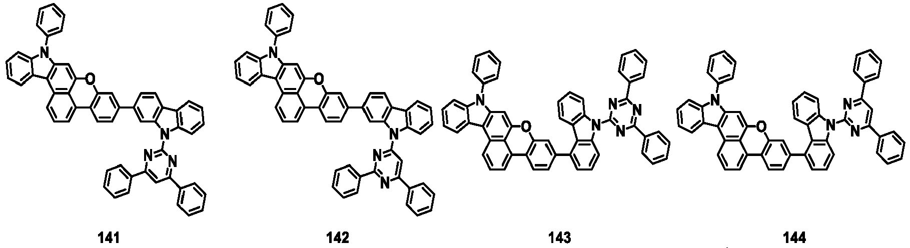

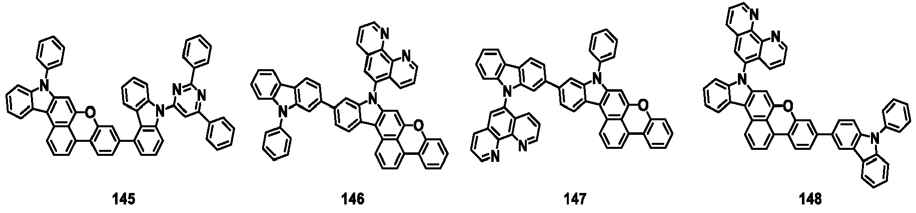

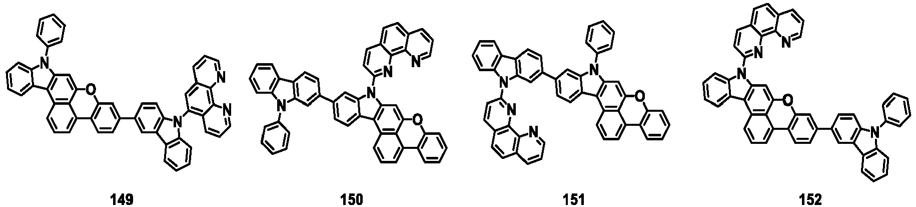

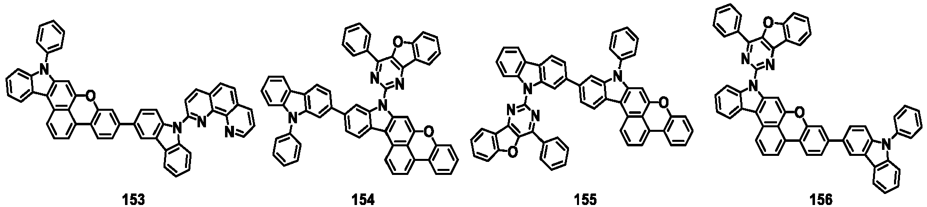

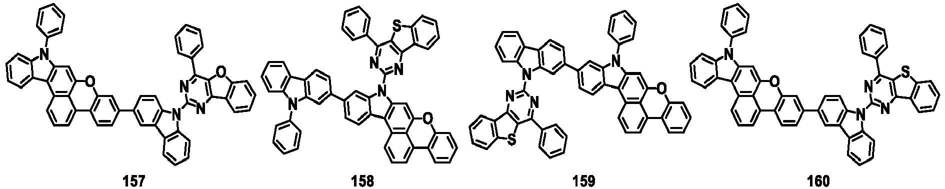

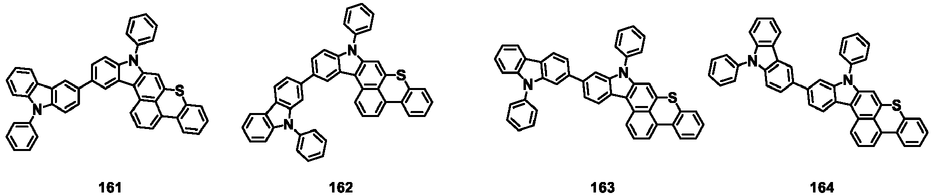

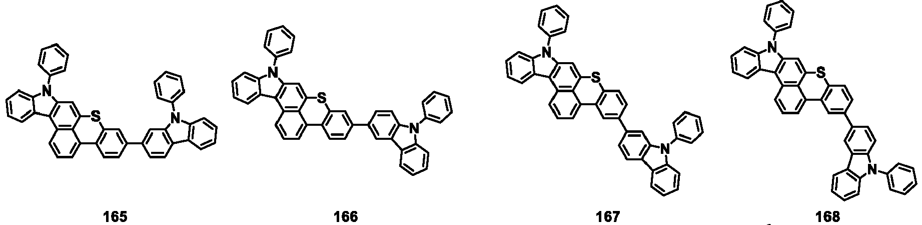

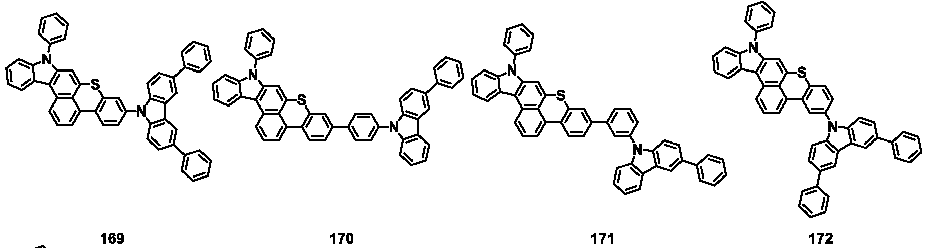

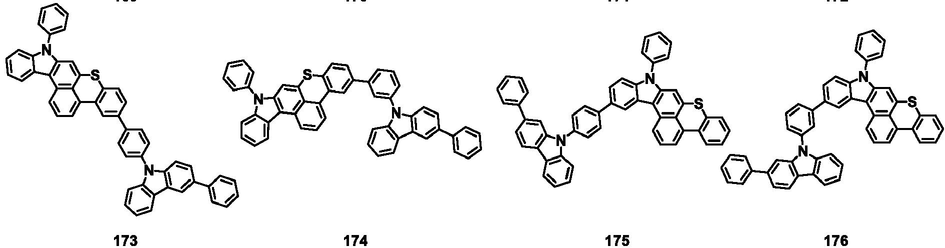

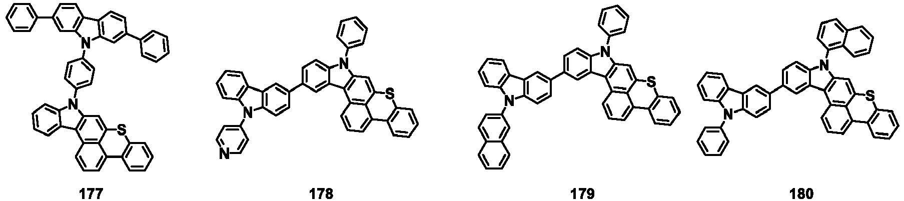

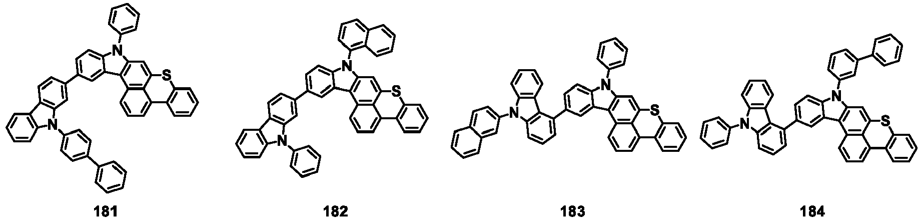

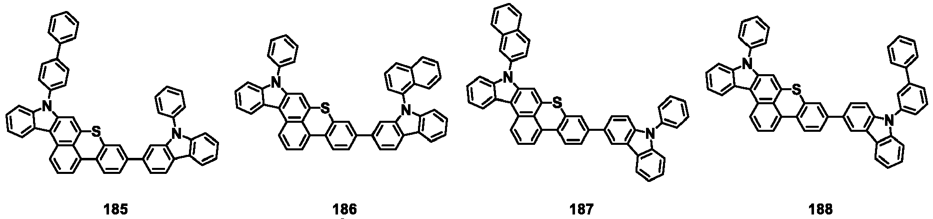

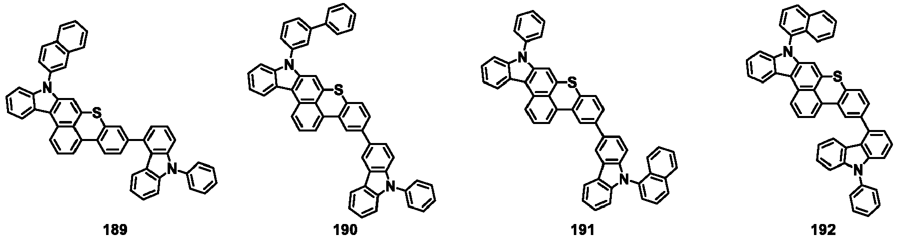

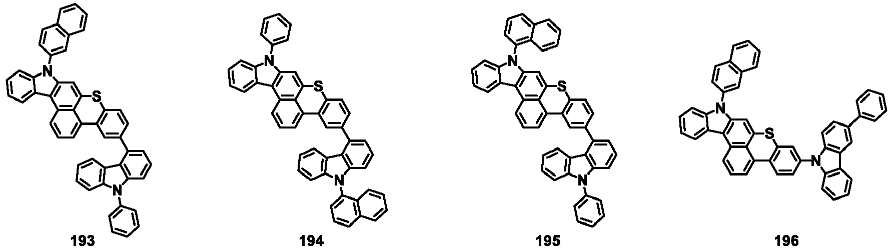

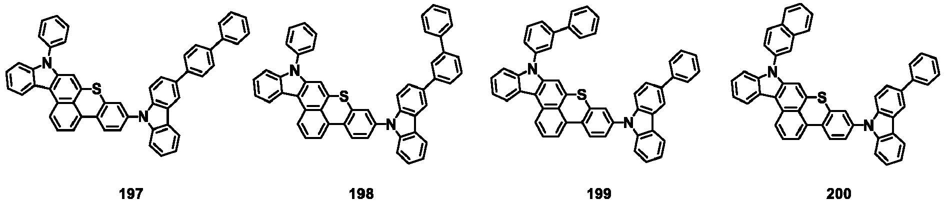

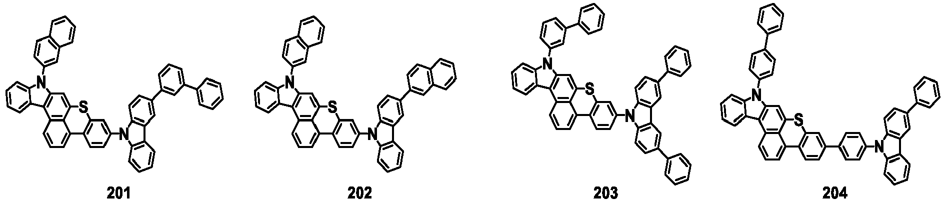

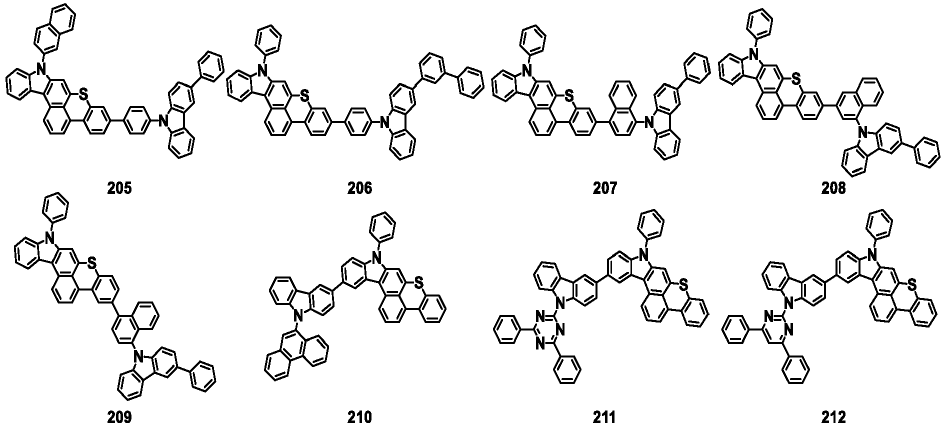

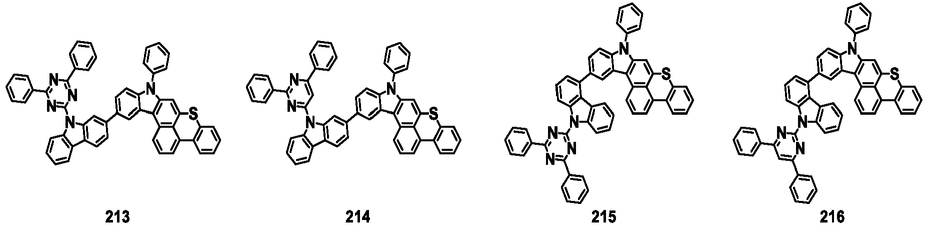

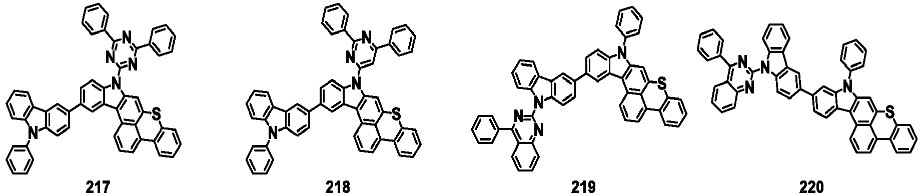

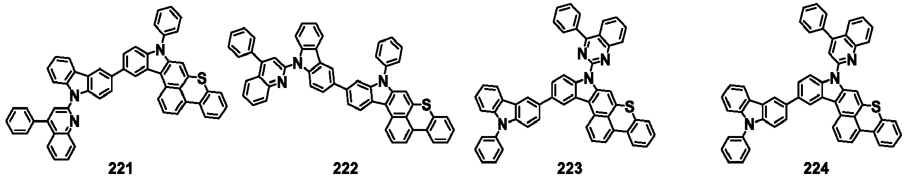

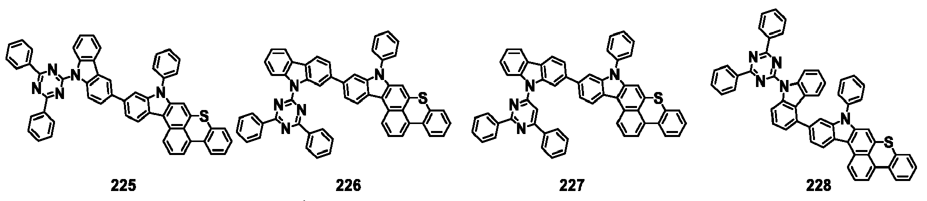

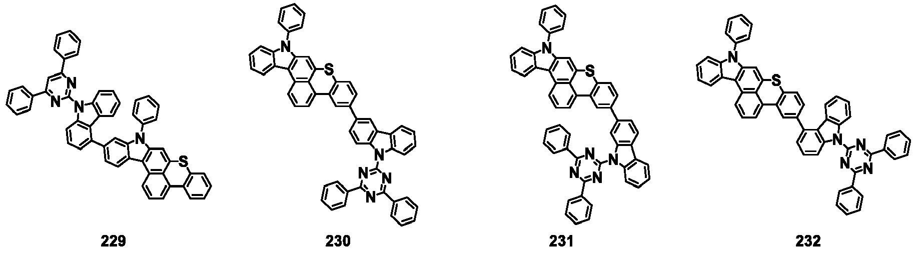

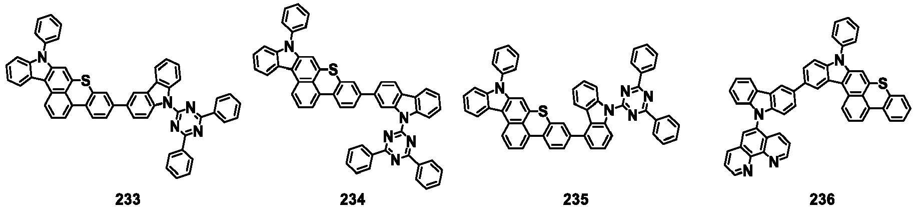

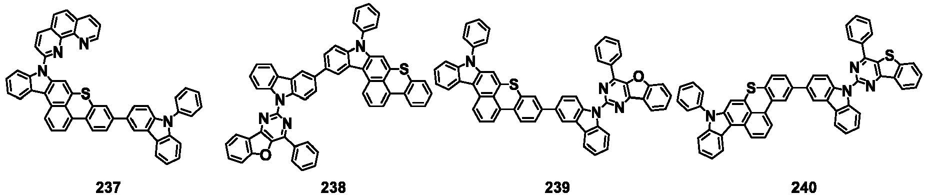

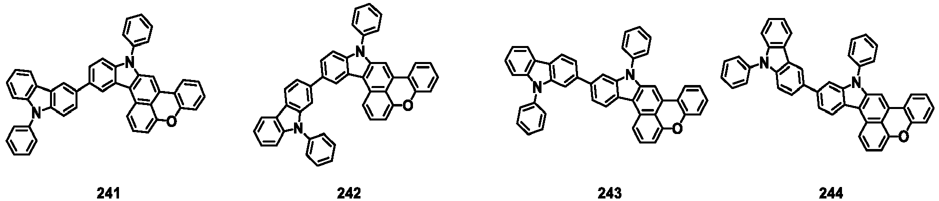

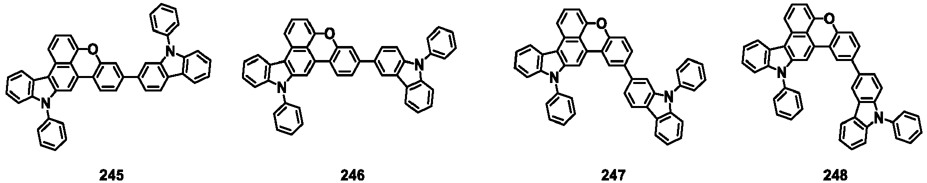

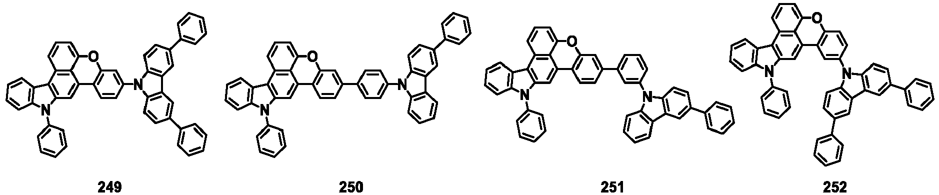

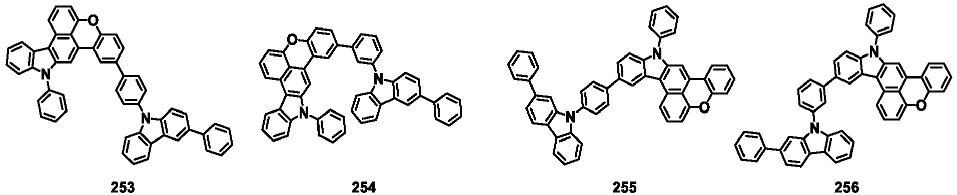

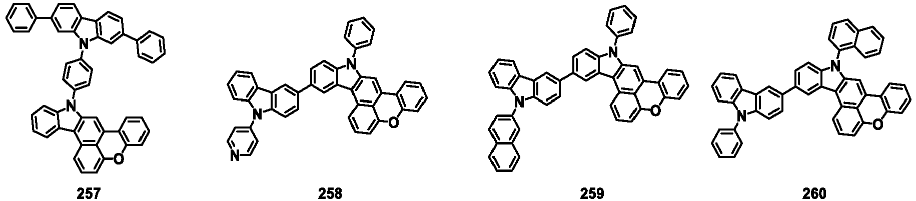

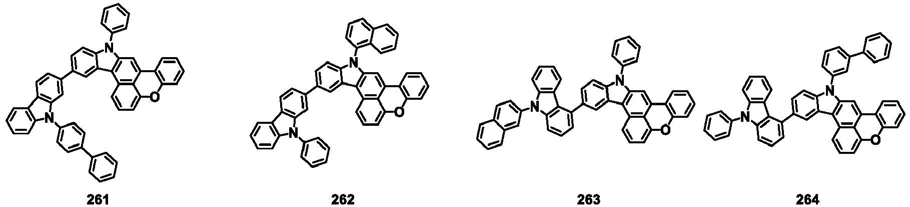

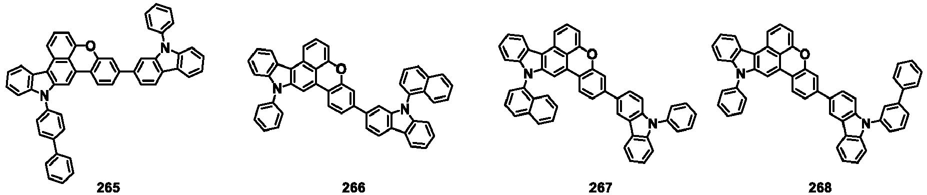

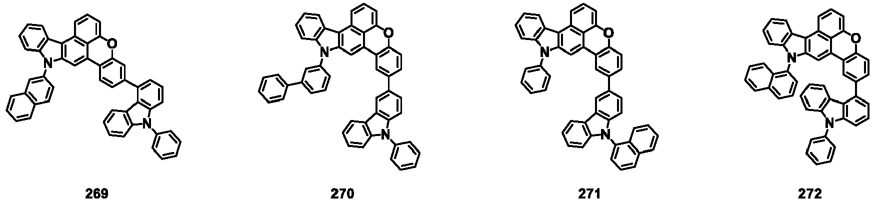

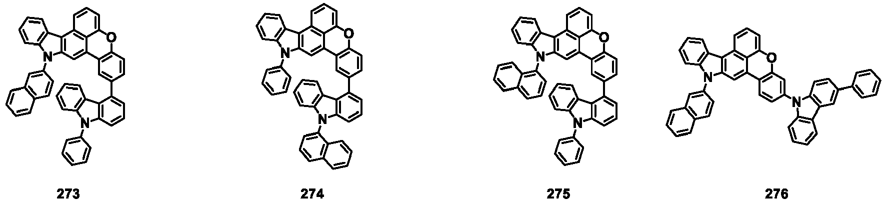

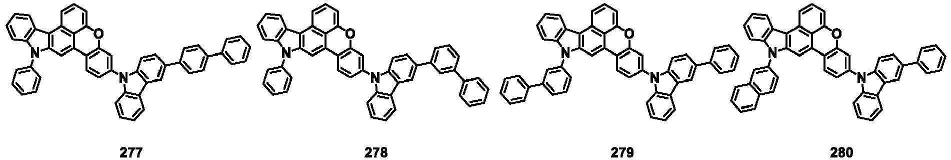

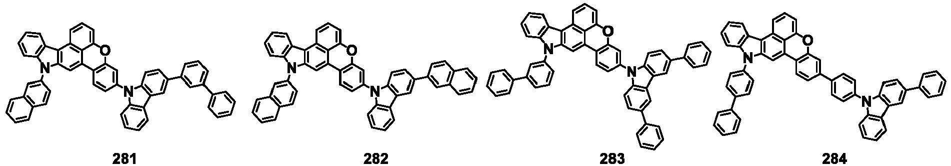

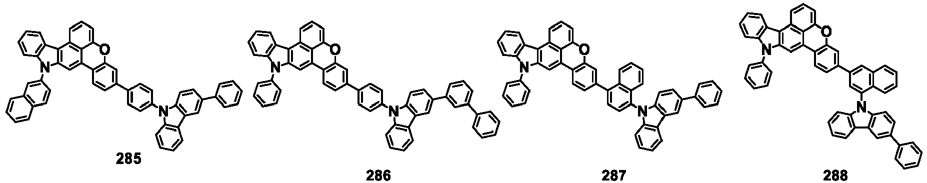

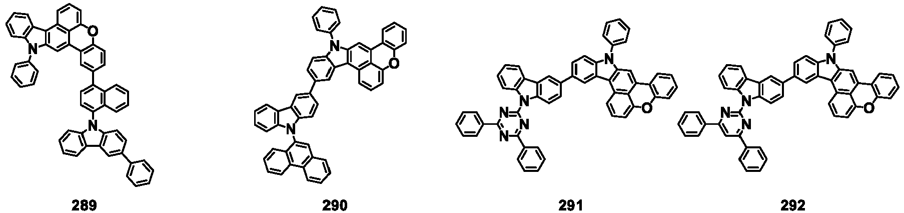

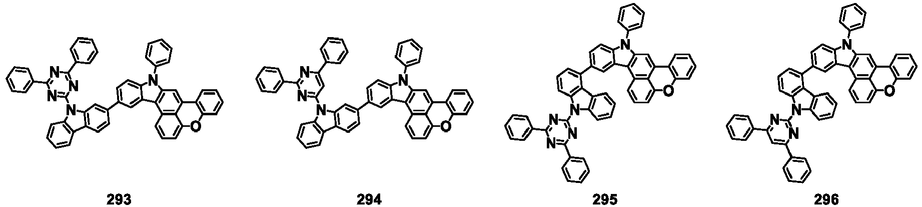

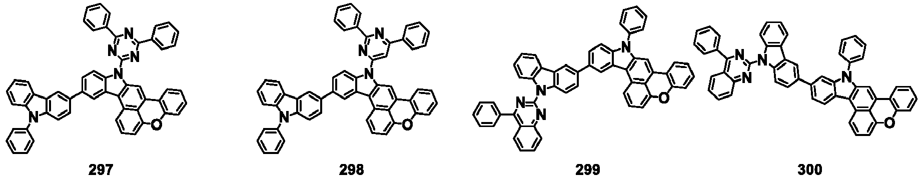

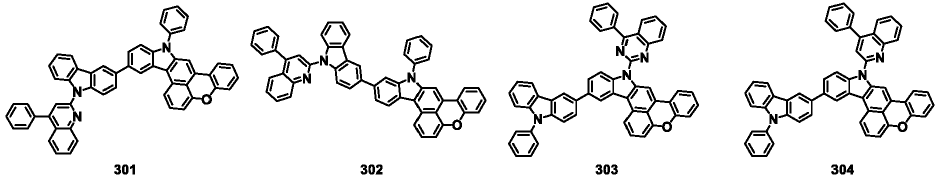

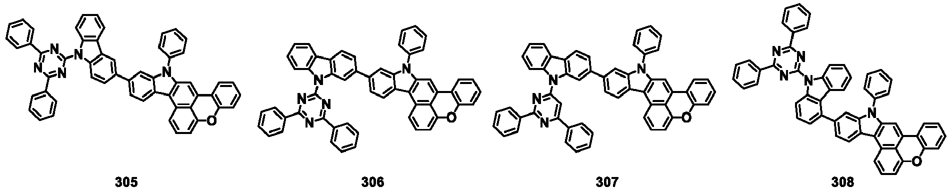

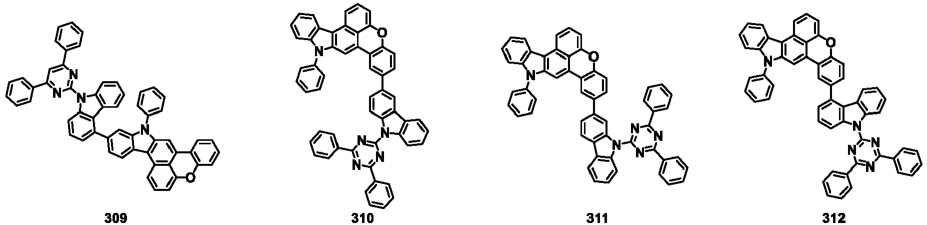

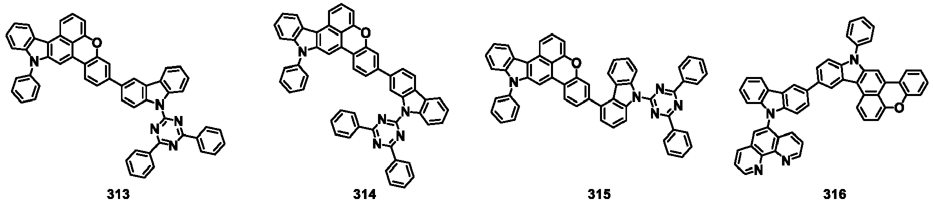

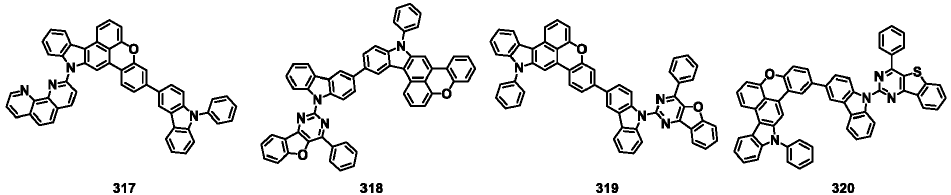

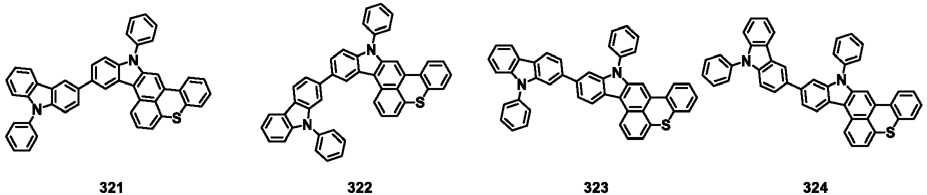

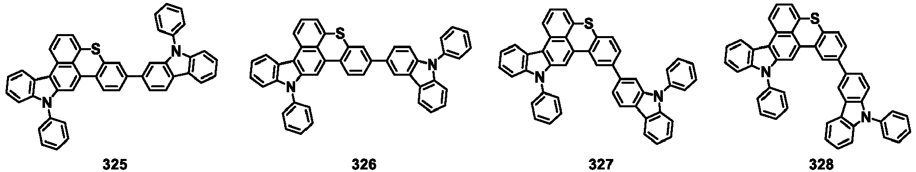

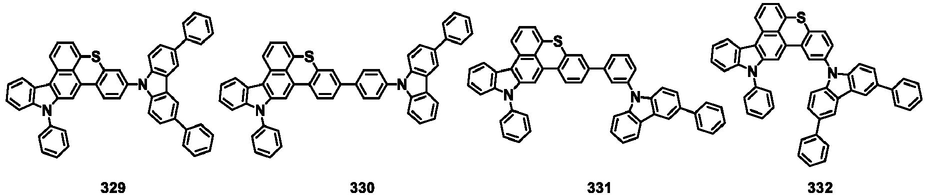

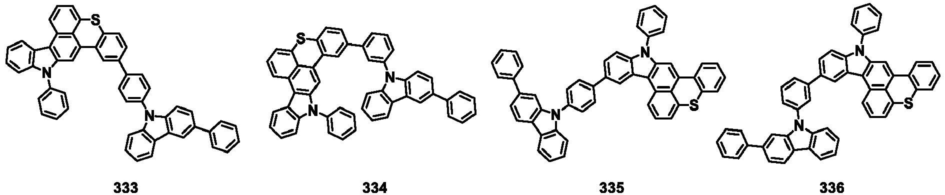

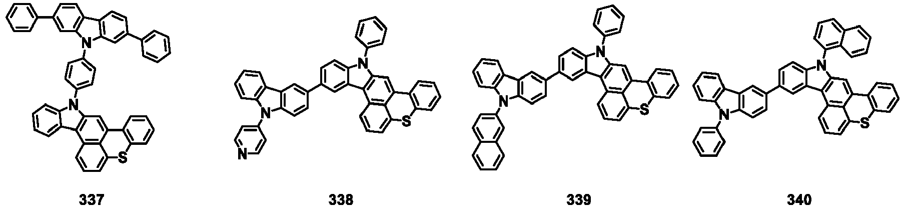

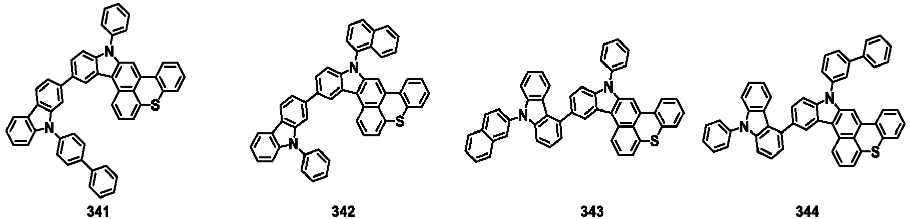

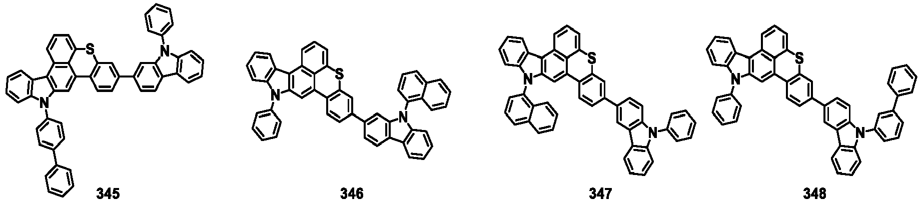

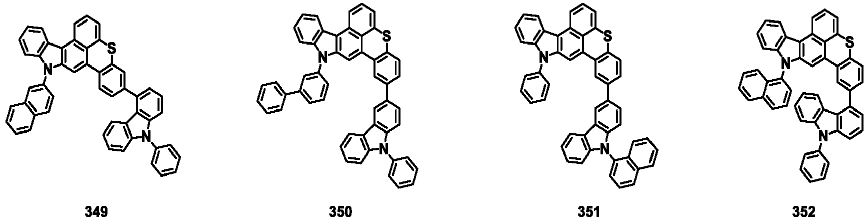

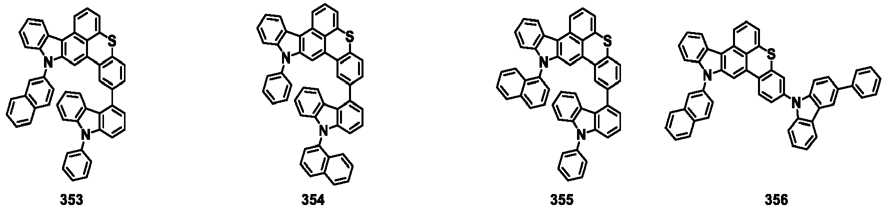

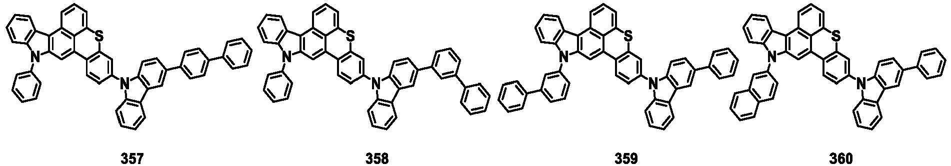

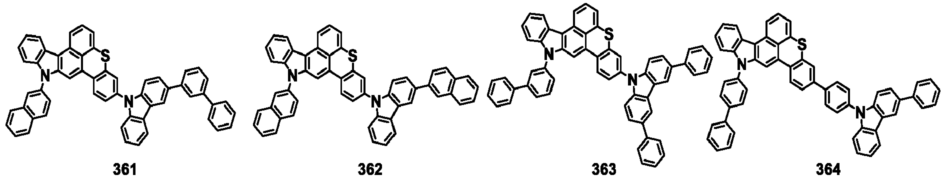

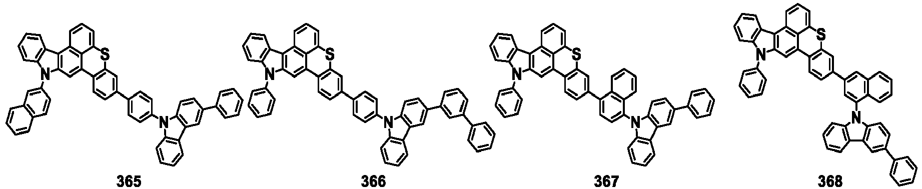

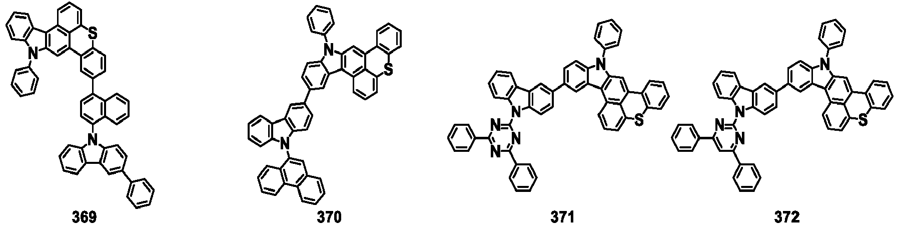

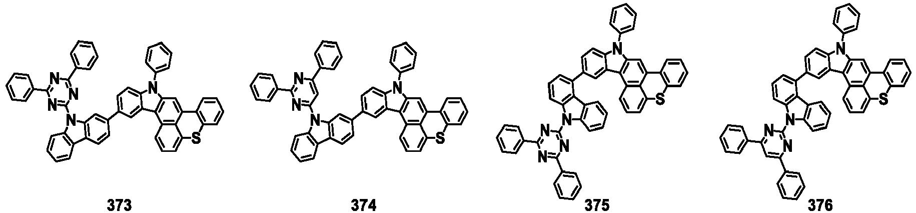

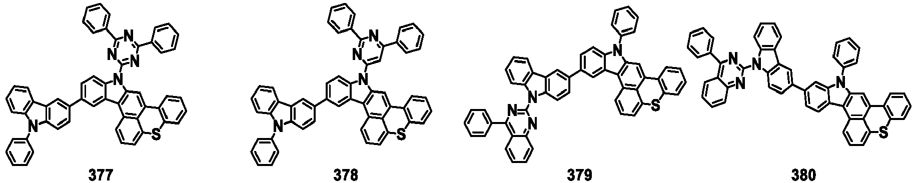

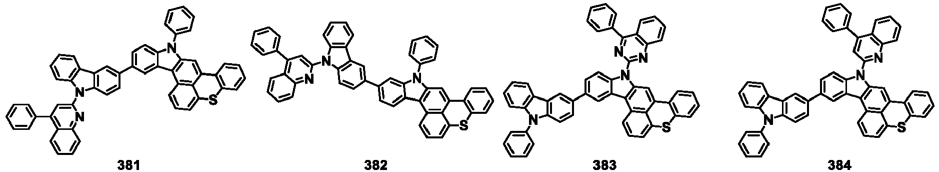

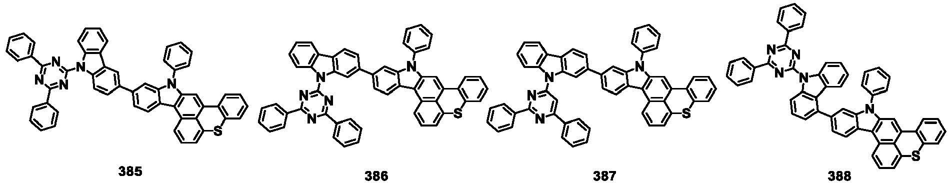

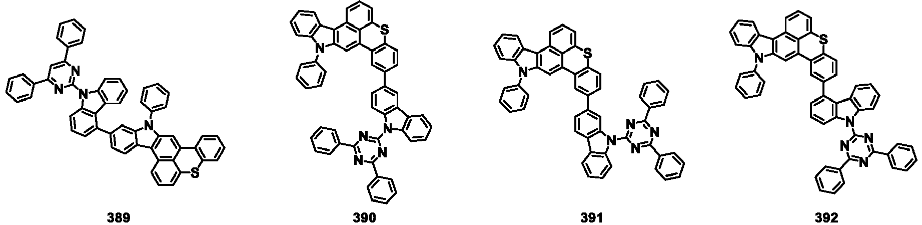

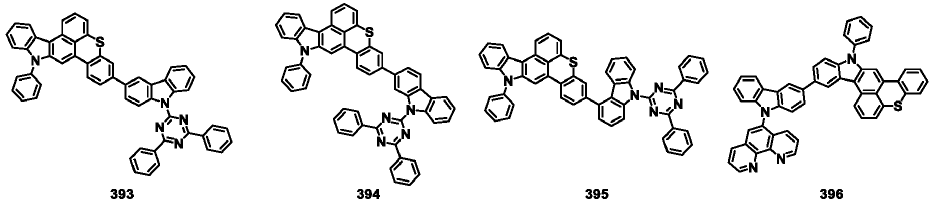

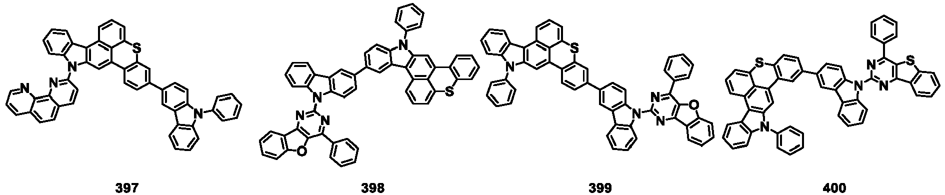

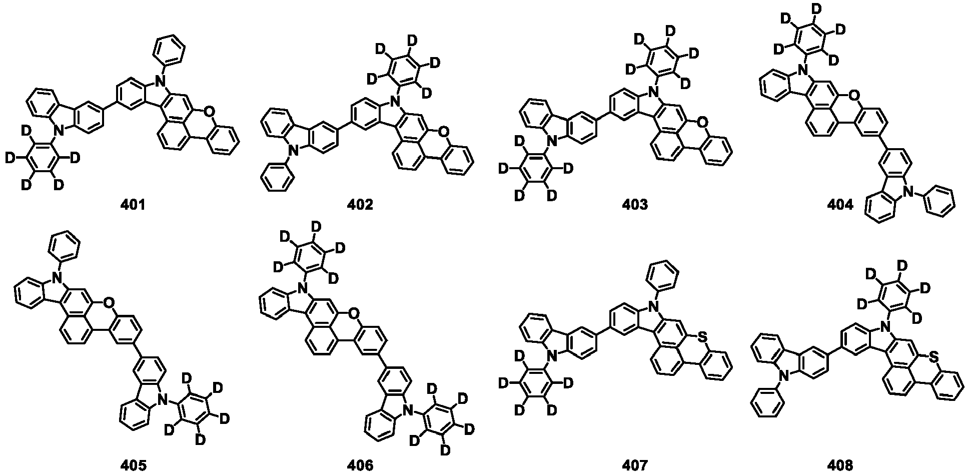

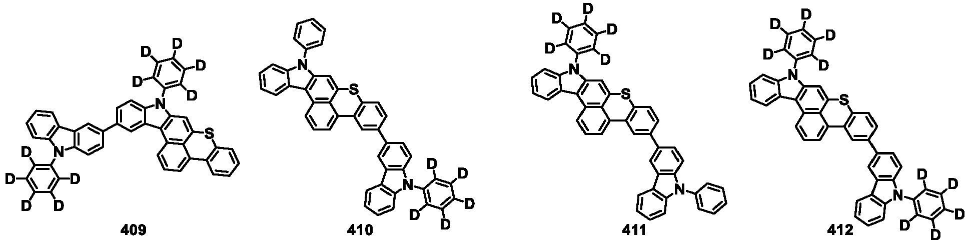

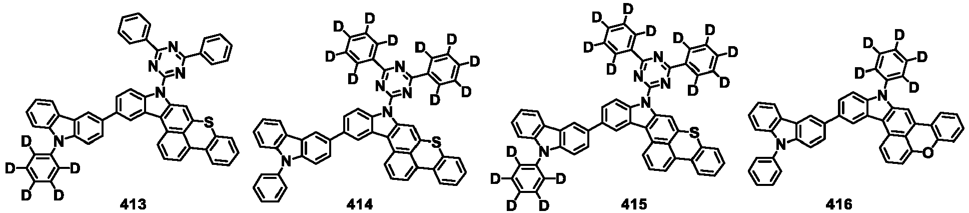

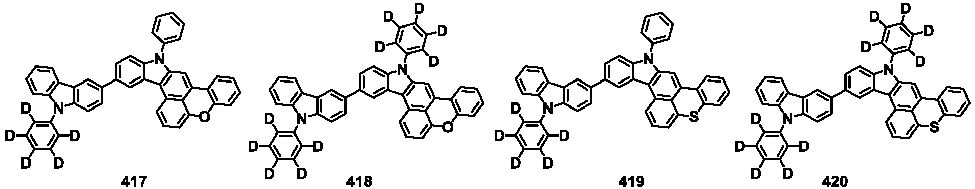

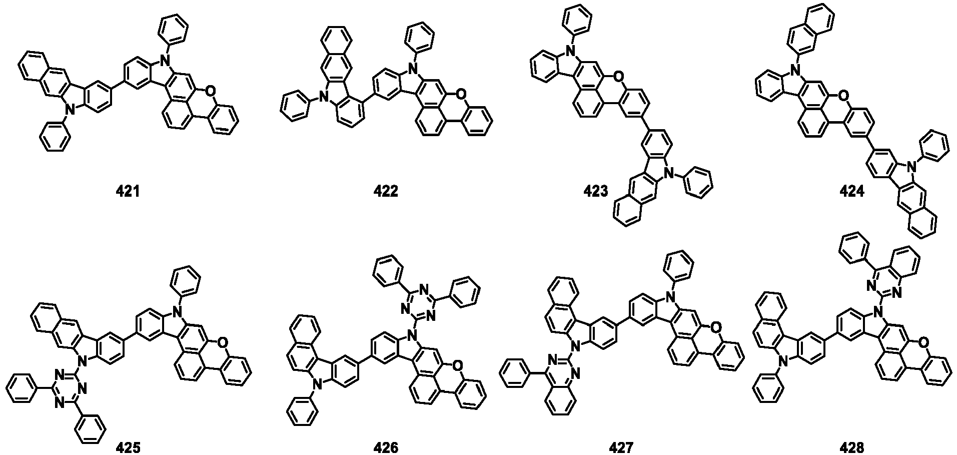

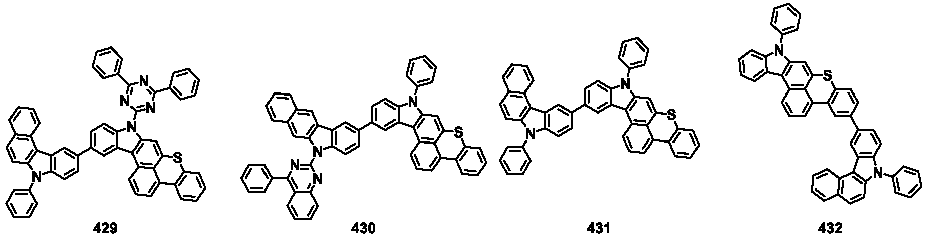

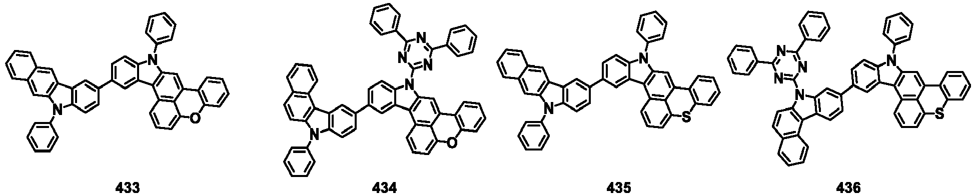

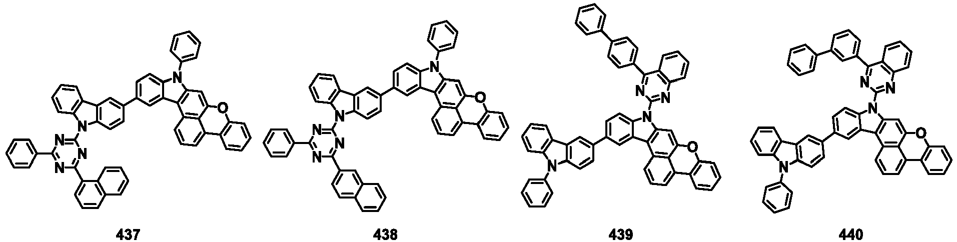

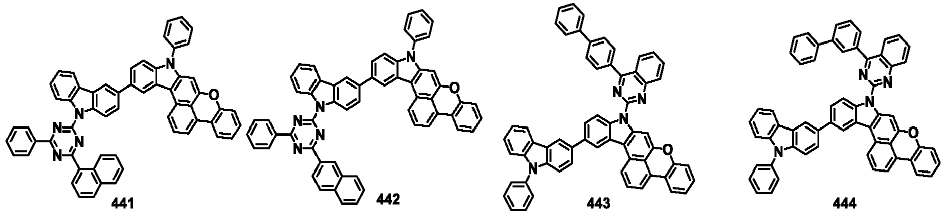

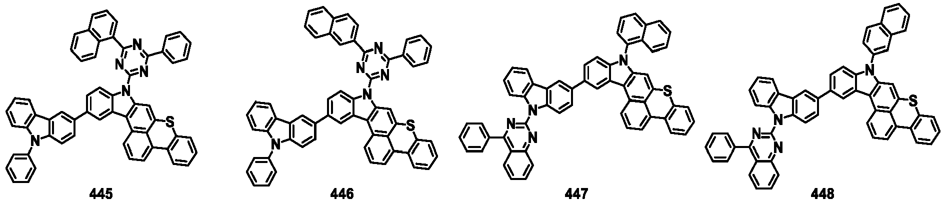

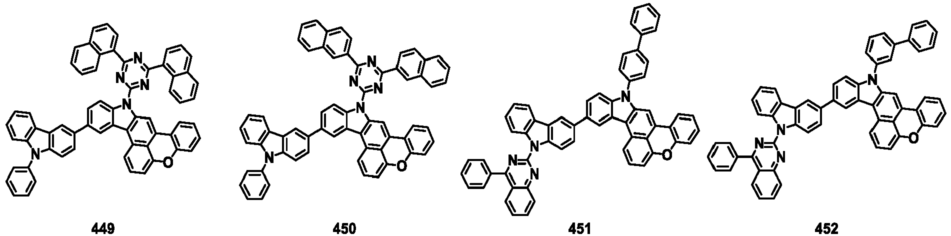

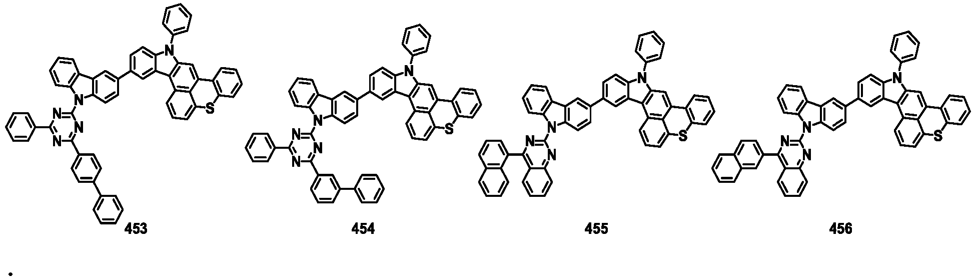

8. An organic light emitting device comprising,
a first electrode;
a second electrode provided to face the first electrode; and
one or more organic layers provided between the first electrode and the second electrode,
wherein at least one of the one or more organic layers comprises the heterocyclic compound according to any one of claims 1 to 7.

9. The organic light emitting device according to claim 8, wherein the one or more organic layers further comprises a heterocyclic compound represented by Formula 2 below: 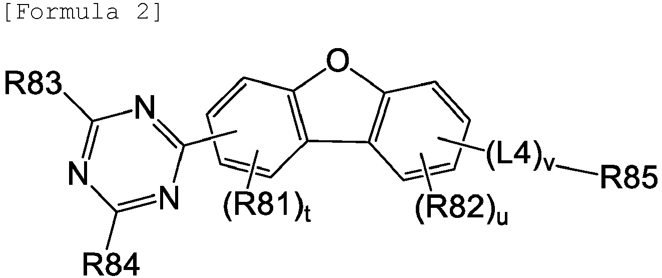 wherein,
L4 is a single bond; a substituted or unsubstituted C6 to C60 arylene group; or a substituted or unsubstituted C2 to C60 heteroarylene group,
R81 to R85 are the same as or different from each other, and are each independently selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; - P(=0)R101R102; -SiR101R102R103; and -NR101R102, or two or more groups adjacent to each other are combined with each other to form a substituted or unsubstituted C6 to C60 aromatic hydrocarbon ring or a substituted or unsubstituted C2 to C60 heterocycle, wherein R101, R102, and R103 are the same as or
different from each other, and are each independently a substituted or unsubstituted C1 to C60 alkyl group; a substituted or unsubstituted C6 to C60 aryl group; or a substituted or unsubstituted C2 to C60 heteroaryl group,
t is an integer from 0 to 3, and when t is 2 or more, each R81 is the same as or different from each other, u is an integer from 0 to 3, and when u is 2 or more, each R82 is the same as or different from each other, v is an integer from 0 to 5, and when v is 2 or more, each L4 is the same as or different from each other.

10. The organic light emitting device according to claim 9, wherein Formula 2 is represented by Formula 2-1 or Formula 2-2 below: 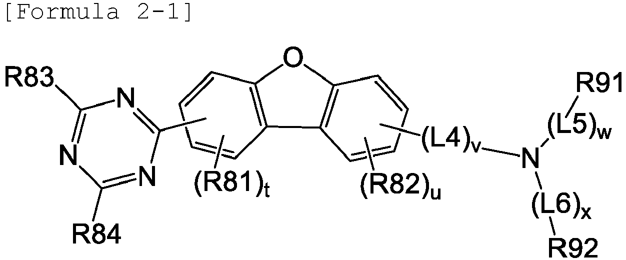 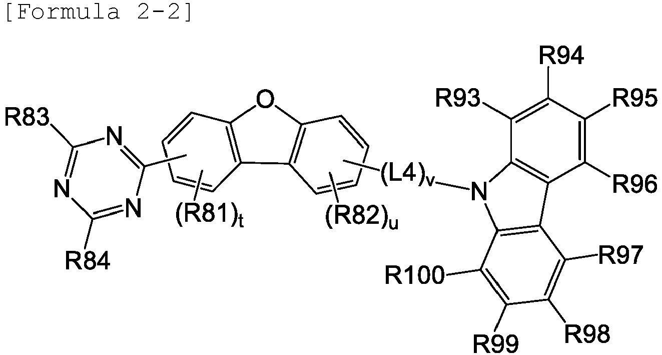 wherein,
L5 and L6 are the same as or different from each other, and are each independently a single bond; a substituted or unsubstituted C6 to C60 arylene group; or a substituted or unsubstituted C2 to C60 heteroarylene group,
w is an integer from 0 to 5, and when w is 2 or more, each L5 is the same as or different from each other, x is an integer from 0 to 5, and when x is 2 or more, each L6 is the same as or different from each other,
R91 to R100 are the same as or different from each other, and are each independently selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; - P(=0)R101R102; -SiR101R102R103; and -NR101R102, or two or more groups adjacent to each other are combined with each other to form a substituted or unsubstituted C6 to C60 aromatic hydrocarbon ring or a substituted or unsubstituted C2 to C60 heterocycle, wherein R101, R102, and R103 are the same as or different from each other, and are each independently a substituted or unsubstituted C1 to C60 alkyl group; a substituted or unsubstituted C6 to C60 aryl group; or a substituted or unsubstituted C2 to C60 heteroaryl group, and
R81 to R84, L4, t, u, and v are the same as defined in Formula 2 above.

11. The organic light emitting device according to claim 9, wherein the heterocyclic compound represented by Formula 2 is any one selected from compounds below:

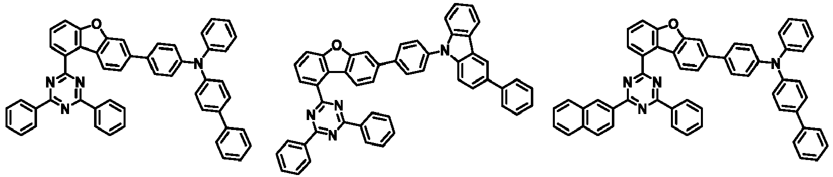

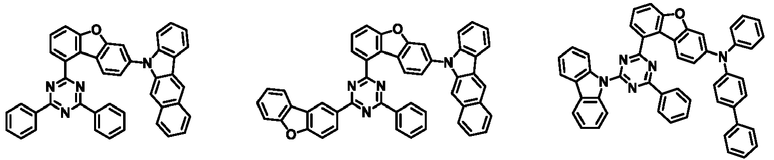

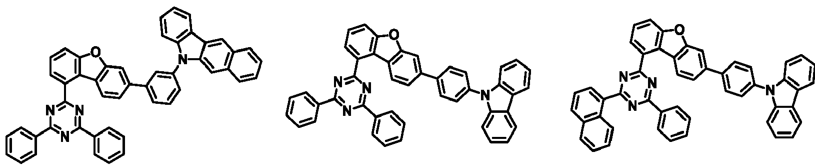

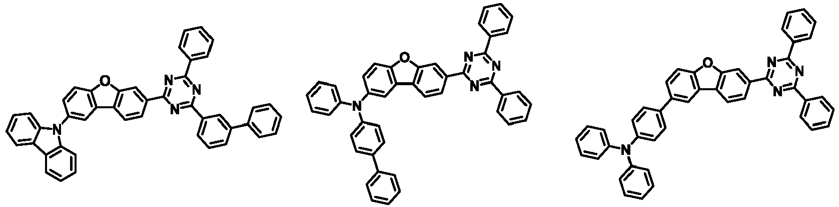

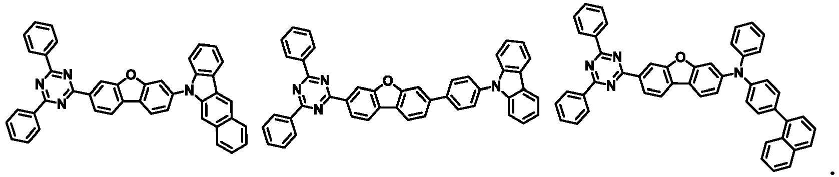

12. The organic light emitting device according to claim 8, wherein the one and more organic layers comprises a light emitting layer, the light emitting layer comprises a host material, and the host material comprises the heterocyclic compound according to any one of claims 1 to 7 and a heterocyclic compound represented by Formula 2 below: 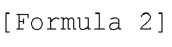 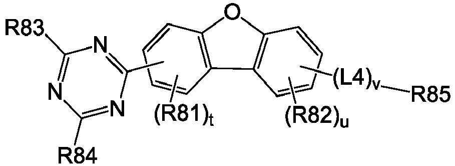 wherein,
L4 is a single bond; a substituted or unsubstituted C6 to C60 arylene group; or a substituted or unsubstituted C2 to C60 heteroarylene group,
R81 to R85 are the same as or different from each other, and are each independently selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; - P(=0)R101R102; -SiR101R102R103; and -NR101R102, or two or more groups adjacent to each other are combined with each other to form a substituted or unsubstituted C6 to C60 aromatic hydrocarbon ring or a substituted or unsubstituted C2 to C60 heterocycle, wherein R101, R102, and R103 are the same as or different from each other, and are each independently a substituted or unsubstituted C1 to C60 alkyl group; a substituted or unsubstituted C6 to C60 aryl group; or a substituted or unsubstituted C2 to C60 heteroaryl group,
t is an integer from 0 to 3, and when t is 2 or more, each R81 is the same as or different from each other, u is an integer from 0 to 3, and when u is 2 or more, each R82 is the same as or different from each other, v is an integer from 0 to 5, and when v is 2 or more, each L4 is the same as or different from each other.

13. The organic light emitting device according to claim 8, wherein the organic light emitting device further comprises one or more layers selected from the group consisting of a light emitting layer, a hole injection layer, a hole transport layer, an electron injection layer, an electron transport layer, an electron blocking layer, and a hole blocking layer.

14. A composition for organic layer of an organic light emitting device comprising a heterocyclic compound according to any one of claims 1 to 7 and a heterocyclic compound represented by Formula 2 below: 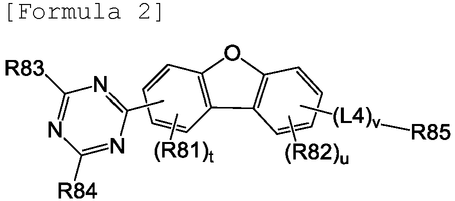 wherein,
L4 is a single bond; a substituted or unsubstituted C6 to C60 arylene group; or a substituted or unsubstituted C2 to C60 heteroarylene group,
R81 to R85 are the same as or different from each other, and are each independently selected from the group consisting of hydrogen; deuterium; halogen; a cyano group; a nitro group; a substituted or unsubstituted C1 to C60 alkyl group; a substituted or unsubstituted C2 to C60 alkenyl group; a substituted or unsubstituted C2 to C60 alkynyl group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; a substituted or unsubstituted C1 to C60 alkoxy group; a substituted or unsubstituted C6 to C60 aryloxy group; - P(=0)R101R102; -SiR101R102R103; and -NR101R102, or two or more groups adjacent to each other are combined with each other to form a substituted or unsubstituted C6 to C60 aromatic hydrocarbon ring or a substituted or unsubstituted C2 to C60 heterocycle, wherein R101, R102, and R103 are the same as or different from each other, and are each independently a substituted or unsubstituted C1 to C60 alkyl group; a substituted or unsubstituted C6 to C60 aryl group; or a substituted or unsubstituted C2 to C60 heteroaryl group,
t is an integer from 0 to 3, and when t is 2 or more, each R81 is the same as or different from each other, u is an integer from 0 to 3, and when u is 2 or more, each R82 is the same as or different from each other, v is an integer from 0 to 5, and when v is 2 or more, each L4 is the same as or different from each other.

15. The composition for organic layer of the organic light emitting device according to claim 14, wherein the weight ratio of the heterocyclic compound according to any one of claims 1 to 7:the heterocyclic compound represented by Formula 2 is 1:10 to 10:1.

16. A method for manufacturing an organic light emitting device comprising the steps of,
preparing a substrate;
forming a first electrode on the substrate;
forming one or more organic layers on the first electrode; and
forming a second electrode on the one or more organic layers,
wherein the step of forming the one or more organic layers comprises a step of forming the one or more organic layers using the composition for organic layer according to claim 14.

17. The method for manufacturing the organic light emitting device according to claim 16, wherein the step of forming the one or more organic layers comprises pre-mixing the heterocyclic compound represented by Formula 1 and the heterocyclic compound represented by Formula 2, and forming the one and more organic layer using a thermal vacuum deposition method.
